# EUROPEAN PATENT SPECIFICATION

(11) **EP 3 500 681 B1**
(45) Date of publication and mention of the grant of the patent: **20.01.2021**
(21) Application number: 17755486.2
(22) Date of filing: 18.08.2017
(51) Int. Cl.: C12Q 1/6886

(54) **MULTIPLEX ALLELE SPECIFIC PCR ASSAYS FOR DETECTION OF ESTROGEN RECEPTOR ESR1 MUTATIONS**
MULTIPLEXE ALLEL-SPEZIFISCHE PCR-ASSAYS ZUR DETEKTION VON ÖSTROGENREZEPTOR-ESR1-MUTATIONEN
DOSAGES PCR MULTIPLEX SPÉCIFIQUES À DES ALLÈLES POUR LA DÉTECTION DE MUTATIONS DU RÉCEPTEUR D'OESTROGÈNES ESR1

(30) Priority: 18.08.2016 US 201662376799 P
(43) Date of publication of application: 26.06.2019
(73) Proprietor: Roche Diagnostics GmbH, 68305 Mannheim (DE); F. Hoffmann-La Roche AG, 4070 Basel (CH)
(72) Inventor: CHEN, Xiaoying, Danville California 94506 (US); CHIEN, Sean, Danville California 94506 (US); LITTERST, Claudia, Walnut Creek California 94597 (US); TRAN, Ha, Daly City California 94014 (US)
(74) Representative: Schwarz, Ralf
(86) International application number: PCT/EP2017/070903
(87) International publication number: WO 2018/033615

(56) References cited:
- WO-A1-2015/136017
- WO-A2-2013/056178
- WO-A2-2015/093948
- TING WANG ET AL: "A multiplex allele-specific real-time PCR assay for screening of ESR1 mutations in metastatic breast cancer", EXPERIMENTAL AND MOLECULAR PATHOLOGY., vol. 98, no. 2, 1 April 2015 (2015-04-01), pages 152-157, XP055419988, US ISSN: 0014-4800, DOI: 10.1016/j.yexmp.2015.03.004
- BACKES FLOOR J ET AL: "Estrogen receptor-alpha as a predictive biomarker in endometrioid endometrial cancer", GYNECOLOGIC ONCOLOGY, vol. 141, no. 2, 10 March 2016 (2016-03-10), pages 312-317, XP029521729, ISSN: 0090-8258, DOI: 10.1016/J.YGYNO.2016.03.006
- P. WANG ET AL: "Sensitive Detection of Mono- and Polyclonal ESR1 Mutations in Primary Tumors, Metastatic Lesions, and Cell-Free DNA of Breast Cancer Patients", CLINICAL CANCER RESEARCH, vol. 22, no. 5, 23 October 2015 (2015-10-23), pages 1130-1137, XP055420000, US ISSN: 1078-0432, DOI: 10.1158/1078-0432.CCR-15-1534
- GELSOMINO LUCA ET AL: "ESR1 mutations affect anti-proliferative responses to tamoxifen through enhanced cross-talk with IGF signaling", BREAST CANCER RESEARCH AND TREATMENT, SPRINGER , NY, US, vol. 157, no. 2, 13 May 2016 (2016-05-13), pages 253-265, XP036219798, ISSN: 0167-6806, DOI: 10.1007/S10549-016-3829-5 [retrieved on 2016-05-13]
- JIAXIN NIU ET AL: "Incidence and clinical significance of ESR1 mutations in heavily pretreated metastatic breast cancer patients", ONCOTARGETS AND THERAPY, 1 November 2015 (2015-11-01), page 3323, XP055420005, DOI: 10.2147/OTT.S92443
- CATHERINE S. GRASSO ET AL: "The mutational landscape of lethal castration-resistant prostate cancer", NATURE, vol. 487, no. 7406, 20 May 2012 (2012-05-20), pages 239-243, XP055420012, ISSN: 0028-0836, DOI: 10.1038/nature11125

## Description

### BACKGROUND OF THE INVENTION

Seventy percent of breast cancers are estrogen receptor (ER) positive and, while the majority of these patients initially respond to hormone therapy, approximately 20-30% will become therapy refractory. Recent data suggest activating mutations in the estrogen receptor gene (ESR1) which are acquired during anti-estrogen treatment and rarely found in primary untreated ER positive breast cancer are associated with resistance. A growing number of activating ESR1 mutations located in the ligand-binding domain have been identified in samples from hormone-refractory breast cancer, the most common mutations include K303R, E380Q, V392I, S463P, K531E, V534E, P535H, L536Q/R, Y537S/N/C, D538G and R555C. While the mechanism of action is not fully elucidated for all mutations, it has been shown by in vitro experiments that mutations in L536Q, Y537S/C/N and D538G stabilize the estrogen receptor ligand binding domain in an active conformation, thus allowing recruitment of transcriptional coactivators in the absence of ligand. Hypersensitivity to estrogen is considered the mechanism for the K303R mutation.

Detection of ESR1 mutations thus has potential for predicting hormone resistance and directing therapy. Next generation sequencing (NGS) is a common approach for such detection, since it enables the simultaneous detection of many mutations with small amounts of samples. However, NGS is very labor-intensive, lengthy and expensive. Digital PCR (dPCR) represents a highly sensitive method that has been employed for ESR1 mutation detection, but it has a number of drawbacks: the dPCR workflow is lengthy and requires special equipment, and multiplexing is limited by the number of optical channels available on current dPCR instruments WANG et al, EXPERIMENTAL AND MOLECULAR PATHOLOGY, 2015 discloses a multiplex allele-specific real-time PCR assay for screening of ESR1 mutations in metastatic breast cancer.

### SUMMARY OF THE INVENTION

Provided herein are kits, assays, and methods for detecting mutations in the ESR1 gene, and methods of treatment for individuals with hormone sensitive cancers.

Provided herein are kits comprising one or more vessels, each of the vessels holding two or more primer pairs, each primer pair specific for a different sequence in the ESR1 gene; one or more probes specific for a different sequence in the ESR1 gene, wherein each probe is labeled; a primer pair specific for an internal control sequence; and a probe specific for the internal control sequence. In some embodiments, the each probe is labeled with a fluorophore and quencher. In some embodiments, the kit comprises 1-10 vessels, *e.g.,* 2-5, 2, 3, 4, 5, 6, 7, 8, 9, 10, or more vessels. In some embodiments, each of the vessels holds two or more primer pairs (including the internal control primer pair), *e.g.,* 3-16, 4-10, or 5-8. For example, all exon 8 mutations, internal control, and two or more other mutation primer pairs can be included in a single tube. In some embodiments, each of the vessels holds three to four probes (including the internal control probe). In some embodiments, each vessel further holds a thermostable DNA polymerase.

In some embodiments, the kit further includes positive controls, *e.g.,* samples with known ESR1 mutations, and/or a negative control, *e.g.,* a sample that does not include ESR1 mutations.

In some embodiments, each different sequence of the ESR1 gene is selected from the group consisting of comprising ESR1 V422DelV, ESR1 S463P, ESR1 L536H, ESR1 L536P, ESR1 L536Q, ESR1 L536R, ESR1 D538G, ESR1 K303R, ESR1 E380Q, ESR1 L536_D538>P, ESR1 Y537C, ESR1 Y537N, ESR1 Y537S, ESR1 S341L, ESR1 L429V, ESR1 V533M, ESR1 V534E, and ESR1 P535H. In some embodiments the kit includes at least two primer pairs selected from the group consisting of a primer pair specific for ESR1 V422DelV, a primer pair specific for ESR1 S463P, a primer pair specific for ESR1 L536H, a primer pair specific for ESR1 L536P, a primer pair specific for ESR1 L536Q, a primer pair specific for ESR1 L536R, a primer pair specific for ESR1 D538G, ESR1 K303R, a primer pair specific for ESR1 E380Q, a primer pair specific for ESR1 L536_D538>P, a primer pair specific for ESR1 Y537C, a primer pair specific for ESR1 Y537N, a primer pair specific for ESR1 Y537S, a primer pair specific for ESR1 S341L, a primer pair specific for ESR1 L429V, a primer pair specific for ESR1 V533M, a primer pair specific for ESR1 V534E, and a primer pair specific for ESR1 P535H. In some embodiments, at least one primer includes a modified, non-naturally occurring nucleotide.

In some embodiments, the kit comprises a primer pair specific for ESR1 V422DelV, a primer pair specific for ESR1 S463P, a primer pair specific for ESR1 L536H, a primer pair specific for ESR1 L536P, a primer pair specific for ESR1 L536Q, a primer pair specific for ESR1 L536R, a primer pair specific for ESR1 D538G, ESR1 K303R, a primer pair specific for ESR1 E380Q, a primer pair specific for ESR1 L536_D538>P, a primer pair specific for ESR1 Y537C, a primer pair specific for ESR1 Y537N, a primer pair specific for ESR1 Y537S, a primer pair specific for ESR1 S341L, a primer pair specific for ESR1 L429V, a primer pair specific for ESR1 V533M, a primer pair specific for ESR1 V534E, and a primer pair specific for ESR1 P535H. In some embodiments, the kit further comprises a probe that specifically detects ESR1 V422DelV, ESR1 S463P, ESR1 L536H, ESR1 L536P, ESR1 L536Q, ESR1 L536R, ESR1 D538G, ESR1 K303R, ESR1 E380Q, ESR1 L536_D538>P, ESR1 Y537C, ESR1 Y537N, ESR1 Y537S, ESR1 S341L, ESR1 L429V, ESR1 V533M, ESR1 V534E, and ESR1 P535H.

In some embodiments, the kit comprises a (i) a first vessel holding a primer pair specific for ESR1 V422DelV, a primer pair specific for ESR1 S463P, a primer pair specific for ESR1 L536H, a primer pair specific for ESR1 L536P, a primer pair specific for ESR1 L536Q, a primer pair specific for L536R, and a primer pair specific for ESR1 D538G; (ii) a second vessel holding a primer pair specific for ESR1 K303R, a primer pair specific for ESR1 E380Q, a primer pair specific for ESR1 L536_D538>P, a primer pair specific for ESR1 Y537C, a primer pair specific for ESR1 Y537N, and a primer pair specific for ESR1 Y537S; and (iii) a third vessel holding a primer pair specific for ESR1 S341L, a primer pair specific for ESR1 L429V, a primer pair specific for ESR1 V533M, a primer pair specific for ESR1 V534E, and a primer pair specific for ESR1 P535H. In some embodiments, the first vessel further holds probes specific for ESR1 V422DelV, ESR1 S463P, ESR1 L536H, ESR1 L536P, ESR1 L536Q, ESR1 L536R, and ESR1 D538G. In some embodiments, a single probe specifically detects ESR1 L536H, ESR1 L536P, ESR1 L536Q, ESR1 L536R, and ESR1 D538G. In some embodiments, the second vessel further holds probes specific for ESR1 K303R, ESR1 E380Q, ESR1 L536_D538>P, ESR1 ESR1 Y537C, ESR Y537N, ESR1 Y537S. In some embodiments, a single probe specifically detects ESR1 L536_D538>P, ESR1 ESR1 Y537C, ESR Y537N, ESR1 Y537S. In some embodiments, the third vessel holds probes specific for ESR1 S341L, ESR1 L429V, ESR1 V533M, ESR1 V534E, and ESR1 P535H. In some embodiments, a single probe specifically detects ESR1 V533M, ESR1 V534E, and ESR1 P535H.

In some embodiments, the allele-specific primer to detect the ESR1 K303R mutation is selected from the group consisting of SEQ ID NOs: 479, 481, and 484, and in some embodiments, the common primer and probe sequences are SEQ ID NOs:476 and 477, respectively. In some embodiments, the allele-specific primer to detect the ESR1 S341L mutation is selected from the group consisting of SEQ ID NOs:239, 240, 242, 245, 246, 247, 253, 254, 255, 256, 257, 258, and 259, and in some embodiments, the common primer and probe sequences are SEQ ID NOs:235 and 237, respectively. In some embodiments, the allele-specific primer to detect the ESR1 E380Q mutation is selected from the group consisting of SEQ ID NOs:32, 34, 36, 37, 38, 39, 40, 41, and 42, and in some embodiments, the common primer and probe sequences are SEQ ID NOs:528 and 31, respectively. In some embodiments, the allele-specific primer to detect the ESR1 V422DELV mutation is selected from the group consisting of SEQ ID NOs:287, 294, 295, 296, 297, 298, and in some embodiments, the common primer and probe sequences are SEQ ID NOs:285 and 286, respectively. In some embodiments, the allele-specific primer to detect the ESR1 L429V mutation is selected from the group consisting of SEQ ID NOs:66, 68, 69, 70, 72, 73, 75, 76, 77, 778, 79, 80, and 81, and in some embodiments, the common primer and probe sequences are SEQ ID NOs:59 and 60, respectively. In some embodiments, the allele-specific primer to detect the ESR1 S463P mutation is selected from the group consisting of SEQ ID NOs:264, 265, 267, 268, 269, 270, 271, 272, 281, 282, 283, and 275, and in some embodiments, the common primer and probe sequences are SEQ ID NOs:261 and 262, respectively.

In some embodiments, a common primer and probe are used to amplify and detect all ESR1 exon 8 mutations in the assay, *e.g*., SEQ ID NOs:314 and/ or 394, and 407, respectively. In some embodiments, the allele-specific primer to detect the ESR1 V533M mutation is selected from the group consisting of SEQ ID NOs:322, 329, 330, 345, 346, 347, 348, 349, 350, 352, and 353. In some embodiments, the allele-specific primer to detect the ESR1 V534E mutation is selected from the group consisting of SEQ ID NOs:364, 365, 367, 368, 370, 371, 373, 378, 388, 391, 392, and 393. In some embodiments, the allele-specific primer to detect the ESR1 P535H mutation is selected from the group consisting of SEQ ID NOs:181, 188, 189, 190, 191, 198, 199, 200, 203, 204, 205, 208, 209, 212, 225, 226, 228, 229, and 231. In some embodiments, the allele-specific primer to detect the ESR1 L536H mutation is selected from the group consisting of SEQ ID NOs:96, 101, 104, 105, 107, 110, 111, 112, 113, 114, 115, 116, and 117. In some embodiments, the allele-specific primer to detect the ESR1 L536P mutation is selected from the group consisting of SEQ ID NOs:134, 135, and 137. In some embodiments, the allele-specific primer to detect the ESR1 L536Q mutation is selected from the group consisting of SEQ ID NOs:141, 151, 154, 155, 157, 158, 159, and 160. In some embodiments, the allele-specific primer to detect the ESR1 L536R mutation is selected from the group consisting of SEQ ID NOs:161, 172, 174, 175, 176, 177, 178, 179, and 180. In some embodiments, the allele-specific primer to detect the ESR1 Y537C mutation is selected from the group consisting of SEQ ID NOs:397, 408, 415, 416, 417, 418, 419, 420, and 424. In some embodiments, the allele-specific primer to detect the ESR1 Y537N mutation is selected from the group consisting of SEQ ID NOs:426, 436, 441, 445, 446, 447, and 448. In some embodiments, the allele-specific primer to detect the ESR1 Y537S mutation is selected from the group consisting of SEQ ID NOs:449, 459, 466, 467, 468, 469, 470, 471, and 472. In some embodiments, the allele-specific primer to detect the ESR1 D538G mutation is selected from the group consisting of SEQ ID NOs: 14, 21, 22, 23, 24, and 25. In some embodiments, the allele-specific primer to detect the ESR1 L536_D538>P mutation is selected from the group consisting of SEQ ID NOs:84, 85, 86, 87, 88, 89, 90, 91, 92, and 93.

In some embodiments, the kit includes oligonucleotides having the sequences of SEQ ID NOs:24, 113, 134, 158, 178, 264, 261, 262, 285, 286, 298, 314, 394, 407, 31, 42, 90, 415, 447, 469, 476, 477, 481, 528, 59, 60, 70, 231, 235, 237, 245, 350, and 388. In some embodiments, each vessel includes an internal control, *e.g.,* SEQ ID NOs:511, 514, and 517.

Further provided are methods for determining (*e.g*., detecting) the presence or absence of two or more ESR1 mutations in a sample from an individual, comprising (i) obtaining a sample from the individual; (ii) carrying out multiplex allele-specific PCR to determine the presence or absence of two or more ESR1 mutations. In some embodiments, the sample is selected from blood, plasma, serum, urine, or mucosal tissue (*e.g.,* from a buccal swab). In some embodiments, the individual has, or was diagnosed with, hormone responsive cancer (*e.g.,* estrogen receptor and/or progesterone receptor positive breast or ovarian cancer). In some embodiments, the individual is undergoing hormone therapy. In some embodiments, the method further comprises providing hormone therapy to the individual prior to step (i) (*e.g.,* treatment with a SERM, aromatase inhibitor, or LH blocking agent). In some embodiments, the method further comprises providing modified treatment to the individual when the presence of an ESR1 mutation is determined in step (ii) (*e.g.,* an additional hormone therapy, or standard chemotherapy).

In some embodiments, the method comprises carrying out multiplex allele-specific PCR to determine the presence or absence of ten or more ESR1 mutations, *e.g*., 10-20, 11, 12, 13, 14, 15, 16, 17, 18, or 19. In some embodiments, the two or more ESR1 mutations are selected from ESR1 V422DelV, ESR1 S463P, ESR1 L536H, ESR1 L536P, ESR1 L536Q, ESR1 L536R, ESR1 D538G, ESR1 K303R, ESR1 E380Q, ESR1 L536_D538>P, ESR1 Y537C, ESR1 Y537N, ESR1 Y537S, ESR1 S341L, ESR1 L429V, ESR1 V533M, ESR1 V534E, and ESR1 P535H. In some embodiments, the two or more ESR1 mutations include ESR1 V422DelV, ESR1 S463P, ESR1 L536H, ESR1 L536P, ESR1 L536Q, ESR1 L536R, ESR1 D538G, ESR1 K303R, ESR1 E380Q, ESR1 L536_D538>P, ESR1 Y537C, ESR1 Y537N, ESR1 Y537S, ESR1 S341L, ESR1 L429V, ESR1 V533M, ESR1 V534E, and ESR1 P535H.

In some embodiments, the multiplex allele-specific PCR is carried out using a kit as described herein, *e.g.,* comprising 1-10 vessels, *e.g.,* 2-5, 2, 3, 4, 5, 6, 7, 8, 9, 10, or more vessels. In some embodiments, the kit comprises three vessels. For example, the allele-specific multiplex PCR can be carried out in three vessels such that (i) ESR1 V422DelV, ESR1 S463P, ESR1 L536H, ESR1 L536P, ESR1 L536Q, L536R, and ESR1 D538G are detected in a first vessel; (ii) ESR1 K303R, ESR1 E380Q, ESR1 L536_D538>P, ESR1 Y537C, ESR1 Y537N, and ESR1 Y537S are detected in a second vessel; and (iii) ESR1 S341L, ESR1 L429V, ESR1 V533M, ESR1 V534E, and ESR1 P535H are detected in a third vessel.

Further provided herein are methods of providing modified treatment of an individual with a hormone responsive cancer (*e.g.,* breast cancer) that is undergoing hormone therapy (*e.g*., SERM, aromatase inhibitors, and/or lutenizing hormone blockers). In some embodiments, the method comprises (i) obtaining a sample from the individual (*e.g.,* blood, plasma, serum, urine, tissue, FFPET, etc.); (ii) carrying out multiplex allele-specific PCR to determine the presence or absence of two or more ESR1 mutations; and (iii) providing modified treatment of the individual if the presence of an ESR1 mutation is determined. In some embodiments, step (ii) is carried out using a kit as described herein.

In some embodiments, the multiplex allele-specific PCR determines the presence or absence of ten or more ESR1 mutations. In some embodiments, the two or more (or ten or more) ESR1 mutations are selected from the group consisting of ESR1 V422DelV, ESR1 S463P, ESR1 L536H, ESR1 L536P, ESR1 L536Q, ESR1 L536R, ESR1 D538G, ESR1 K303R, ESR1 E380Q, ESR1 L536_D538>P, ESR1 Y537C, ESR1 Y537N, ESR1 Y537S, ESR1 S341L, ESR1 L429V, ESR1 V533M, ESR1 V534E, and ESR1 P535H. In some embodiments, the presence or absence of ESR1 V422DelV, ESR1 S463P, ESR1 L536H, ESR1 L536P, ESR1 L536Q, ESR1 L536R, ESR1 D538G, ESR1 K303R, ESR1 E380Q, ESR1 L536_D538>P, ESR1 Y537C, ESR1 Y537N, ESR1 Y537S, ESR1 S341L, ESR1 L429V, ESR1 V533M, ESR1 V534E, and ESR1 P535H is determined.

In some embodiments, steps (i)-(iii) are carried out 0.5 to 5 years after the individual starts taking hormone therapy. In some embodiments, the method is carried out more than once during hormone therapy, *e.g.,* to monitor potential resistance of the tumor to hormone therapy. In some embodiments, the method is carried out periodically, *e.g*., every 6 months, while in some embodiments, the method is carried out upon clinical progression in the individual (*e.g*., tumor growth, reduced response to hormone therapy, etc.). In some embodiments, step (iii) comprises providing an additional hormone therapy or standard chemotherapy to the individual.

In addition, provided herein are methods of treating an individual with a hormone responsive cancer (*e.g.,* breast or ovarian cancer). In some embodiments, the method comprises (i) providing hormone therapy to the individual; (ii) obtaining a sample from the individual; (iii) carrying out multiplex allele-specific PCR to determine the presence or absence of two or more ESR1 mutations; and (iv) providing modified treatment to the individual if the presence of an ESR1 mutation is determined. In some embodiments, the hormone therapy is SERM.

In some embodiments, the multiplex allele-specific PCR determines the presence or absence of ten or more ESR1 mutations. In some embodiments, the two or more (or ten or more) ESR1 mutations are selected from the group consisting of ESR1 V422DelV, ESR1 S463P, ESR1 L536H, ESR1 L536P, ESR1 L536Q, ESR1 L536R, ESR1 D538G, ESR1 K303R, ESR1 E380Q, ESR1 L536_D538>P, ESR1 Y537C, ESR1 Y537N, ESR1 Y537S, ESR1 S341L, ESR1 L429V, ESR1 V533M, ESR1 V534E, and ESR1 P535H. In some embodiments, the presence or absence of ESR1 V422DelV, ESR1 S463P, ESR1 L536H, ESR1 L536P, ESR1 L536Q, ESR1 L536R, ESR1 D538G, ESR1 K303R, ESR1 E380Q, ESR1 L536_D538>P, ESR1 Y537C, ESR1 Y537N, ESR1 Y537S, ESR1 S341L, ESR1 L429V, ESR1 V533M, ESR1 V534E, and ESR1 P535H is determined.

In some embodiments, steps (ii)-(iii) are carried out 0.5-5 years after step (i) (after the individual starts taking hormone therapy). In some embodiments, steps (ii)-(iii) are carried out more than once during hormone therapy. In some embodiments, the method is carried out periodically, *e.g*., every 6 months, while in some embodiments, the method is carried out upon clinical progression in the individual (*e.g*., tumor growth, reduced response to hormone therapy, etc.). In some embodiments, step (iv) comprises providing an additional hormone therapy (*e.g*., aromatase inhibitor and/or lutenizing hormone blocker) or standard chemotherapy to the individual.

### BRIEF DESCRIPTION OF THE DRAWINGS

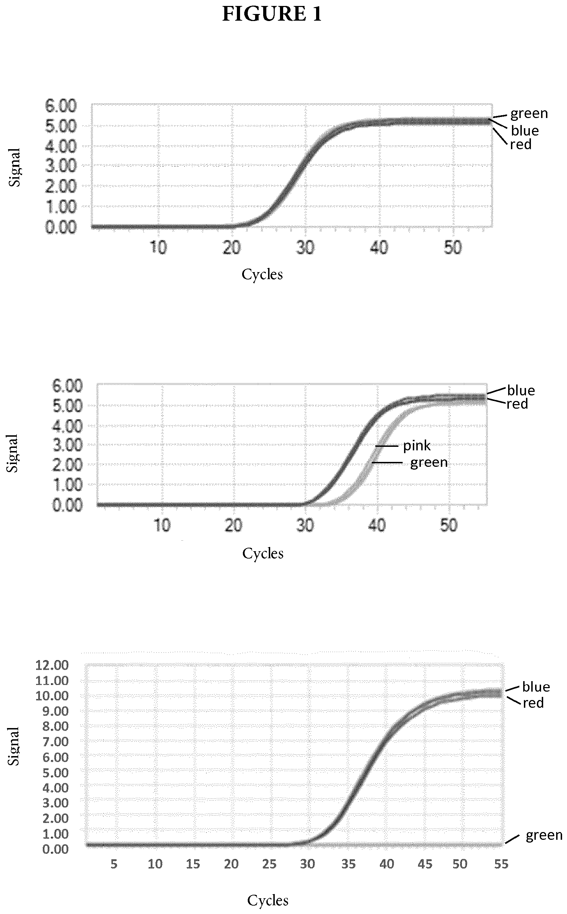

Figures 1A, 1B, and 1C compare discrimination of primers and probes for detecting mutant and wild type samples. Figure 1A shows results (amplification growth curves) using primers and a probe designed to be specific for K303R that shows poor discrimination between wild type and mutant. Figure 1B shows results using primers and a probe designed to be specific for K303R that shows better discrimination between wild type (green) and mutant (blue; red). Figure 1C shows results using primers and a probe designed to be specific for L536_D538>P that shows good specificity detecting mutant (blue; red) compared to wild type sample, with wild type sample (green) showing no signal.

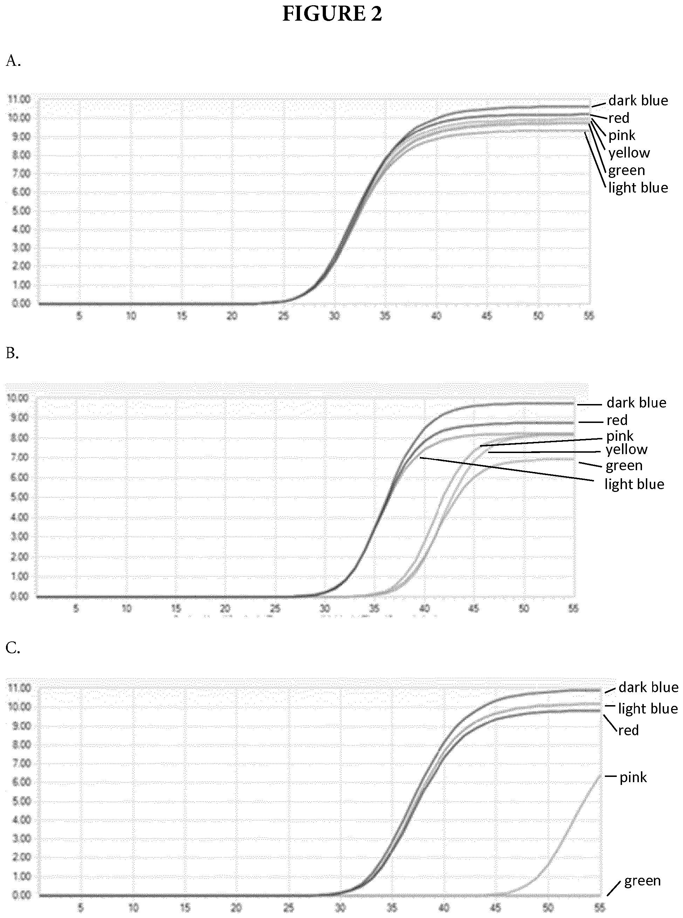

Figures 2A, 2B, and 2C also compare discrimination of primers and probes for detecting the L536R mutant and wild type samples. The amplification growth curves show cycles on the X axis and signal on the Y axis. Figure 2A shows results using the L536R_RS01 allele-specific primer that shows poor discrimination between wild type and mutant. Figure 2B shows results using the L536R_RS09 allele-specific primer that shows better discrimination between wild type (green) and mutant. Figure 2C shows results using the L536R_RS04 allele-specific primer that shows good specificity detecting mutant compared to wild type sample, with wild type sample (green) showing essentially no signal.
Figure 3 shows specificity of primers and probe designed to be specific for the P535H mutation (pink; dark blue) compared to other ESR1 mutant samples (all the other curves/colors).
Figure 4A shows amplification growth curves for an E380Q mutation positive control (MC, top/left two curves), a E380Q mutation positive sample (IN008, next lower two curves), E380 wild type (FFPET_WT, next lower two curves), and non-template control (NTC, flat bottom line).
Figure 4B shows the same amplification growth curves without the E380Q mutation positive control curves.
Figure 5 shows amplification growth curves for a D538G mutation positive control (MC, top/left curve), 3 D538G mutation positive samples (D538G, next lower three curves), D538 wild type (next lower 48 curves), and non-template control (NTC, flat bottom line).

### DETAILED DESCRIPTION OF THE INVENTION

### I. Introduction

Provided herein is a multiplex PCR assay for detecting the presence of mutations in the human estrogen receptor (ESR1). These mutations are associated with resistance to hormone therapy in cancer patients with hormone responsive cancers (ESR1 positive and/or progesterone receptor positive) that were sensitive to hormone therapy when initially administered. The assays can be carried out with non-invasive samples (*e.g.,* blood, plasma, serum, urine, etc.) so that repeated testing can be carried out in a patient on hormone therapy without taking multiple tissue biopsies.

The presently described assays rely on proven, widely adopted technology and provide accurate, reproducible, and rapid results.

### II. Definitions

The terms "estrogen receptor", "ER", and "ESR1" are used interchangeably herein unless otherwise noted. ESR1 can also be used to refer to the gene encoding the ER protein.

The term "multiplex" refers to an assay in which more than one target is detected.

The terms "receptacle," "vessel," "tube," "well," "chamber," "microchamber," etc. refer to a container that can hold reagents or an assay. If the receptacle is in a kit and holds reagents, or is being used for an amplification reaction, it can be closed or sealed to avoid contamination or evaporation. If the receptacle is being used for an assay, it can be open or accessible, at least during set up of the assay.

The terms "individually detected" or "individual detection," referring to a marker gene or marker gene product, indicates that each marker in a multiplex reaction is detected. That is, each marker is associated with a different label (detected by a differently labeled probe).

The terms "nucleic acid," "polynucleotide," and "oligonucleotide" refer to polymers of nucleotides (*e.g*., ribonucleotides or deoxyribo-nucleotides) and includes naturally-occurring (adenosine, guanidine, cytosine, uracil and thymidine), non-naturally occurring, and modified nucleic acids. The term is not limited by length (*e.g.,* number of monomers) of the polymer. A nucleic acid may be single-stranded or doublestranded and will generally contain 5'-3' phosphodiester bonds, although in some cases, nucleotide analogs may have other linkages. Monomers are typically referred to as nucleotides. The term "non-natural nucleotide" or "modified nucleotide" refers to a nucleotide that contains a modified nitrogenous base, sugar or phosphate group, or that incorporates a non-natural moiety in its structure. Examples of non-natural nucleotides include dideoxynucleotides, biotinylated, aminated, deaminated, alkylated, benzylated and fluorophor-labeled nucleotides.

The term "primer" refers to a short nucleic acid (an oligonucleotide) that acts as a point of initiation of polynucleotide strand synthesis by a nucleic acid polymerase under suitable conditions. Polynucleotide synthesis and amplification reactions typically include an appropriate buffer, dNTPs and/or rNTPs, and one or more optional cofactors, and are carried out at a suitable temperature. A primer typically includes at least one target-hybridized region that is at least substantially complementary to the target sequence (*e.g.,* having 0, 1, 2, or 3 mismatches). This region of is typically about 8 to about 40 nucleotides in length, *e.g.,* 12-25 nucleotides. A "primer pair" refers to a forward and reverse primer that are oriented in opposite directions relative to the target sequence, and that produce an amplification product in amplification conditions. In some embodiments, multiple primer pairs rely on a single common forward or reverse primer. For example, multiple allele-specific forward primers can be considered part of a primer pair with the same, common reverse primer, *e.g.,* if the multiple alleles are in close proximity to each other.

As used herein, "probe" means any molecule that is capable of selectively binding to a specifically intended target biomolecule, for example, a nucleic acid sequence of interest that hybridizes to the probes. The probe is detectably labeled with at least one non-nucleotide moiety. In some embodiments, the probe is labeled with a fluorophore and quencher.

The words "complementary" or "complementarity" refer to the ability of a nucleic acid in a polynucleotide to form a base pair with another nucleic acid in a second polynucleotide. For example, the sequence A-G-T (A-G-U for RNA) is complementary to the sequence T-C-A (U-C-A for RNA). Complementarity may be partial, in which only some of the nucleic acids match according to base pairing, or complete, where all the nucleic acids match according to base pairing. A probe or primer is considered "specific for" a target sequence if it is at least partially complementary to the target sequence. Depending on the conditions, the degree of complementarity to the target sequence is typically higher for a shorter nucleic acid such as a primer (*e.g*., greater than 80%, 90%, 95%, or higher) than for a longer sequence. In some embodiments, the term "each primer pair specific for a different sequence in the ESR1 gene" indicates that each primer pair specifically amplifies a different sequence, *e.g.,* a different allele or mutation, of the ESR1 gene.

The term "specifically amplifies" indicates that a primer set amplifies a target sequence more than non-target sequence at a statistically significant level. The term "specifically detects" indicates that a probe will detect a target sequence more than non-target sequence at a statistically significant level. As will be understood in the art, specific amplification and detection can be determined using a negative control, *e.g.,* a sample that includes the same nucleic acids as the test sample, but not the target sequence or a sample lacking nucleic acids. For example, primers and probes that specifically amplify and detect a target sequence result in a Ct that is readily distinguishable from background (non-target sequence), *e.g.,* a Ct that is at least 2, 3, 4, 5, 5-10, 10-20, or 10-30 cycles less than background. The term "allele-specific" PCR refers to amplification of a target sequence using primers that specifically amplify a particular allelic variant of the target sequence. Typically, the forward or reverse primer includes the exact complement of the allelic variant at that position.

The terms "identical" or "percent identity," in the context of two or more nucleic acids, or two or more polypeptides, refer to two or more sequences or subsequences that are the same or have a specified percentage of nucleotides, or amino acids, that are the same (*e.g.,* about 60% identity, *e.g*., at least any of 65%, 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters, or by manual alignment and visual inspection. See *e.g*., the NCBI web site at ncbi.nlm.nih.gov/BLAST. Such sequences are then said to be "substantially identical." Percent identity is typically determined over optimally aligned sequences, so that the definition applies to sequences that have deletions and/or additions, as well as those that have substitutions. The algorithms commonly used in the art account for gaps and the like. Typically, identity exists over a region comprising an a sequence that is at least about 8-25 amino acids or nucleotides in length, or over a region that is 50-100 amino acids or nucleotides in length, or over the entire length of the reference sequence.

The term "kit" refers to any manufacture (*e.g*., a package or a container) including at least one reagent, such as a nucleic acid probe or probe pool or the like, for specifically amplifying, capturing, tagging/converting or detecting RNA or DNA as described herein.

The term "amplification conditions" refers to conditions in a nucleic acid amplification reaction (*e.g.,* PCR amplification) that allow for hybridization and template-dependent extension of the primers. The term "amplicon" or "amplification product" refers to a nucleic acid molecule that contains all or a fragment of the target nucleic acid sequence and that is formed as the product of in vitro amplification by any suitable amplification method. One of skill will understand that a forward and reverse primer (primer pair) defines the borders of an amplification product. The term "generate an amplification product" when applied to primers, indicates that the primers, under appropriate conditions (*e.g*., in the presence of a nucleotide polymerase and NTPs), will produce the defined amplification product. Various PCR conditions are described in PCR Strategies (Innis et al., 1995, Academic Press, San Diego, CA) at Chapter 14; PCR Protocols : A Guide to Methods and Applications (Innis et al., Academic Press, NY, 1990).

The term "amplification product" refers to the product of an amplification reaction. The amplification product includes the primers used to initiate each round of polynucleotide synthesis. An "amplicon" is the sequence targeted for amplification, and the term can also be used to refer to amplification product. The 5' and 3' borders of the amplicon are defined by the forward and reverse primers.

The terms "individual", "subject", and "patient" are used interchangeably herein. The individual can be pre-diagnosis, post-diagnosis but pre-therapy, undergoing therapy, or post-therapy. In the context of the present disclosure, the individual is typically seeking medical care.

The term "sample" or "biological sample" refers to any composition containing or presumed to contain nucleic acid. The term includes purified or separated components of cells, tissues, or blood, *e.g.,* DNA, RNA, proteins, cell-free portions, or cell lysates. The sample can be FFPET, *e.g.,* from a tumor or metastatic lesion. The sample can also be from frozen or fresh tissue, or from a liquid sample, *e.g.,* blood or a blood component (plasma or serum), urine, semen, saliva, sputum, mucus, semen, tear, lymph, cerebral spinal fluid, mouth/throat rinse, bronchial alveolar lavage, material washed from a swab, etc. Samples also may include constituents and components of *in vitro* cultures of cells obtained from an individual, including cell lines. The sample can also be partially processed from a sample directly obtained from an individual, *e.g*., cell lysate or blood depleted of red blood cells.

The term "obtaining a sample from an individual" means that a biological sample from the individual is provided for testing. The obtaining can be directly from the individual, or from a third party that directly obtained the sample from the individual.

The term "providing therapy for an individual" means that the therapy is prescribed, recommended, or made available to the individual. The therapy may be actually administered to the individual by a third party (*e.g.,* an in-patient injection), or by the individual herself.

A "control" sample or value refers to a value that serves as a reference, usually a known reference, for comparison to a test sample or test conditions. For example, a test sample can be taken from a test condition, *e.g*., from an individual suspected of having cancer, and compared to samples from known conditions, *e.g*., from a cancer-free individual (negative control), or from an individual known to have cancer (positive control). In the context of the present disclosure, the test sample is typically from a breast cancer patient. A control can also represent an average value or a range gathered from a number of tests or results. A control can also be prepared for reaction conditions. For example, a control for the presence, quality, and/ or quantity of nucleic acid (*e.g.,* internal control) can include primers or probes that will detect a sequence known to be present in the sample (*e.g.,* a housekeeping gene such as beta actin, beta globin, glyceraldehyde 3-phosphate dehydrogenase (GAPDH), ribosomal protein L37 and L38, PPIase, EIF3, eukaryotic translation elongation factor 2 (eEF2), DHFR, or succinate dehydrogenase). In some embodiments, the internal control can be a sequence from a region of the same gene that is not commonly variant (*e.g*., in a different exon). A known added polynucleotide, *e.g*., having a designated length, can also be added. An example of a negative control is one free of nucleic acids, or one including primers or probes specific for a sequence that would not be present in the sample, *e.g*., from a different species. One of skill will understand that the selection of controls will depend on the particular assay, *e.g.,* so that the control is cell type and organism-appropriate. One of skill in the art will recognize that controls can be designed for assessment of any number of parameters. For example, a control can be devised to compare therapeutic benefit based on pharmacological data (*e.g.,* half-life) or therapeutic measures (*e.g.,* comparison of benefit and/or side effects). Controls can be designed for in vitro applications. One of skill in the art will understand which controls are valuable in a given situation and be able to analyze data based on comparisons to control values. Controls are also valuable for determining the significance of data. For example, if values for a given parameter are widely variant in controls, variation in test samples will not be considered as significant.

The terms "label," "tag," "detectable moiety," and like terms refer to a composition detectable by spectroscopic, photochemical, biochemical, immunochemical, chemical, or other physical means. For example, useful labels include fluorescent dyes (fluorophores), luminescent agents, radioisotopes (*e.g*., ³²P, ³H), electron-dense reagents, or an affinity-based moiety, *e.g.,* a poly-A (interacts with poly-T) or poly-T tag (interacts with poly-A), a His tag (interacts with Ni), or a strepavidin tag (separable with biotin). One of skill will understand that a detectable label conjugated to a nucleic acid is not naturally occurring.

Unless defined otherwise, technical and scientific terms used herein have the same meaning as commonly understood by a person of ordinary skill in the art. See, *e.g.,* Lackie, DICTIONARY OF CELL AND MOLECULAR BIOLOGY, Elsevier (4th ed. 2007); Sambrook et al., MOLECULAR CLONING, A LABORATORY MANUAL, Cold Springs Harbor Press (Cold Springs Harbor, N.Y. 1989). The term "a" or "an" is intended to mean "one or more." The terms "comprise," "comprises," and "comprising," when preceding the recitation of a step or an element, are intended to mean that the addition of further steps or elements is optional and not excluded.

### III. Nucleic acid samples

Samples for nucleic acid amplification can be obtained from any source suspected of containing nucleic acid. Samples can be taken from formalin fixed paraffin embedded tissue (FFPET), tissue biopsy, or cultured cells (*e.g.,* obtained from a patient, or representing a control). In some embodiments, the sample is obtained in a non-invasive manner, *e.g*., from urine, skin, swab, saliva, blood or a blood fraction.

In a sample that includes cells, the cells can be separated out (*e.g.,* using size-based filtration or centrifugation), thereby leaving cell free nucleic acids (cfNA), including nucleic acids in exosomes, microvesicles, viral particles, or those circulating freely. Alternatively, the cells can be lysed to obtain cellular nucleic acids, either in the presence of magnetic glass particles (MGPs) or before addition of the cellular lysate to the MGPs.

Methods for isolating nucleic acids from biological samples are known, *e.g*., as described in Sambrook, and several kits are commercially available (*e.g.,* High Pure RNA Isolation Kit, High Pure Viral Nucleic Acid Kit, and MagNA Pure LC Total Nucleic Acid Isolation Kit, DNA Isolation Kit for Cells and Tissues, DNA Isolation Kit for Mammalian Blood, High Pure FFPET DNA Isolation Kit, available from Roche). In the context of the presently disclosed methods, RNA is collected, though in some embodiments, the classifier can be used on previously prepared cDNA.

### IV. Estrogen Receptor Mutated Cancer and Therapies

Hormone sensitive tumors are commonly treated with hormone therapy. Hormone insensitive tumors typically do not respond to hormone therapy. The term "hormone therapy" applies to a variety of treatments that block the effect of hormones that affect growth of the tumor. In some cases however, such as certain womb and kidney cancers, progesterone or a synthetic version thereof is prescribed.

Breast cancer is often hormone sensitive, and patients diagnosed with breast cancer are tested to determine if the tumor is estrogen receptor (ESR1) and/ or progesterone receptor positive. Receptor positive tumors can be treated with agents that interfere with production of these hormones. Ovarian ablation can be carried out to remove ovaries using surgery or radiation. Surgery is often followed up with additional chemotherapy. Selective estrogen receptor modulators (SERMS) that block the effect of estrogen on the ESR include tamoxifen, raloxifene, toremifene, and fulvestrant. Additional treatments include aromatase inhibitors (*e.g*., anastrozole, exemestane, letrozole) and lutenizing hormone (LH) blockers (*e.g.,* goserelin, leuprolide). These therapies can be used in combination, *e.g*., a SERM with an aromatase inhibitor for post-menopausal patient, or a SERM with an LH blocker for pre-menopausal patient. Similar therapies are considered effective for receptor positive ovarian cancers, while prostate cancer can be treated with lutenizing hormone blockers, anti-androgens, and/ or gonadotrophin releasing hormone blockers.

In addition, patients can benefit from standard chemotherapy. This can include CHOP (cyclophosphamide; doxorubicin; vincristine; and prednisolone) or R-CHOP, which further includes rituximab and/or etoposide. The cocktail can be administered periodically for a set period of time, or until reduction in tumor size and/or symptoms are detected. For example, the CHOP or R-CHOP can be administered every 2 or 3 weeks.

Regardless of which treatment is selected, it typically begins with a low dose so that side effects can be determined, and the dose increased, *e.g.,* until side effects appear or within the patient's tolerance, or until clinical benefit is observed.

After initial treatment, patients can become resistant to hormone therapy. Resistance to hormone therapy is thought to be due, at least in part, to mutations in the estrogen receptor. Many of these mutations are in the ligand binding domain, so that the receptor is active in the absence of estrogen release. Examples include K303R, E380Q, V392I, S463P, K531E, V534E, P535H, L536Q/R, Y537S/C/N, D538G, and R555C. Testing for mutations in the estrogen receptor allows for more effective therapeutic decisions for patients. The testing can be periodic during hormone therapy, *e.g.,* before, during, or after observation of resistance. For example, a patient receiving SERM therapy can be switched to a different hormone therapy or combination thereof, or to standard chemotherapy.

### V. Amplification and detection

A nucleic acid sample can be used for detection and quantification, *e.g*., using nucleic acid amplification, *e.g*., using any primer-dependent method. In some embodiments, a preliminary reverse transcription step is carried out (also referred to as RT-PCR, not to be confused with real time PCR). *See, e.g.,* Hierro *et al.* (2006) 72:7148. The term "qRT-PCR" as used herein refers to reverse transcription and quantitative PCR. Both reactions can be carried out in a single tube without interruption, *e.g.,* to add reagents. For example, a polyT primer can be used to reverse transcribe all mRNAs in a sample with a polyA tail, random oligonucleotides can be used, or a primer can be designed that is specific for a particular target transcript that will be reverse transcribed into cDNA. The cDNA, or DNA from the sample, can form the initial template to be for quantitative amplification (real time or quantitative PCR, *i.e.,* RTPCR or qPCR). qPCR allows for reliable detection and measurement of products generated during each cycle of PCR process. Such techniques are well known in the art, and kits and reagents are commercially available, *e.g.,* from Roche Molecular Systems, Life Technologies, Bio-Rad, etc. *See, e.g.,* Pfaffl (2010) Methods: The ongoing evolution of qPCR vol. 50.

A separate reverse transcriptase and thermostable DNA polymerase can be used, *e.g.,* in a two-step (reverse transcription followed by addition of DNA polymerase and amplification) or combined reaction (with both enzymes added at once). In some embodiments, the target nucleic acid is amplified with a thermostable polymerase with both reverse transcriptase activity and DNA template-dependent activity. Exemplary enzymes include Tth DNA polymerase, the C. therm Polymerase system, and those disclosed in US20140170730 and US20140051126.

Probes for use as described herein can be labeled with a fluorophore and quencher (*e.g*., TaqMan, LightCycler, Molecular Beacon, Scorpion, or Dual Labeled probes). Appropriate fluorophores include FAM, JOE, TET, Cal Fluor Gold 540, HEX, VIC, Cal Fluor Orang 560, TAMRA, Cyanine 3, Quasar 570, Cal Fluor Red 590, Rox, Texas Red, Cyanine 5, Quasar 670, and Cyanine 5.5. Appropriate quenchers include TAMRA (for FAM, JOE, and TET), DABCYL, and BHQ1-3.

Detection devices are known in the art and can be selected as appropriate for the selected labels. Detection devices appropriate for quantitative PCR include the **cobas**® and Light Cycler® systems (Roche), PRISM 7000 and 7300 real-time PCR systems (Applied Biosystems), etc. Six-channel detection is available on the CFX96 Real Time PCR Detection System (Bio-Rad) and Rotorgene Q (Qiagen), allowing for a higher degree of multiplexing.

Results can be expressed in terms of a threshold cycle (abbreviated as Ct, and in some instances Cq or Cp). A lower Ct value reflects the rapid achievement of a predetermined threshold level, *e.g.,* because of higher target nucleic acid concentration or a more efficient amplification. A higher Ct value may reflect lower target nucleic acid concentration, or inefficient or inhibited amplification. The threshold cycle is generally selected to be in the linear range of amplification for a given target. In some embodiments, the Ct is set as the cycle at which the growth signal exceeds a pre-defined threshold line, e.g., in relation to the baseline, or by determining the maximum of the second derivation of the growth curve. Determination of Ct is known in the art, and described, *e.g.,* in US Patent No. 7363168.

### VI. Kits

Provided herein are kits for carrying out multiplex, allele-specific PCR to detect ESR1 mutations. The kits include primers and probes for specifically detecting particular ESR1 mutations as described herein.

A primer pair and probe for detecting ESR1 V422DelV can be selected from forward primer sequences SEQ ID NOs:284 or 287-300, reverse primer sequences SEQ ID NOs:285 or 302-311, and probe sequences SEQ ID NOs:286 or 301. A primer pair and probe for detecting ESR1 S463P can be selected from forward primer sequences SEQ ID NOs:260 or 263-272, reverse primer sequences SEQ ID NOs:261 or 274-283, and probe sequences SEQ ID NOs:262 or 273. A primer pair and probe for detecting ESR1 L536H can be selected from forward primer sequences SEQ ID NOs:96-105, reverse primer sequences SEQ ID NOs:106-120, and probe sequences SEQ ID NOs:94 or 95. A primer pair for detecting ESR1 L536P can be selected from forward primer sequences SEQ ID NOs:121-130 and reverse primer sequences SEQ ID NOs:131-140. A primer pair for detecting ESR1 L536Q can be selected from forward primer sequences SEQ ID NOs:141-150 and reverse primer sequences SEQ ID NOs:151-160. A primer pair for detecting ESR1 L536R can be selected from forward primer sequences SEQ ID NOs:161-170 and reverse primer sequences SEQ ID NOs:171-180. A primer pair for detecting ESR1 D538G can be selected from forward primer sequences SEQ ID NOs:4-13 and reverse primer sequences SEQ ID NOs:14-27 or 550. A primer pair and probe for detecting ESR1 K303R can be selected from forward primer sequences SEQ ID NOs:473, 474, or 478-487, reverse primer sequences SEQ ID NOs:477, 489-498 or 576, and probe sequences SEQ ID NOs:477 or 488. A primer pair and probe for detecting ESR1 E380Q can be selected from forward primer sequences SEQ ID NOs:28 or 32-42, reverse primer sequences SEQ ID NOs:29, 30, or 44-53, and probe sequences SEQ ID NOs:43 or 54. Primers for detecting ESR1 L536_D538>P can be selected from reverse primer sequences SEQ ID NOs:84-93. A primer pair and probe for detecting ESR1 Y537C can be selected from forward primer sequences SEQ ID NOs:394 or 397-406, reverse primer sequences SEQ ID NOs:395, 408-425, or 574, and probe sequences SEQ ID NOs:396 or 407. A primer pair for detecting ESR1 Y537N can be selected from forward primer sequences SEQ ID NOs:426-435 and reverse primer sequences SEQ ID NOs:436-448 or 575-577. A primer pair for detecting ESR1 Y537S can be selected from forward primer sequences SEQ ID NOs:449-458 and reverse primer sequences SEQ ID NOs:459-472 or 578. A primer pair and probe for detecting ESR1 S341L can be selected from forward primer sequences SEQ ID NOs:232, 233, or 238-247, reverse primer sequences SEQ ID NOs:234, 235, or 250-259, and probe sequences SEQ ID NOs:248 or 249. A primer pair and probe for detecting ESR1 L429V can be selected from forward primer sequences SEQ ID NOs:58 or 61-70, reverse primer sequences SEQ ID NOs:59 or 72-81, and probe sequences SEQ ID NOs:60 or 71. A primer pair and probe for detecting ESR1 V533M can be selected from forward primer sequences SEQ ID NOs:312-318, 322-333, 355, or 356, reverse primer sequences SEQ ID NOs:319, 320, or 336354, and probe sequences SEQ ID NOs:321, 334, or 335. A primer pair and probe for detecting ESR1 V534E can be selected from forward primer sequences SEQ ID NOs:357 o r363-373, reverse primer sequences SEQ ID NOs:358-362 or 378-393, and probe sequences SEQ ID NOs:363 or 374-377. A primer pair for detecting ESR1 P535H can be selected from forward primer sequences SEQ ID NOs:181-190 and reverse primer sequences SEQ ID NOs:191-231.

In addition, for mutations in exon 8, a common forward or common reverse primer can be used to amplify each amplification product, and a common probe can be used to detect each amplification product. One of skill will understand that slight variations can be made to the sequences, *e.g*., addition or deletion of 1-3 nucleotides.

Allele-specific primers selected from those disclosed in Table 5 for each mutation can be included in the kit, along with common primers and probes corresponding to that mutation.

In some embodiments, the kit comprises 1-18 stock solutions comprising allele-specific primer pairs and probes. The stock solutions can optionally include DNA polymerase, and optionally include a primer pair and probe to detect an internal control. In some embodiment, the kit includes 2-5 such stock solutions, *e.g.,* 2, 3, 4, or 5 stock solutions.

In some embodiments, the stock solution mixtures further comprise buffers, dNTPs, and other elements (*e.g*., cofactors or aptamers) appropriate for reverse transcription and amplification. Typically, the mixture is concentrated, so that an aliquot is added to the final reaction volume, along with sample (*e.g.,* DNA), enzymes, and/ or water. In some embodiments, the kit further comprises reverse transcriptase (or an enzyme with reverse transcriptase activity), and/or DNA polymerase (*e.g*., thermostable DNA polymerase such as Taq, ZO5, and derivatives thereof).

In some embodiments, the kit further includes components for DNA or RNA purification from a sample, *e.g.,* a non-invasive or tissue sample. For example, the kit can include components from MagNA Pure LC Total Nucleic Acid Isolation Kit, DNA Isolation Kit for Cells and Tissues, DNA Isolation Kit for Mammalian Blood, High Pure FFPET DNA Isolation Kit, High Pure or MagNA Pure RNA Isolation Kits (Roche), DNeasy or RNeasy Kits (Qiagen), PureLink DNA or RNA Isolation Kits (Thermo Fisher), etc.

In some embodiments, the kit further includes at least one control sample, *e.g*., nucleic acids from non-cancer sample (or pooled samples), or from a known ESR1 mutated sample (or pooled samples). In some embodiments, the kit includes a negative control, *e.g.,* lacking nucleic acids, or lacking mutated ESR1 nucleic acids. In some embodiments, the kit further includes consumables, *e.g*., plates or tubes for nucleic acid preparation, tubes for sample collection, etc. In some embodiments, the kit further includes instructions for use, reference to a website, or software.

### VII. Examples

### Assay design

Multiplex, allele-specific PCR assays to detect 18 mutations in the ESR1 gene were designed. The mutations detected are shown in Table 1.

**Table 1. ESR1 mutations**

| | **ESR1 mutation (AA)** | **ESR1 mutation (NA)** | **Exon** |
|---|---|---|---|
| 1 | K303R | 908 A>G | 4 |
| 2 | S341L | 1022 C>T | 4 |
| 3 | E380Q | 1138 G>C | 5 |
| 4 | V422DelV | 1262_1264delTGG | 6 |
| 5 | L429V | 1285 C>G | 6 |
| 6 | S463P | 1387 T>C | 7 |
| 7 | V533M | 1597 G>A | 8 |
| 8 | V534E | 1601 T>A | 8 |
| 9 | P535H | 1604 C>A | 8 |
| 10 | L536Q | 1607/8 TC>AC | 8 |
| 11 | L536R | 1607 T>G | 8 |
| 12 | L536H | 1607 T>A | 8 |
| 13 | L536P | 1607 T>C | 8 |
| 14 | L536_D538>P | 1607_1614 TCTATGAC>CC | 8 |
| 15 | Y537N | 1609 T>A | 8 |
| 16 | Y537C | 1610 A>G | 8 |
| 17 | Y537S | 1610 A>C | 8 |
| 18 | D538G | 1613 A>G | 8 |

The primers and probes generated for the assays are shown in Table 2. One of skill will understand that the list is not exhaustive, and that primers and/or probe sequences can be slightly modified from those shown, *e.g*., adding or subtracting one or a few nucleotides, shifting the position of the modified nucleotide, or using a different modified nucleotide at a given site (*e.g.,* N4_Et_dC instead of t_BB_dC). The mutation detected is indicated in the primer/ probe name. FS# and RS# refer to forward specific primer and reverse specific primer, respectively. CF# and CR# refer to common forward primer and common reverse primer, respectively. F_P# and R_P# refer to forward and reverse probes. While probes in exon 8 are labeled for specific mutations, they can be used to detect any exon 8 mutation; the allele specificity for this assay is determined by allele-specific primers. As an example, for detecting the D538G mutation, any ESR1_D538G_FS# primer can be used with any ESR1_[Exon8mut]_CR# primer and any ESR1_[Exon8mut]_F_P# probe, and any ESR1_D538G_RS# primer can be used with any ESR1_[Exon8mut]_CF# primer and any ESR1_[Exon8mut]_R_P# probe.

Modifications include t_BB_[dNTP] = N6-tert-butyl-benzyl [dNTP]; N4_Et_[dNTP] = N4-Ethyl [dNTP]; N4_Bz_[dNTP] = N4-benzyl [dNTP]; N6_Me_[dNTP] = N6-methyl [dNTP]; pdU = 5-propynyl-deoxyuracil; 7_Dz_dG = 7-deaza dG; LNA-[dNTP] = locked nucleic acid [dNTP].

**Table 2. Primer and probe sequences**

| **SEQ ID NO** | **OLIGO NAME** | **SEQUENCE** | **MODIFICATION** |
|---|---|---|---|
| 1 | ESR1_EXWT1_FS03 | TGGCCCTACTGCATCAGATCCAA | |
| 2 | ESR1_EXWT1_FS04 | ATCAGATCCAAGGGAACGAGCT | |
| 3 | ESR1_EXWT1_RS03 | GCTTGCTGCTGTCCAGGTACA | |
| 4 | ESR1_D538G_FS01 | CGTGGTGCCCCTCTATGG | |
| 5 | ESR1_D538G_FS02 | CGTGGTGCCCCTCTATAG | |
| 6 | ESR1_D538G_FS03 | CGTGGTGCCCCTCTATCG | |
| 7 | ESR1_D538G_FS04 | CGTGGTGCCCCTCTATTG | |
| 8 | ESR1_D538G_FS05 | CGTGGTGCCCCTCTAAGG | |
| 9 | ESR1_D538G_FS06 | CGTGGTGCCCCTCTACGG | |
| 10 | ESR1_D538G_FS07 | CGTGGTGCCCCTCTAGGG | |
| 11 | ESR1_D538G_FS08 | CGTGGTGCCCCTCTCTGG | |
| 12 | ESR1_D538G_FS09 | CGTGGTGCCCCTCTGTGG | |
| 13 | ESR1_D538G_FS10 | CGTGGTGCCCCTCTTTGG | |
| 14 | ESR1_D538G_RS01 | GCATCTCCAGCAGC1GGC | 1=A or optional t_BB_dA |
| 15 | ESR1_D538G_RS02 | GCATCTCCAGCAGCAGAC | |
| 16 | ESR1_D538G_RS03 | GCATCTCCAGCAGCAGCC | |
| 17 | ESR1_D538G_RS04 | GCATCTCCAGCAGCAGTC | |
| 18 | ESR1_D538G_RS05 | GCATCTCCAGCAGCAAGC | |
| 19 | ESR1_D538G_RS06 | GCATCTCCAGCAGCACGC | |
| 20 | ESR1_D538G_RS07 | GCATCTCCAGCAGCATGC | |
| 21 | ESR1_D538G_RS08 | GCATCTCCAGCAG1CGGC | 1=C or optional N4_Et_dC or t_BB_dC |
| 22 | ESR1_D538G_RS09 | GCATCTCCAGCAG1GGGC | 1=C or optional N4_Et_dC or t_BB_dC |
| 23 | ESR1_D538G_RS10 | GCATCTCCAGCAG1TGGC | 1=C or optional N4_Et_dC or t_BB_dC |
| 24 | ESR1_D538G_RS11 | GCATCTCCAGCAGCG1GC | 1=G or optional 7_Dz_dG |
| 25 | ESR1_D538G_RS12 | CAGCATCTCCAGCAGCTGGC | |
| 26 | ESR1_D538G_TBBDC_RS01 | CAGCATCTCCAGCAG1AGGC | 1=C or optional t_BB_dC |
| 27 | ESR1_D538G_TBBDC_RS09B | CAGCATCTCCAGCAG1GGGC | 1=C or optional t_BB_dC |
| 28 | ESR1_E380Q_CF01 | TGAGTCAGCAGGGTTT | |
| 29 | ESR1_E380Q_CR01 | TAGGAGCAAACAGTAGCTTCC | |
| 30 | ESR1_E380Q_CR02 | AGTTAGGAGCAAACAGTAGCTTCC | |
| 31 | ESR1_E380Q_F_P01 | 1TGTGCCT2GGCTAGAGATCCTGATGATTGGT3 | 1=Reporter |
| | | | 2=Quencher |
| | | | 3=Phosphate |
| 32 | ESR1_E380Q_FS01 | CCATGATCAGGTCCACCTT1TAC | 1=C or optional t_BB_dC |
| 33 | ESR1_E380Q_FS02 | CCATGATCAGGTCCACCTTCTCC | |
| 34 | ESR1_E380Q_FS03 | CCATGATCAGGTCCACCTTCTGC | |
| 35 | ESR1_E380Q_FS04 | CCATGATCAGGTCCACCTTCTTC | |
| 36 | ESR1_E380Q_FS05 | CCATGATCAGGTCCACCTTCAAC | |
| 37 | ESR1_E380Q_FS06 | CCATGATCAGGTCCACCTTCCAC | |
| 38 | ESR1_E380Q_FS07 | CCATGATCAGGTCCACCTTCGAC | |
| 39 | ESR1_E380Q_FS08 | CCATGATCAGGTCCACCTTATAC | |
| 40 | ESR1_E380Q_FS09 | CCATGATCAGGTCCACCTTGTAC | |
| 41 | ESR1_E380Q_FS10 | CCATGATCAGGTCCACCTTTTAC | |
| 42 | ESR1_E380Q_FS11 | CTCCATGATCAGGTCCACCTTGTAC | |
| 43 | ESR1_E380Q_R_P01 | 1TGGACCTG2ATCATGGAGGGTCAAATCCACA3 | 1=Reporter |
| | | | 2=Quencher |
| | | | 3=Phosphate |
| 44 | ESR1_E380Q_RS01 | GGATCTCTAGCCAGGCACATTG | |
| 45 | ESR1_E380Q_RS02 | GGATCTCTAGCCAGGCACATAG | |
| 46 | ESR1_E380Q_RS03 | GGATCTCTAGCCAGGCACATCG | |
| 47 | ESR1_E380Q_RS04 | GGATCTCTAGCCAGGCACATGG | |
| 48 | ESR1_E380Q_RS05 | GGATCTCTAGCCAGGCACAATG | |
| 49 | ESR1_E380Q_RS06 | GGATCTCTAGCCAGGCACACTG | |
| 50 | ESR1_E380Q_RS07 | GGATCTCTAGCCAGGCACAGTG | |
| 51 | ESR1_E380Q_RS08 | GGATCTCTAGCCAGGCACCTTG | |
| 52 | ESR1_E380Q_RS09 | GGATCTCTAGCCAGGCACGTTG | |
| 53 | ESR1_E380Q_RS10 | GGATCTCTAGCCAGGCACTTTG | |
| 54 | ESR1_EXWT1_F_P01 | 1CCCTGAAC2CGTCCGCAGCTCAAGAT3 | 1=Reporter |
| | | | 2=Quencher |
| | | | 3=Phosphate |
| 55 | ESR1_EXWT1_FS01 | CCCTACTGCATCAGATCCAA | |
| 56 | ESR1_EXWT1_FS02 | AT CAGATC CAAGGGAACGAG | |
| 57 | ESR1_EXWT1_RS01 | TTGCTGCTGTCCAGGTACA | |
| 58 | ESR1_L429V_CF01 | TGCTATGTTTTCATAGGAACCAGG | |
| 59 | ESR1_L429V_CR01 | GATTTGAGGCACACAAACTCCT | |
| 60 | ESR1_L429V_F_P01 | 1TCATCTCGGT2TCCGCATGATGAATCTGCAG3 | 1=Reporter |
| | | | 2=Quencher |
| | | | 3=Phosphate |
| 61 | ESR1_L429V_FS01 | TGGAGATCTTCGACATG1TGG | 1=C or optional N4_Bz_dC or N4_Et_dC or t_BB_dC |
| 62 | ESR1_L429V_FS02 | TGGAGATCTTCGACATGCTAG | |
| 63 | ESR1_L429V_FS03 | TGGAGATCTTCGACATGCTCG | |
| 64 | ESR1_L429V_FS04 | TGGAGATCTTCGACATGCTTG | |
| 65 | ESR1_L429V_FS05 | TGGAGATCTTCGACATGCAGG | |
| 66 | ESR1_L429V_FS06 | TGGAGATCTTCGACATGCCGG | |
| 67 | ESR1_L429V_FS07 | TGGAGATCTTCGACATGCGGG | |
| 68 | ESR1_L429V_FS08 | TGGAGATCTTCGACATGATGG | |
| 69 | ESR1_L429V_FS09 | TGGAGATCTTCGACATGGTGG | |
| 70 | ESR1_L429V_FS10 | TGGAGATCTTCGACATGTTGG | |
| 71 | ESR1_L429V_R_P01 | 1TCGAAGATCTC2CACCATGCCCTCTACACAT3 | 1=Reporter |
| | | | 2=Quencher |
| | | | 3=Phosphate |
| 72 | ESR1_L429V_RS01 | GGAACCGAGATGATGTAGCCAC | |
| 73 | ESR1_L429V_RS02 | GGAACCGAGATGATGTAGCCCC | |
| 74 | ESR1_L429V_RS03 | GGAACCGAGATGATGTAGCCGC | |
| 75 | ESR1_L429V_RS04 | GGAACCGAGATGATGTAGCCTC | |
| 76 | ESR1_L429V_RS05 | GGAACCGAGATGATGTAGCAAC | |
| 77 | ESR1_L429V_RS06 | GGAACCGAGATGATGTAGCGAC | |
| 78 | ESR1_L429V_RS07 | GGAACCGAGATGATGTAGCTAC | |
| 79 | ESR1_L429V_RS08 | GGAACCGAGATGATGTAGACAC | |
| 80 | ESR1_L429V_RS09 | GGAACCGAGATGATGTAGGCAC | |
| 81 | ESR1_L429V_RS10 | GGAACCGAGATGATGTAGTCAC | |
| 82 | ESR1_L536_CF01 | CTTTCTGTGTCTTCCCACCTACAG | |
| 83 | ESR1_L536_CR01 | AAGTGGCTTTGGTCCGTC | |
| 84 | ESR1_L536_D538>P_RS01 | CCAGCAGCAGG1GGG | 1=G or optional 7_Dz_dG |
| 85 | ESR1_L536_D538>P_RS02 | CAGCAGCAGGG1GG | 1=G or optional 7_Dz_dG |
| 86 | ESR1_L536_D538>P_RS03 | CCAGCAGCAGG1GG | 1=G or optional 7_Dz_dG |
| 87 | ESR1_L536_D538>P_RS04 | TCCAGCAGCAGG1GG | 1=G or optional 7_Dz_dG |
| 88 | ESR1_L536_D538>P_RS05 | CTCCAGCAGCAGG1GG | 1=G or optional 7_Dz_dG |
| 89 | ESR1_L536_D538>P_RS06 | CTCCAGCAGCAGG1G | 1=G or optional 7_Dz_dG |
| 90 | ESR1_L536_D538>P_RS07 | TCTCCAGCAGCAGG1G | 1=G or optional 7_Dz_dG |
| 91 | ESR1_L536_D538>P_RS08 | ATCTCCAGCAGCAGG1G | 1=G or optional 7_Dz_dG |
| 92 | ESR1_L536_D538>P_RS09 | CATCTCCAGCAGCAGGG | |
| 93 | ESR1_L536_D538>P_RS10 | GCATCTCCAGCAGCAGGG | |
| | ESR1_L536_F_P01 | 1TACATG2CGCCCACTAGCCGTGGA3 | 1=Reporter |
| | | | 2=Quencher |
| 94 | | | 3=Phosphate |
| | ESR1_L536_R_P01 | 1TGCACTT2CATGCTGTACAGATGCTCCATGC3 | 1=Reporter |
| | | | 2=Quencher |
| 95 | | | 3=Phosphate |
| 96 | ESR1_L536H_FS01 | GCAAGAACGTGGTGCCCCA | |
| 97 | ESR1_L536H_FS02 | GCAAGAACGTGGTGCCCAA | |
| 98 | ESR1_L536H_FS03 | GCAAGAACGTGGTGCCCGA | |
| 99 | ESR1_L536H_FS04 | GCAAGAACGTGGTGCCCTA | |
| 100 | ESR1_L536H_FS05 | GCAAGAACGTGGTGCCACA | |
| 101 | ESR1_L536H_FS06 | GCAAGAACGTGGTGCCGCA | |
| 102 | ESR1_L536H_FS07 | GCAAGAACGTGGTGCCTCA | |
| 103 | ESR1_L536H_FS08 | GCAAGAACGTGGTGCACCA | |
| 104 | ESR1_L536H_FS09 | GCAAGAACGTGGTGCGCCA | |
| 105 | ESR1_L536H_FS10 | GCAAGAACGTGGTGCTCCA | |
| 106 | ESR1_L536H_RS01 | CTCCAGCAGCAGGTCAT1GT | 1=A or optional t_BB_dA |
| 107 | ESR1_L536H_RS02 | CTCCAGCAGCAGGTCATAAT | |
| 108 | ESR1_L536H_RS03 | CTCCAGCAGCAGGTCATACT | |
| 109 | ESR1_L536H_RS04 | CTCCAGCAGCAGGTCATATT | |
| 110 | ESR1_L536H_RS05 | CTCCAGCAGCAGGTCATCGT | |
| 111 | ESR1_L536H_RS06 | CTCCAGCAGCAGGTCATGGT | |
| 112 | ESR1_L536H_RS07 | CTCCAGCAGCAGGTCATTGT | |
| 113 | ESR1_L536H_RS08 | CTCCAGCAGCAGGTCAAAGT | |
| 114 | ESR1_L536H_RS09 | CTCCAGCAGCAGGTCACAGT | |
| 115 | ESR1_L536H_RS10 | CTCCAGCAGCAGGTCAGAGT | |
| 116 | ESR1_L536H_RS11 | CATCTCCAGCAGCAGGTCATAAT | |
| 117 | ESR1_L536H_RS12 | CATCTCCAGCAGCAGGTCATATT | |
| 118 | ESR1_L536H_RS13 | CATCTCCAGCAGCAGGTCGTAGT | |
| 119 | ESR1_L536H_RS14 | CATCTCCAGCAGCAGGTCTTAGT | |
| 120 | ESR1_L536H_RS15 | CATCTCCAGCAGCAGGTCCTAGT | |
| 121 | ESR1_L536P_FS01 | AGAACGTGGTGCCCCC | |
| 122 | ESR1_L536P_FS02 | AGAACGTGGTGCCCAC | |
| 123 | ESR1_L536P_FS03 | AGAACGTGGTGCCCGC | |
| 124 | ESR1_L536P_FS04 | AGAACGTGGTGCCCTC | |
| 125 | ESR1_L536P_FS05 | AGAACGTGGTGCCACC | |
| 126 | ESR1_L536P_FS06 | AGAACGTGGTGCCGCC | |
| 127 | ESR1_L536P_FS07 | AGAACGTGGTGCCTCC | |
| 128 | ESR1_L536P_FS08 | AGAACGTGGTGCACCC | |
| 129 | ESR1_L536P_FS09 | AGAACGTGGTGCGCCC | |
| 130 | ESR1_L536P_FS10 | AGAACGTGGTGCTCCC | |
| 131 | ESR1_L536P_RS01 | CTCCAGCAGCAGGTCAT1GG | 1=A or optional t_BB_dA |
| 132 | ESR1_L536P_RS02 | CTCCAGCAGCAGGTCATAAG | |
| 133 | ESR1_L536P_RS03 | CTCCAGCAGCAGGTCATACG | |
| 134 | ESR1_L536P_RS04 | CTCCAGCAGCAGGTCATATG | |
| 135 | ESR1_L536P_RS05 | CTCCAGCAGCAGGTCATCGG | |
| 136 | ESR1_L536P_RS06 | CTCCAGCAGCAGGTCATGGG | |
| 137 | ESR1_L536P_RS07 | CTCCAGCAGCAGGTCATTGG | |
| 138 | ESR1_L536P_RS08 | CTCCAGCAGCAGGTCAAAGG | |
| 139 | ESR1_L536P_RS09 | CTCCAGCAGCAGGTCACAGG | |
| 140 | ESR1_L536P_RS10 | CTCCAGCAGCAGGTCAGAGG | |
| 141 | ESR1_L536Q_FS01 | AGAACGTGGTGCCCCAG | |
| 142 | ESR1_L536Q_FS02 | AGAACGTGGTGCCCCCG | |
| 143 | ESR1_L536Q_FS03 | AGAACGTGGTGCCCCGG | |
| 144 | ESR1_L536Q_FS04 | AGAACGTGGTGCCCCTG | |
| 145 | ESR1_L536Q_FS05 | AGAACGTGGTGCCCAAG | |
| 146 | ESR1_L536Q_FS06 | AGAACGTGGTGCCCGAG | |
| 147 | ESR1_L536Q_FS07 | AGAACGTGGTGCCCTAG | |
| 148 | ESR1_L536Q_FS08 | AGAACGTGGTGCCACAG | |
| 149 | ESR1_L536Q_FS09 | AGAACGTGGTGCCGCAG | |
| 150 | ESR1_L536Q_FS10 | AGAACGTGGTGCCTCAG | |
| 151 | ESR1_L536Q_RS01 | CTCCAGCAGCAGGTCAT1CT | 1=A or optional t_BB_dA |
| 152 | ESR1_L536Q_RS02 | CTCCAGCAGCAGGTCATAAT | |
| 153 | ESR1_L536Q_RS03 | CTCCAGCAGCAGGTCATAGT | |
| 154 | ESR1_L536Q_RS04 | CTCCAGCAGCAGGTCATATT | |
| 155 | ESR1_L536Q_RS05 | CTCCAGCAGCAGGTCATCCT | |
| 156 | ESR1_L536Q_RS06 | CTCCAGCAGCAGGTCATGCT | |
| 157 | ESR1_L536Q_RS07 | CTCCAGCAGCAGGTCATTCT | |
| 158 | ESR1_L536Q_RS08 | CTCCAGCAGCAGGTCAAACT | |
| 159 | ESR1_L536Q_RS09 | CTCCAGCAGCAGGTCACACT | |
| 160 | ESR1_L536Q_RS10 | CTCCAGCAGCAGGTCAGACT | |
| 161 | ESR1_L536R_FS01 | AGAACGTGGTGCCCCG | |
| 162 | ESR1_L536R_FS02 | AGAACGTGGTGCCCAG | |
| 163 | ESR1_L536R_FS03 | AGAACGTGGTGCCCGG | |
| 164 | ESR1_L536R_FS04 | AGAACGTGGTGCCCTG | |
| 165 | ESR1_L536R_FS05 | AGAACGTGGTGCCACG | |
| 166 | ESR1_L536R_FS06 | AGAACGTGGTGCCGCG | |
| 167 | ESR1_L536R_FS07 | AGAACGTGGTGCCTCG | |
| 168 | ESR1_L536R_FS08 | AGAACGTGGTGCACCG | |
| 169 | ESR1_L536R_FS09 | AGAACGTGGTGCGCCG | |
| 170 | ESR1_L536R_FS10 | AGAACGTGGTGCTCCG | |
| 171 | ESR1_L536R_RS01 | CTCCAGCAGCAGGTCAT1GC | 1=A or optional t_BB_dA |
| 172 | ESR1_L536R_RS02 | CTCCAGCAGCAGGTCATAAC | |
| 173 | ESR1_L536R_RS03 | CTCCAGCAGCAGGTCATACC | |
| 174 | ESR1_L536R_RS04 | CTCCAGCAGCAGGTCATATC | |
| 175 | ESR1_L536R_RS05 | CTCCAGCAGCAGGTCATCGC | |
| 176 | ESR1_L536R_RS06 | CTCCAGCAGCAGGTCATGGC | |
| 177 | ESR1_L536R_RS07 | CTCCAGCAGCAGGTCATTGC | |
| 178 | ESR1_L536R_RS08 | CTCCAGCAGCAGGTCAAAGC | |
| 179 | ESR1_L536R_RS09 | CTCCAGCAGCAGGTCACAGC | |
| 180 | ESR1_L536R_RS10 | CTCCAGCAGCAGGTCAGAGC | |
| 181 | ESR1_P535H_FS01 | AAGTGCAAGAACGTGGTGC1 | 1=A or optional N6_Bz_dA |
| 182 | ESR1_P535H_FS02 | AAGTGCAAGAACGTGGTGAA | |
| 183 | ESR1_P535H_FS03 | AAGTGCAAGAACGTGGTGGA | |
| 184 | ESR1_P535H_FS04 | AAGTGCAAGAACGTGGTGTA | |
| 185 | ESR1_P535H_FS05 | AAGTGCAAGAACGTGGTACA | |
| 186 | ESR1_P535H_FS06 | AAGTGCAAGAACGTGGTCCA | |
| 187 | ESR1_P535H_FS07 | AAGTGCAAGAACGTGGTTCA | |
| 188 | ESR1_P535H_FS08 | AAGTGCAAGAACGTGGAGCA | |
| 189 | ESR1_P535H_FS09 | AAGTGCAAGAACGTGGCGCA | |
| 190 | ESR1_P535H_FS10 | AAGTGCAAGAACGTGGGGCA | |
| 191 | ESR1_P535H_RS01 | AGCAGCAGGTCATAGAGGT | |
| 192 | ESR1_P535H_RS02 | AGCAGCAGGTCATAGAGAT | |
| 193 | ESR1_P535H_RS03 | AGCAGCAGGTCATAGAGCT | |
| 194 | ESR1_P535H_RS04 | AGCAGCAGGTCATAGAGTT | |
| 195 | ESR1_P535H_RS05 | AGCAGCAGGTCATAGAAGT | |
| 196 | ESR1_P535H_RS06 | AGCAGCAGGTCATAGACGT | |
| 197 | ESR1_P535H_RS07 | AGCAGCAGGTCATAGATGT | |
| 198 | ESR1_P535H_RS08 | AGCAGCAGGTCATAGCGGT | |
| 199 | ESR1_P535H_RS09 | AGCAGCAGGTCATAGGGGT | |
| 200 | ESR1_P535H_RS10 | AGCAGCAGGTCATAGTGGT | |
| 201 | ESR1_P535H_RS11 | CCAGCAGCAGGTCAT1GAGGT | 1=A or optional t_BB_dA |
| 202 | ESR1_P535H_RS12 | CCAGCAGCAGGTCATG1GGT | 1=A or C or optional t_BB_dA or t_BB_dC |
| 203 | ESR1_P535H_RS13 | CCAGCAGCAGGTCATAGGGT | |
| 204 | ESR1_P535H_RS14 | CCAGCAGCAGGTCAT1GTGGT | 1=A or optional t_BB_dA |
| 205 | ESR1_P535H_RS15 | AGCAGCAGGTCATAAAGGT | |
| 206 | ESR1_P535H_RS16 | AGCAGCAGGTCATACAGGT | |
| 207 | ESR1_P535H_RS17 | AGCAGCAGGTCATATAGGT | |
| 208 | ESR1_P535H_RS18 | AGCAGCAGGTCATAGAGG1 | 1=Optional LNA-T |
| 209 | ESR1_P535H_RS19 | AGCAGCAGGTCATAGAG1T | 1=Optional LNA-G |
| 210 | ESR1_P535H_RS20 | AGCAGCAGGTCATAGA1GT | 1=Optional LNA-G |
| 211 | ESR1_P535H_RS21 | AGCAGCAGGTCATAG1GGT | 1=Optional LNA-A |
| 212 | ESR1_P535H_RS22 | CCAGCAGCAGGTCATAG1GGT | 1=C or optional N4_Et_dC |
| 213 | ESR1_P535H_RS23 | AGCAGCAGGTCATAAACGT | |
| 214 | ESR1_P535H_RS24 | AGCAGCAGGTCATACACGT | |
| 215 | ESR1_P535H_RS25 | AGCAGCAGGTCATATACGT | |
| 216 | ESR1_P535H_RS26 | AGCAGCAGGTCATAAATGT | |
| 217 | ESR1_P535H_RS27 | AGCAGCAGGTCATACATGT | |
| 218 | ESR1_P535H_RS28 | AGCAGCAGGTCATATATGT | |
| 219 | ESR1_P535H_RS29 | AGCAGCAGGTCATAAAAGT | |
| 220 | ESR1_P535H_RS30 | AGCAGCAGGTCATACAAGT | |
| 221 | ESR1_P535H_RS31 | AGCAGCAGGTCATATAAGT | |
| 222 | ESR1_P535H_RS32 | AGCAGCAGGTCAT1GAGGT | 1=A or optional N6_Me_dA |
| 223 | ESR1_P535H_RS33 | CCAGCAGCAGGTCATAG1GGT | 1=A or optional N6_Me_dA |
| 224 | ESR1_P535H_RS34 | CCAGCAGCAGGTCAT1GTGGT | 1=A or optional N6_Me_dA |
| 225 | ESR1_P535H_RS35 | AGCAGCAGGTCATAG1GGT | 1=A or optional t_BB_dA |
| 226 | ESR1_P535H_RS36 | CAGCAGCAGGTCATAAAGGT | |
| 227 | ESR1_P535H_RS37 | CAGCAGCAGGTCATACAGGT | |
| 228 | ESR1_P535H_RS38 | CAGCAGCAGGTCATATAGGT | |
| 229 | ESR1_P535H_RS39 | CCAGCAGCAGGTCATAAAGGT | |
| 230 | ESR1_P535H_RS40 | CCAGCAGCAGGTCATACAGGT | |
| 231 | ESR1_P535H_RS41 | CCAGCAGCAGGTCATATAGGT | |
| 232 | ESR1_S341L_CF01 | AGATGGTCAGTGCCTTGTTG | |
| 233 | ESR1_S341L_CF02 | CAGATGGTCAGTGCCTTGTT | |
| 234 | ESR1_S341L_CR01 | ATTCTTACCTGGCACCCTCTTC | |
| 235 | ESR1_S341L_CR02 | CTCTTCGCCCAGTTGATCAT | |
| 236 | ESR1_S341L_F_P01 | 1AGACAGG2GAGCTGGTTCACATGATCAACTG3 | 1=Reporter 2=Quencher 3=Phosphate |
| 237 | ESR1_S341L_F_P02 | 1TGATGG2GCTTACTGACCAACCTGGCAGACA3 | 1=Reporter 2=Quencher 3=Phosphate |
| 238 | ESR1_S341L_FS01 | CCAGACCCTTCAGTGAAG1TTT | 1=C or optional t_BB_dC |
| 239 | ESR1_S341L_FS02 | CCAGACCCTTCAGTGAAGCTAT | |
| 240 | ESR1_S341L_FS03 | CCAGACCCTTCAGTGAAGCTCT | |
| 241 | ESR1_S341L_FS04 | CCAGACCCTTCAGTGAAGCTGT | |
| 242 | ESR1_S341L_FS05 | CCAGACCCTTCAGTGAAGCATT | |
| 243 | ESR1_S341L_FS06 | CCAGACCCTTCAGTGAAGCCTT | |
| 244 | ESR1_S341L_FS07 | CCAGACCCTTCAGTGAAGCGTT | |
| 245 | ESR1_S341L_FS08 | CCAGACCCTTCAGTGAAGATTT | |
| 246 | ESR1_S341L_FS09 | CCAGACCCTTCAGTGAAGGTTT | |
| 247 | ESR1_S341L_FS10 | CCAGACCCTTCAGTGAAGTTTT | |
| 248 | ESR1_S341L_R_P01 | 1ACTGAAG2GGTCTGGTAGGATCATACTCGGA3 | |
| 249 | ESR1_S341L_R_P02 | 1CTGAAG23GTCTGGTAGGATCATACTCGGAATA4 | 1=Reporter 2=Quencher 3=G or optional 7_Dz_dG 4=Phosphate |
| 250 | ESR1_S341L_RS01 | GTTGGTCAGTAAGCCCATCATCA | |
| 251 | ESR1_S341L_RS02 | GTTGGTCAGTAAGCCCATCATAA | |
| 252 | ESR1_S341L_RS03 | GTTGGTCAGTAAGCCCATCATGA | |
| 253 | ESR1_S341L_RS04 | GTTGGTCAGTAAGCCCATCATTA | |
| 254 | ESR1_S341L_RS05 | GTTGGTCAGTAAGCCCATCAACA | |
| 255 | ESR1_S341L_RS06 | GTTGGTCAGTAAGCCCATCACCA | |
| 256 | ESR1_S341L_RS07 | GTTGGTCAGTAAGCCCATCAGCA | |
| 257 | ESR1_S341L_RS08 | GTTGGTCAGTAAGCCCATCCTCA | |
| 258 | ESR1_S341L_RS09 | GTTGGTCAGTAAGCCCATCGTCA | |
| 259 | ESR1_S341L_RS10 | GTTGGTCAGTAAGCCCATCTTCA | |
| 260 | ESR1_S463P_CF01 | CTAGACCTCATCCTCTTTGAGC | |
| 261 | ESR1_S463P_CR01 | CCATCAGGTGGATCAAAGTGTCTG | |
| 262 | ESR1_S463P_F_P01 | 1ACCATAT2CCACCGAGTCCTGGACAAGATCA3 | 1=Reporter |
| | | | 2=Quencher |
| | | | 3=Phosphate |
| 263 | ESR1_S463P_FS01 | CATTCAGGAGTGTACACATTT1TGC | 1=C or optional t_BB_dC |
| 264 | ESR1_S463P_FS02 | CATTCAGGAGTGTACACATTTCTAC | |
| 265 | ESR1_S463P_FS03 | CATTCAGGAGTGTACACATTTCTCC | |
| 266 | ESR1_S463P_FS04 | CATTCAGGAGTGTACACATTTCTTC | |
| 267 | ESR1_S463P_FS05 | CATTCAGGAGTGTACACATTTCAGC | |
| 268 | ESR1_S463P_FS06 | CATTCAGGAGTGTACACATTTCCGC | |
| 269 | ESR1_S463P_FS07 | CATTCAGGAGTGTACACATTTCGGC | |
| 270 | ESR1_S463P_FS08 | CATTCAGGAGTGTACACATTTATGC | |
| 271 | ESR1_S463P_FS09 | CATTCAGGAGTGTACACATTTGTGC | |
| 272 | ESR1_S463P_FS10 | CATTCAGGAGTGTACACATTTTTGC | |
| 273 | ESR1_S463P_R_P01 | 1TGTGTAC2ACTCCTGAATGCGCAGAGAGAGA3 | 1=Reporter |
| | | | 2=Quencher |
| | | | 3=Phosphate |
| 274 | ESR1_S463P_RS01 | AGACTTCAGGGTGCTGGG | |
| 275 | ESR1_S463P_RS11 | CAGACTTCAGGGTGCTGAG | |
| 276 | ESR1_S463P_RS03 | AGACTTCAGGGTGCTGCG | |
| 277 | ESR1_S463P_RS04 | AGACTTCAGGGTGCTGTG | |
| 278 | ESR1_S463P_RS05 | AGACTTCAGGGTGCTAGG | |
| 279 | ESR1_S463P_RS06 | AGACTTCAGGGTGCTCGG | |
| 280 | ESR1_S463P_RS07 | AGACTTCAGGGTGCTTGG | |
| 281 | ESR1_S463P_RS08 | AGACTTCAGGGTGCAGGG | |
| 282 | ESR1_S463P_RS09 | AGACTTCAGGGTGCCGGG | |
| 283 | ESR1_S463P_RS10 | AGACTTCAGGGTGCGGGG | |
| 284 | ESR1_V422DELV_CF01 | GTCTTGTGGAAGATTTTCTGT | |
| 285 | ESR1_V422DELV_CR01 | TTGAGGCACACAAACTCCTC | |
| 286 | ESR1_V422DELV_F_P01 | 1TGGCTACATCA2TCTCGGTTCCGCATGATGA3 | 1=Reporter |
| | | | 2=Quencher |
| | | | 3=Phosphate |
| 287 | ESR1_V422DELV_FS01 | ATGTGTAGAGGGCATGG1GAT | 1=A or optional t_BB_dA |
| 288 | ESR1_V422DELV_FS02 | ATGTGTAGAGGGCATGGAGCT | |
| 289 | ESR1_V422DELV_FS03 | ATGTGTAGAGGGCATGGAGGT | |
| 290 | ESR1_V422DELV_FS04 | ATGTGTAGAGGGCATGGAGTT | |
| 291 | ESR1_V422DELV_FS05 | ATGTGTAGAGGGCATGGAAAT | |
| 292 | ESR1_V422DELV_FS06 | ATGTGTAGAGGGCATGGACAT | |
| 293 | ESR1_V422DELV_FS07 | ATGTGTAGAGGGCATGGATAT | |
| 294 | ESR1_V422DELV_FS08 | ATGTGTAGAGGGCATGGCGAT | |
| 295 | ESR1_V422DELV_FS09 | ATGTGTAGAGGGCATG12GAT | 1=G or optional 7_Dz_dG 2=G or optional 7_Dz_dG |
| 296 | ESR1_V422DELV_FS10 | ATGTGTAGAGGGCATGGTGAT | |
| 297 | ESR1_V422DELV_FS14 | AAATGTGTAGAG1GCATGGCGAT | 1=G or optional d_I |
| 298 | ESR1_V422DELV_FS15 | AAATGTGTAGAGGGCATGGTGAT | |
| 299 | ESR1_V422DELV_FS16 | AAATGTGTAGAGGGCATGTAGAT | |
| 300 | ESR1_V422DELV_FS17 | AAATGTGTAGAGGGCATGAAGAT | |
| 301 | ESR1_V422DELV_R_P01 | 1CCTCTACACATT2TTCCCTGGTTCCTATGA3 | 1=Reporter |
| | | | 2=Quencher |
| | | | 3=Phosphate |
| 302 | ESR1_V422DELV_RS01 | GCAGCATGTCGAAGATCTCCAT | |
| 303 | ESR1_V422DELV_RS02 | GCAGCATGTCGAAGATCTCCCT | |
| 304 | ESR1_V422DELV_RS03 | GCAGCATGTCGAAGATCTCCGT | |
| 305 | ESR1_V422DELV_RS04 | GCAGCATGTCGAAGATCTCCTT | |
| 306 | ESR1_V422DELV_RS05 | GCAGCATGTCGAAGATCTCAAT | |
| 307 | ESR1_V422DELV_RS06 | GCAGCATGTCGAAGATCTCGAT | |
| 308 | ESR1_V422DELV_RS07 | GCAGCATGTCGAAGATCTCTAT | |
| 309 | ESR1_V422DELV_RS08 | GCAGCATGTCGAAGATCTACAT | |
| 310 | ESR1_V422DELV_RS09 | GCAGCATGTCGAAGATCTGCAT | |
| 311 | ESR1_V422DELV_RS10 | GCAGCATGTCGAAGATCTTCAT | |
| 312 | ESR1_V533M_CF01 | GTAGTCCTTTCTGTGTCTTCCC | |
| 313 | ESR1_V533M_CF02 | CTTTCTGTGTCTTCCCACCTAC | |
| 314 | ESR1_V533M_CF03 | TGTCTTCCCACCTACAGTAACAAA | |
| 315 | ESR1_V533M_CF04 | CTCTAAAGTAGTCCTTTCTGTGTCTTC | |
| 316 | ESR1_V533M_CF05 | TCTAAAGTAGTCCTTTCTGTGTCTTC | |
| 317 | ESR1_V533M_CF06 | TAAAGTAGTCCTTTCTGTGTCTTCC | |
| 318 | ESR1_V533M_CF07 | AGTAGTCCTTTCTGTGTCTTCC | |
| 319 | ESR1_V533M_CR01 | GCTAGTGGGCGCATGTA | |
| 320 | ESR1_V533M_CR02 | CTAGTGGGCGCATGTA | |
| 321 | ESR1_V533M_F_P01 | 1TCTATG2ACCTGCTGCTGGAGATGCTGGA3 | 1=Reporter |
| | | | 2=Quencher |
| | | | 3=Phosphate |
| 322 | ESR1_V533M_FS01 | ACAGCATGAAGTGCAAGAA12 | 1=C or optional N4_Bz_dC or N4_Et_dC 2=A or optional N6_Bz_dA |
| 323 | ESR1_V533M_FS02 | ACAGCATGAAGTGCAAGAAAA | |
| 324 | ESR1_V533M_FS03 | ACAGCATGAAGTGCAAGAAGA | |
| 325 | ESR1_V533M_FS04 | ACAGCATGAAGTGCAAGAATA | |
| 326 | ESR1_V533M_FS05 | ACAGCATGAAGTGCAAGACCA | |
| 327 | ESR1_V533M_FS06 | ACAGCATGAAGTGCAAGAGCA | |
| 328 | ESR1_V533M_FS07 | ACAGCATGAAGTGCAAGATCA | |
| 329 | ESR1_V533M_FS08 | ACAGCATGAAGTGCAAGCACA | |
| 330 | ESR1_V533M_FS09 | ACAGCATGAAGTGCAAGGACA | |
| 331 | ESR1_V533M_FS10 | ACAGCATGAAGTGCAAGTACA | |
| 332 | ESR1_V533M_FS12 | TGTACAGCATGAAGTGCAAGCACA | |
| 333 | ESR1_V533M_FS13 | TGTACAGCATGAAGTGCAAGGACA | |
| 334 | ESR1_V533M_R_P01 | 1TGCACT2TCATGCTGTACAGATGCTCCATGC3 | 1=Reporter |
| | | | 2=Quencher |
| | | | 3=Phosphate |
| 335 | ESR1_V533M_R_P02 | 1TGCACTT2CATGCTGTACAGATGCTCCATGC3 | 1=Reporter |
| | | | 2=Quencher |
| | | | 3=Phosphate |
| 336 | ESR1_V533M_RS01 | GGTCATAGAG1GGCACCAT | 1=Optional 7_Dz_dG |
| 337 | ESR1_V533M_RS018 | GGTCATAGAG1GGCA2CAT | 1=Optional 7_Dz_dG 2=C or optional t_BB_dC or N4_Et_dC |
| 338 | ESR1_V533M_RS02 | GGTCATAGAG1GGCACCCT | 1=Optional 7_Dz_dG |
| 339 | ESR1_V533M_RS20 | GGTCATAGAG1GGCACC2T | 1=Optional 7_Dz_dG 2=C or optional LNA-A |
| 340 | ESR1_V533M_RS03 | GGTCATAGAG1GGCACCGT | 1=Optional 7_Dz_dG |
| 341 | ESR1_V533M_RS04 | GGTCATAGAG1GGCACCTT | 1=Optional 7_Dz_dG |
| 342 | ESR1_V533M_RS05 | GGTCATAGAG1GGCACAAT | 1=Optional 7_Dz_dG |
| 343 | ESR1_V533M_RS06 | GGTCATAGAG1GGCACGAT | 1=Optional 7_Dz_dG |
| 344 | ESR1_V533M_RS07 | GGTCATAGAG1GGCACTAT | 1=Optional 7_Dz_dG |
| 345 | ESR1_V533M_RS08 | GGTCATAGAG1GGCAACAT | 1=Optional 7_Dz_dG |
| 346 | ESR1_V533M_RS09 | GGTCATAGAG1GGCAGCAT | 1=Optional 7_Dz_dG |
| 347 | ESR1_V533M_RS10 | GGTCATAGAG1GGCATCAT | 1=Optional 7_Dz_dG |
| 348 | ESR1_V533M_RS11 | AGGTCATAGAG1GGCAGCAT | 1=Optional 7_Dz_dG |
| 349 | ESR1_V533M_RS12 | AGGTCATAGAG1GGCTCCAT | 1=Optional 7_Dz_dG |
| 350 | ESR1_V533M_RS13 | AGGTCATAGAG1GGCGCCAT | 1=Optional 7_Dz_dG |
| 351 | ESR1_V533M_RS14 | AGGTCATAGAG1GGCCCCAT | 1=Optional 7_Dz_dG |
| 352 | ESR1_V533M_RS15 | CAGGTCATAGAG1GGCTCCAT | 1=Optional 7_Dz_dG |
| 353 | ESR1_V533M_RS16 | CAGGTCATAGAG1GGCGCCAT | 1=Optional 7_Dz_dG |
| 354 | ESR1_V533M_RS17 | CAGGTCATAGAG1GGCCCCAT | 1=Optional 7_Dz_dG |
| 355 | ESR1_V533M_TBBA_FS01 | ACAGCATGAAGTGCAAG1ACA | 1=A or optional t_BB_dA |
| 356 | ESR1_V533M_TBBDC_FS01 | ACAGCATGAAGTGCAAGAA1A | 1=C or optional t_BB_dC |
| 357 | ESR1_V534E_CF01 | CTTTCTGTGTCTTCCCACCTAC | |
| 358 | ESR1_V534E_CR01 | GCTTTGGTCCGTCTCCT | |
| 359 | ESR1_V534E_CR02 | TGGCTTTGGTCCGTCTCCT | |
| 360 | ESR1_V534E_CR03 | ATGTAGGCGGTGGGCGTC | |
| 361 | ESR1_V534E_CR04 | CTCCACGGCTAGTGGGCG | |
| 362 | ESR1_V534E_CR05 | TGCCCCTCCACGGCTAGT | |
| 363 | ESR1_V534E_F_P01 | 1TCTATGA2CCTGCTGCTGGAGATGCTGGA3 | 1=Reporter |
| | | | 2=Quencher |
| | | | 3=Phophate |
| 364 | ESR1_V534E_FS01 | GCATGAAGTGCAAGAACGTGG1 | 1=A or optional N6_Bz_dA |
| 365 | ESR1_V534E_FS02 | GCATGAAGTGCAAGAACGTGAA | |
| 366 | ESR1_V534E_FS03 | GCATGAAGTGCAAGAACGTGCA | |
| 367 | ESR1_V534E_FS04 | GCATGAAGTGCAAGAACGTGTA | |
| 368 | ESR1_V534E_FS05 | GCATGAAGTGCAAGAACGTAGA | |
| 369 | ESR1_V534E_FS06 | GCATGAAGTGCAAGAACGTCGA | |
| 370 | ESR1_V534E_FS07 | GCATGAAGTGCAAGAACGTTGA | |
| 371 | ESR1_V534E_FS08 | GCATGAAGTGCAAGAACGAGGA | |
| 372 | ESR1_V534E_FS09 | GCATGAAGTGCAAGAACGCGGA | |
| 373 | ESR1_V534E_FS10 | GCATGAAGTGCAAGAACGGGGA | |
| 374 | ESR1_V534E_R_P01 | 1TGCACTT2CATGCTGTACAGATGCTCCATG3 | 1=Reporter |
| | | | 2=Quencher |
| | | | 3=Phosphate |
| 375 | ESR1_V534E_R_P03 | 1TGCACTT2CA3GCTG3ACAGATGC3CCATG4 | 1=Reporter |
| | | | 2=Quencher |
| | | | 3=pdU(3x) |
| | | | 4=Phosphate |
| 376 | ESR1_V534E_R_P04 | 1TGCACTT2CATGCTGTACAGATGCTCCAT3 | 1=Reporter |
| | | | 2=Quencher |
| | | | 3=Phosphate |
| 377 | ESR1_V534E_R_P05 | 1TGCACTT2CA3GCTG3ACAGA3GCTCCA34 | 1=Reporter |
| | | | 2=Quencher |
| | | | 3=pdU(4x) |
| | | | 4=Phosphate |
| 378 | ESR1_V534E_RS01 | GCAGGTCATAGAG1GGCT | 1=G or optional 7_Dz_dG |
| 379 | ESR1_V534E_RS02 | GCAGGTCATAGAGGGGAT | |
| 380 | ESR1_V534E_RS03 | GCAGGTCATAGAGGGGGT | |
| 381 | ESR1_V534E_RS04 | GCAGGTCATAGAGGGGTT | |
| 382 | ESR1_V534E_RS05 | GCAGGTCATAGAGGGACT | |
| 383 | ESR1_V534E_RS06 | GCAGGTCATAGAGGGCCT | |
| 384 | ESR1_V534E_RS07 | GCAGGTCATAGAGGGTCT | |
| 385 | ESR1_V534E_RS08 | GCAGGTCATAGAGGAGCT | |
| 386 | ESR1_V534E_RS09 | GCAGGTCATAGAGGCGCT | |
| 387 | ESR1_V534E_RS10 | GCAGGTCATAGAGGTGCT | |
| 388 | ESR1_V534E_RS11 | GCAGGTCATAGAG1GGCT | 1=G or optional 7_Dz_dG |
| 389 | ESR1_V534E_RS13 | GCAGGTCATAGAGTGGCT | |
| 390 | ESR1_V534E_RS14 | GCAGGTCATAGAGCGGCT | |
| 391 | ESR1_V534E_RS15 | CAGCAGGTCATAGAGAGGCT | |
| 392 | ESR1_V534E_RS16 | CAGCAGGTCATAGAGTGGCT | |
| 393 | ESR1_V534E_RS17 | CAGCAGGTCATAGAGCGGCT | |
| 394 | ESR1_Y537C_CF01 | CTGTGTCTTCCCACCTACAGTA | |
| 395 | ESR1_Y537C_CR01 | AAGTGGCTTTGGTCCGT | |
| 396 | ESR1_Y537C_F_P01 | 1TACATG2CGCCCACTAGCCGTGGA3 | 1=Reporter |
| | | | 2=Quencher |
| | | | 3=Phosphate |
| 397 | ESR1_Y537C_FS01 | ACGTGGTGCCCCTCTG | |
| 398 | ESR1_Y537C_FS02 | ACGTGGTGCCCCTCAG | |
| 399 | ESR1_Y537C_FS03 | ACGTGGTGCCCCTCCG | |
| 400 | ESR1_Y537C_FS04 | ACGTGGTGCCCCTCGG | |
| 401 | ESR1_Y537C_FS05 | ACGTGGTGCCCCTATG | |
| 402 | ESR1_Y537C_FS06 | ACGTGGTGCCCCTGTG | |
| 403 | ESR1_Y537C_FS07 | ACGTGGTGCCCCTTTG | |
| 404 | ESR1_Y537C_FS08 | ACGTGGTGCCCCACTG | |
| 405 | ESR1_Y537C_FS09 | ACGTGGTGCCCCCCTG | |
| 406 | ESR1_Y537C_FS10 | ACGTGGTGCCCCGCTG | |
| 407 | ESR1_Y537C_R_P01 | 1TGCACTT2CATGCTGTACAGATGCTCCATGC3 | 1=Reporter |
| | | | 2=Quencher |
| | | | 3=Phosphate |
| 408 | ESR1_Y537C_RS01 | CTCCAGCAGCAGGT1AC | 1=C or optional t_BB_dC |
| 409 | ESR1_Y537C_RS02 | CTCCAGCAGCAGGTCCC | |
| 410 | ESR1_Y537C_RS03 | CTCCAGCAGCAGGTCGC | |
| 411 | ESR1_Y537C_RS04 | CTCCAGCAGCAGGTCTC | |
| 412 | ESR1_Y537C_RS05 | CTCCAGCAGCAGGTAAC | |
| 413 | ESR1_Y537C_RS06 | CTCCAGCAGCAGGTGAC | |
| 414 | ESR1_Y537C_RS07 | CTCCAGCAGCAGGTTAC | |
| 415 | ESR1_Y537C_RS08 | CTCCAGCAGCAGGACAC | |
| 416 | ESR1_Y537C_RS09 | CTCCAGCAGCAGGCCAC | |
| 417 | ESR1_Y537C_RS10 | CTCCAGCAGCAGGGCAC | |
| 418 | ESR1_Y537C_RS11 | ATCTCCAGCAGCAGGACAC | |
| 419 | ESR1_Y537C_RS12 | CATCTCCAGCAGCAGGACAC | |
| 420 | ESR1_Y537C_RS13 | ATCTCCAGCAGCAGGT1AC | 1=C or optional t_BB_dC |
| 421 | ESR1_Y537C_RS14 | ATCTCCAGCAGCAGGTC1C | 1=A or optional t_BB_dA |
| 422 | ESR1_Y537C_RS15 | ATCTCCAGCAGCAGGTCA1 | 1=C or optional t_BB_dC |
| 423 | ESR1_Y537C_RS16 | CTCCAGCAGCAGGT1AC | 1=C or optional t_BB_dC |
| 424 | ESR1_Y537C_RS17 | CTCCAGCAGCAGGTC1C | 1=A or optional t_BB_dA or LNA-A |
| 425 | ESR1_Y537C_RS18 | CTCCAGCAGCAGGTCA1 | 1=C or optional t_BB_dC |
| 426 | ESR1_Y537N_FS01 | AACGTGGTGCCCCTCA | |
| 427 | ESR1_Y537N_FS02 | AACGTGGTGCCCCTAA | |
| 428 | ESR1_Y537N_FS03 | AACGTGGTGCCCCTGA | |
| 429 | ESR1_Y537N_FS04 | AACGTGGTGCCCCTTA | |
| 430 | ESR1_Y537N_FS05 | AACGTGGTGCCCCACA | |
| 431 | ESR1_Y537N_FS06 | AACGTGGTGCCCCCCA | |
| 432 | ESR1_Y537N_FS07 | AACGTGGTGCCCCGCA | |
| 433 | ESR1_Y537N_FS08 | AACGTGGTGCCCATCA | |
| 434 | ESR1_Y537N_FS09 | AACGTGGTGCCCGTCA | |
| 435 | ESR1_Y537N_FS10 | AACGTGGTGCCCTTCA | |
| 436 | ESR1_Y537N_RS01 | CTCCAGCAGCAGGT1ATT | 1=C or optional t_BB_dC |
| 437 | ESR1_Y537N_RS02 | CTCCAGCAGCAGGTCAAT | |
| 438 | ESR1_Y537N_RS03 | CTCCAGCAGCAGGTCACT | |
| 439 | ESR1_Y537N_RS04 | CTCCAGCAGCAGGTCAGT | |
| 440 | ESR1_Y537N_RS05 | CTCCAGCAGCAGGTCCTT | |
| 441 | ESR1_Y537N_RS06 | CTCCAGCAGCAGGTCGTT | |
| 442 | ESR1_Y537N_RS07 | CTCCAGCAGCAGGTCTTT | |
| 443 | ESR1_Y537N_RS08 | CTCCAGCAGCAGGTAATT | |
| 444 | ESR1_Y537N_RS09 | CTCCAGCAGCAGGTGATT | |
| 445 | ESR1_Y537N_RS10 | CTCCAGCAGCAGGTTATT | |
| 446 | ESR1_Y537N_RS11 | ATCTCCAGCAGCAGGTTATT | |
| 447 | ESR1_Y537N_RS12 | CATCTCCAGCAGCAGGTTATT | |
| 448 | ESR1_Y537N_RS13 | GCATCTCCAGCAGCAGGTTATT | |
| 449 | ESR1_Y537S_FS01 | ACGTGGTGCCCCTCTC | |
| 450 | ESR1_Y537S_FS02 | ACGTGGTGCCCCTCAC | |
| 451 | ESR1_Y537S_FS03 | ACGTGGTGCCCCTCCC | |
| 452 | ESR1_Y537S_FS04 | ACGTGGTGCCCCTCGC | |
| 453 | ESR1_Y537S_FS05 | ACGTGGTGCCCCTATC | |
| 454 | ESR1_Y537S_FS06 | ACGTGGTGCCCCTGTC | |
| 455 | ESR1_Y537S_FS07 | ACGTGGTGCCCCTTTC | |
| 456 | ESR1_Y537S_FS08 | ACGTGGTGCCCCACTC | |
| 457 | ESR1_Y537S_FS09 | ACGTGGTGCCCCCCTC | |
| 458 | ESR1_Y537S_FS10 | ACGTGGTGCCCCGCTC | |
| 459 | ESR1_Y537S_RS01 | ATCTCCAGCAGCAGGT1AG | 1=C or optional t_BB_dC |
| 460 | ESR1_Y537S_RS02 | ATCTCCAGCAGCAGGTCCG | |
| 461 | ESR1_Y537S_RS03 | ATCTCCAGCAGCAGGTCGG | |
| 462 | ESR1_Y537S_RS04 | ATCTCCAGCAGCAGGTCTG | |
| 463 | ESR1_Y537S_RS05 | ATCTCCAGCAGCAGGTAAG | |
| 464 | ESR1_Y537S_RS06 | ATCTCCAGCAGCAGGTGAG | |
| 465 | ESR1_Y537S_RS07 | ATCTCCAGCAGCAGGTTAG | |
| 466 | ESR1_Y537S_RS08 | ATCTCCAGCAGCAGGACAG | |
| 467 | ESR1_Y537S_RS09 | ATCTCCAGCAGCAGGCCAG | |
| 468 | ESR1_Y537S_RS10 | ATCTCCAGCAGCAGGGCAG | |
| 469 | ESR1_Y537S_RS11 | CATCTCCAGCAGCAGGACAG | |
| 470 | ESR1_Y537S_RS12 | CATCTCCAGCAGCAGGGCAG | |
| 471 | ESR1_Y537S_RS13 | GCATCTCCAGCAGCAGGACAG | |
| 472 | ESR1_Y537S_RS14 | CTCCAGCAGCAGGGCAG | |
| 473 | ESR1K303RWSNP_CF01 | AGAGATGATGGGGAGGGCA | |
| 474 | ESR1K303RWSNP_CF02 | AGATGATGGGGAGGGCA | |
| 475 | ESR1K303RWSNP_CR01 | TCAGCATCCAACAAGGCA | |
| 476 | ESR1K303RWSNP_CR02 | CTCAGCATCCAACAAGGCA | |
| 477 | ESR1K303RWSNP_F_P01 | 1TTGTCCCTGAC2GGCCGACCAGATGGTCA3 | 1=Reporter |
| | | | 2=Quencher |
| | | | 3=Phosphate |
| 478 | ESR1K303RWSNP_FS01 | CGCTCATGATCAAACGCTCTAAGAG | |
| 479 | ESR1K303RWSNP_FS02 | CGCTCATGATCAAACGCTCTAAGCG | |
| 480 | ESR1K303RWSNP_FS03 | CGCTCATGATCAAACGCTCTAAGGG | |
| 481 | ESR1K303RWSNP_FS04 | CGCTCATGATCAAACGCTCTAAGTG | |
| 482 | ESR1K303RWSNP_FS05 | CGCTCATGATCAAACGCTCTAAAAG | |
| 483 | ESR1K303RWSNP_FS06 | CGCTCATGATCAAACGCTCTAACAG | |
| 484 | ESR1K303RWSNP_FS07 | CGCTCATGATCAAACGCTCTAATAG | |
| 485 | ESR1K303RWSNP_FS08 | CGCTCATGATCAAACGCTCTACGAG | |
| 486 | ESR1K303RWSNP_FS09 | CGCTCATGATCAAACGCTCTAGGAG | |
| 487 | ESR1K303RWSNP_FS10 | CGCTCATGATCAAACGCTCTATGAG | |
| 488 | ESR1K303RWSNP_R_P01 | 1TTTGATCATGA2GCGGGCTTGGCCAAAGGTT3 | 1=Reporter |
| | | | 2=Quencher |
| | | | 3=Phosphate |
| 489 | ESR1K303RWSNP_RS01 | ACAAGGCCAGGCTGTTCC | |
| 490 | ESR1K303RWSNP_RS02 | ACAAGGCCAGGCTGTTAC | |
| 491 | ESR1K303RWSNP_RS03 | ACAAGGCCAGGCTGTTGC | |
| 492 | ESR1K303RWSNP_RS04 | ACAAGGCCAGGCTGTTTC | |
| 493 | ESR1K303RWSNP_RS05 | ACAAGGCCAGGCTGTACC | |
| 494 | ESR1K303RWSNP_RS06 | ACAAGGCCAGGCTGTCCC | |
| 495 | ESR1K303RWSNP_RS07 | ACAAGGCCAGGCTGTGCC | |
| 496 | ESR1K303RWSNP_RS08 | ACAAGGCCAGGCTGATCC | |
| 497 | ESR1K303RWSNP_RS09 | ACAAGGCCAGGCTGCTCC | |
| 498 | ESR1K303RWSNP_RS10 | ACAAGGCCAGGCTGGTCC | |
| 499 | SC_ESR1_D01F | GTCTGGCGAGAGATGCAAA | |
| 500 | SC_ESR1_D01FP1 | | 1=Reporter |
| | | | 2=Quencher |
| | | 1CTCTAC2TTTCCTTACCTCCTTCCTTCCA3 | 3=Phosphate |
| 501 | SC_ESR1_D01R | GCCTCAATGAAGACAACTTGAA | |
| 502 | SC_ESR1_D02F | AGGATAAAGTGGATCTGCTGCA | |
| 503 | SC_ESR1_D02R | CCTGGCGTCGATTATCTGAA | |
| 504 | SC_ESR1_D03F | GCTGTTAATTGTCCATGCATAA | |
| 505 | SC_ESR1_D03R | GAAAGGGGAGAACAAGCTAAA | |
| 506 | SC_ESR1_D04F | GAGGAATGGATTTCAATGGAA | |
| 507 | SC_ESR1_D04R | CCCTGGGTCTGTGATCACTAA | |
| 508 | SC_ESR1EX1WTASR1 | GCCACGGACCATGACCATGA | |
| 509 | SC_ESR1EX1WTCRP1 | CTTGAGCTGCGGACGGTTCA | |
| 510 | SC_ESR1EX1WTPRB1 | 1TGGCCCTA2CTGCATCAGATCCAAGG3 | 1=Reporter |
| | | | 2=Quencher |
| | | | 3=Phosphate |
| 511 | SC_ESR1EX2WTASR1 | CAGAGAAAGATTGGCCAGTACC | |
| 512 | SC_ESR1EX2WTASR2 | GCCAGTACCAATGACAAGGGAAG | |
| 513 | SC_ESR1EX2WTASR3 | CCAGGGTGGCAGAGAAAGATT | |
| 514 | SC_ESR1EX2WTCRP1 | CAGACTCCATAATGGTAGCCTGA | |
| 515 | SC_ESR1EX2WTCRP2 | TAATGGTAGCCTGAAGCATAGTCAT | |
| 516 | SC_ESR1EX2WTCRP3 | CACTGCACAGTAGCGAGTCTCCT | |
| 517 | SC_ESR1EX2WTPRB1 | 1TGACAAGG2GAAGTATGGCTATGGAATCT3 | 1=Reporter |
| | | | 2=Quencher |
| | | | 3=Phosphate |
| 518 | SC_ESR1EX2WTPRB2 | 1TGACAAG2G3AAGTATGGCTATGGAATCT4 | 1=Reporter 2=7_Dz_dG 3=Quencher 4=Phosphate |
| 519 | SC_ESR1EX2WTPRB2B | 1CTATGGAA2TCTGCCAAGGAGACTCGCTA3 | 1=Reporter |
| | | | 2=Quencher |
| | | | 3=Phosphate |
| 520 | SC_ESR1EX2WTPRB3 | 1TGACAAGGGA2AGTATGGCTATGGAATCT3 | 1=Reporter |
| | | | 2=Quencher |
| | | | 3=Phosphate |
| 521 | SC_ESR1EX2WTPRB3B | 1CAGTACCA2ATGACAAGGGAAGTATGGCT3 | 1=Reporter |
| | | | 2=Quencher |
| | | | 3=Phosphate |
| 522 | SC_ESR1EX2WTPRB4 | 1TGACAAGG2GAAGTATGGCTATGGAATCT3 | 1=Reporter |
| | | | 2=Quencher |
| | | | 3=Phosphate |
| 523 | SC_ESR1EX5E380QASR1 | CATGATCAGGTCCACCTTCTAC | |
| 524 | SC_ESR1EX5E380QASR2 | CCATGATCAGGTCCACCTTCTAC | |
| 525 | SC_ESR1EX5E380QASR3 | TCCATGATCAGGTCCACCTTCTAC | |
| 526 | SC_ESR1EX5E380QCRP1 | GAGCAAGTTAGGAGCAAACAGTA | |
| 527 | SC_ESR1EX5E380QCRP2 | AGAGCAAGTTAGGAGCAAACAGTA | |
| 528 | SC_ESR1EX5E380QCRP3 | AAGAGCAAGTTAGGAGCAAACAGTA | |
| 529 | SC_ESR1EX5WTASR1 | CATGATCAGGTCCACCTTCTAG | |
| 530 | SC_ESR1EX5WTCRP1 | GAGCAAGTTAGGAGCAAACAGTA | |
| 531 | SC_ESR1EX5WTCRP2 | AGAGCAAGTTAGGAGCAAACAGTA | |
| 532 | SC_ESR1EX5WTCRP3 | AAGAGCAAGTTAGGAGCAAACAGTA | |
| 533 | SC_ESR1EX5WTPBR1 | 1TGCCTG2GCTAGAGATCCTGATGATTGGT3 | 1=Reporter |
| | | | 2=Quencher |
| | | | 3=Phosphate |
| 534 | SC_ESR1EX6L429DVASR1 | GGTAGAGATCTTCGACATGCTGG | |
| 535 | SC_ESR1EX6L429DVASR2 | ATGGTAGAGATCTTCGACATGCTGG | |
| 536 | SC_ESR1EX6V422DVASR1 | ATGTGTAGAGGGCATGGAGATCT | |
| 537 | SC_ESR1EX6V422DVCRP1 | GTTATCAACTCACCAGAATTAAGCAA | |
| 538 | SC_ESR1EX6V422DVCRP2 | TGTTATCAACTCACCAGAATTAAGCAA | |
| 539 | SC_ESR1EX6V422DVCRP3 | GTGTTATCAACTCACCAGAATTAAGCAA | |
| 540 | SC_ESR1EX6V422DVPRB1 | 1CATCTCGGTT2CCGCATGATGAATCTGC3 | 1=Reporter |
| | | | 2=Quencher |
| | | | 3=Phosphate |
| 541 | SC_ESR1EX6WT2ASR1 | GGTAGAGATCTTCGACATGCTGC | |
| 542 | SC_ESR1EX6WT2CRP1 | GTTATCAACTCACCAGAATTAAGCAA | |
| 543 | SC_ESR1EX6WTASR1 | GGTAGAGATCTTCGACATGCTGC | |
| 544 | SC_ESR1EX6WTCRP1 | TCACCAGAATTAAGCAAAATAATAGATT | |
| 545 | SC_ESR1EX6WTPRB1 | 1CATCTCGGTT2CCGCATGATGAATCTGCA3 | 1=Reporter |
| | | | 2=Quencher |
| | | | 3=Phosphate |
| 546 | SC_ESR1EX7S463PASR1 | ATTCAGGACACATTTCTGC | |
| 547 | SC_ESR1EX7WTASR1 | CATTCAGGAGTGTACACATTTCTGT | |
| 548 | SC_ESRTEX7WTCRP1 | ATCAGGTGGATCAAAGTGTCTGT | |
| 549 | SC_ESR1EX7WTPRB1 | 1TCTCTG2GAAGAGAAGGACCATATCCACC3 | 1=Reporter |
| | | | 2=Quencher |
| | | | 3=Phosphate |
| 550 | SC_ESR1EX8D538GASR1 | ACGTGGTGCCCCTCTATGG | |
| 551 | SC_ESR1EX8L536HASR1 | CAAGAACGTGGTGCCCCA | |
| 552 | SC_ESR1EX8L536HASR2 | TGCAAGAACGTGGTGCCCCA | |
| 553 | SC_ESR1EX8L536HASR3 | GTGCAAGAACGTGGTGCCCCA | |
| 554 | SC_ESR1EX8L536HASR4 | GTGCAAGAACGTGGTGCCACA | |
| 555 | SC_ESR1EX8L536PASR1 | CAAGAACGTGGTGCCCCC | |
| 556 | SC_ESR1EX8L536QASR1 | CAAGAACGTGGTGCCCCAG | |
| 557 | SC_ESR1EX8L536RASR1 | CAAGAACGTGGTGCCCCG | |
| 558 | SC_ESR1EX8P535H1ASR1 | TGAAGTGCAAGAACGTGGTGCA | |
| 559 | SC_ESR1EX8P535H1ASR2 | CATGAAGTGCAAGAACGTGGTGCA | |
| 560 | SC_ESR1EX8P535H2ASR1 | TGAAGTACAAGAACGTGGTGCA | |
| 561 | SC_ESR1EX8P535H2ASR2 | ATGAAGTACAAGAACGTGGTGCA | |
| 562 | SC_ESR1EX8P535H2ASR3 | CATGAAGTACAAGAACGTGGTGCA | |
| 563 | SC_ESR1EX8P535H2ASR4 | TGAAGTGCAAGAACGTGATGCA | |
| 564 | SC_ESR1EX8P535H2ASR5 | AGCATGAAGTACAAGAACGTGGTGCA | |
| 565 | SC_ESR1EX8P535H3ASR1 | TGAAGTGCAAGAACGTGGTACA | |
| 566 | SC_ESR1EX8P535H3ASR2 | ATGAAGTGCAAGAACGTGGTACA | |
| 567 | SC_ESR1EX8P535H3ASR3 | CATGAAGTGCAAGAACGTGGTACA | |
| 568 | SC_ESR1EX8V534EASR1 | CATGAAGTGCAAGAACGTGGA | |
| 569 | SC_ESR1EX8V534EASR2 | GCATGAAGTGCAAGAACGTGGA | |
| 570 | SC_ESR1EX8V534EASR3 | AGCATGAAGTGCAAGAACGTGGA | |
| 571 | SC_ESR1EX8WTASR1 | ACGTGGTGCCCCTCTATGAC | |
| 572 | SC_ESR1EX8WTCRP1 | GCTTTGGTCCGTCTCCTCC | |
| 573 | SC_ESR1EX8WTPRB1 | 1CTGCTG2GAGATGCTGGACGCCCACC3 | 1=Reporter |
| | | | 2=Quencher |
| | | | 3=Phosphate |
| 574 | SC_ESR1EX8Y537CASR1 | AGAACGTGGTGCCCCTCTG | |
| 575 | SC_ESR1EX8Y537NASR1 | AGAACGTGGTGCCCCTCA | |
| 576 | SC_ESR1EX8Y537NASR2 | AAGAACGTGGTGCCCCTCA | |
| 577 | SC_ESR1EX8Y537NASR3 | CAAGAACGTGGTGCCCCTCA | |
| 578 | SC_ESR1EX8Y537SASR1 | AGAACGTGGTGCCCCTCTC | |
| 579 | ESR1_S463P_CF02 | CTCCTAGACCTCATCCTCTTTGA | |

### Oligonucleotide selection for high sensitivity and specificity detection of mutants

Primers were selected to ensure that mutant signal could be sensitively and specifically detected compared to wild type. A primer pair and probe designed to be specific for the ESR1 K303R mutation and for the L536_D538>P mutation were used detect mutant template added to wild type DNA at a ratio of 1:1600 (100 copies of mutant DNA in 160,000 copies of wild type) and in wild type DNA alone. Allele specific amplification and detection were carried out on the **cobas z** 480. The data are expressed in Ct curves plotting amplification cycles vs fluorescence signal (indicating detection of the amplification product)

Figure 1A shows a reaction specific for K303R with no discrimination. The selected primers and probe amplify and detect signal from the mutant and wild type samples at essentially the same level. Figure 1B shows a reaction specific for K303R with better discrimination. The Ct's of the two wild type samples are shifted to the right by at least 2 cycles. Figure 1C shows a much more specific reaction for L536_D538>P that is also sensitive enough to detect mutant DNA at a ratio of at least 1:1600. The wild type samples are not detected at all, while the mutant samples have a Ct around 35-38.

A similar comparison is shown in Figures 2A, 2B, and 2C with primers and probes designed to be specific for the L536R mutation. Figure 2A shows a reaction with no discrimination using the L536R_RS01 allele-specific primer. Wild type is amplified at essentially the same level as mutant. Figure 2B shows a reaction with better discrimination using the L536R_RS09 allele-specific primer, where wild type has a Ct delayed by at least 5 cycles compared to mutant. Figure 2C shows a reaction with good discrimination using the L536R_RS04 allele-specific primer. Wild type has a Ct over 50 cycles, considered not detected, compared to a mutant Ct well in the detectable range around 35.

### Specificity of multiplex mutation detection

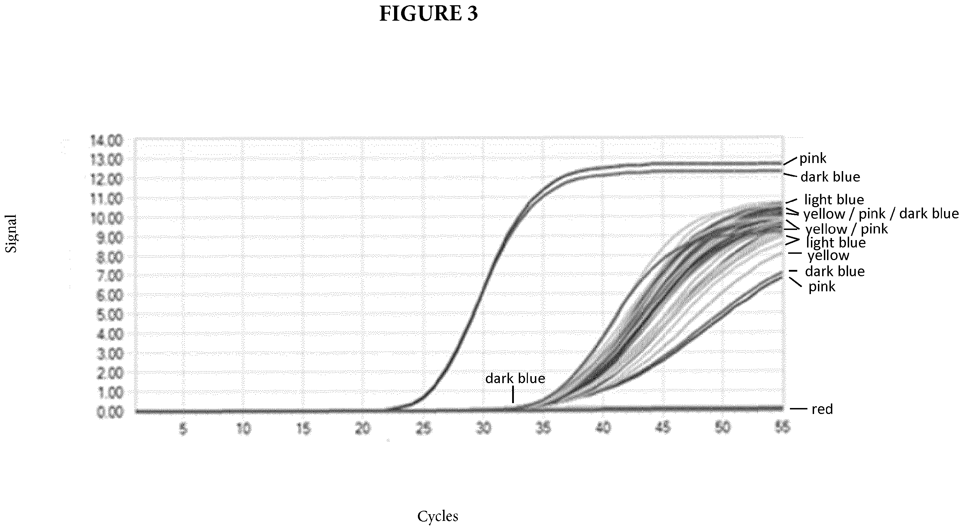

The primer pair and probe sets were tested in multiplex to determine specificity and ensure that they would not detect other ESR1 mutations. Separate sample mixtures were prepared for each of the 18 mutants listed in Table 1, with mutant DNA added 1:1 with wild type (5000 copies of each). Primer pairs and probes specific for all 18 mutations were added. Figure 3 shows an exemplary Ct curve with high specificity for ESR1 P535H. The two left-most curves with a Ct of around 25-30 represent specific detection of the ESR1 P535H mutation present in the sample (indicated with light arrow). The Ct's for the other primer pair and probe sets have a much more delayed Ct (indicated between two black arrows).

### Assay linearity

Linearity of detection was also detected across a range of template concentrations for each mutation. As an example primer pair and probe specific for ESR1 S341L were used to amplify 5-5000 copies mutant template in a background of 10,000 wild type copies. The results are shown in Table 3, and show that the assay is highly linear.

**Table 3**

| Mutant copy number | Ct |
|---|---|
| 5 | 33.04 |
| 50 | 29.32 |
| 500 | 26.23 |
| 5000 | 22.89 |
| Slope | -3.36 |
| R² | 0.9987 |

### Detection of ESR1 mutations in contrived plasma samples

The assay was tested in plasma background to determine if plasma components would interfere. Mutant DNA was added at 1000 copies into 2 ml normal (wild type) plasma. DNA was extracted with the **cobas®** DNA Sample Preparation Kit. DNA equivalent to that from 0.5 ml plasma was added to each PCR reaction. Primer pairs and probe sets were added corresponding to each mutation. Exemplary results are shown in Table 4. The data show that mutant DNA can be sensitively detected in wild type plasma background.

**Table 4**

| **Mutation** | **Average mutant Ct with mutant added** | **Average mutant Ct in plasma only sample** |
|---|---|---|
| L536Q | 28.62 | 45.20 |
| V422delV | 28.55 | ND |
| L536R | 28.55 | 45.20 |
| L536H | 28.40 | 45.20 |
| Y537S | 28.41 | ND |
| E380Q | 28.28 | ND |
| L536P | 29.41 | 45.20 |

### Selected oligonucleotides

Allele-specific primers that show good specificity for mutant sequence were selected based on the criteria above. These are shown in Table 5 below with the primer name, and SEQ ID NO in parenthesis. Bolded entries indicate primers that show selectivity and specificity in the assay configuration in Table 6 below, though other combinations also perform well.

**Table 5: Selected allele-specific primers**

| **Mutation** | **Selected allele-specific primers** |
|---|---|
| K303R | FS02 (479), **FS04 (481),** FS07 (484) |
| S341L | FS02 (239), FS03 (240), FS05 (242), **FS08 (245),** FS09 (246), FS10 (247), RS04 (253), RS05 (254), RS06 (255), RS07 (256), RS08 (257), RS09 (258), RS10 (259) |
| E380Q | FS01 (32), FS03 (34), FS05 (36), FS06 (37), FS07 (38), FS08 (39), FS09 (40), FS10 (41), **FS11 (42)** |
| V422DELV | FS01 (287), FS08 (294), FS09 (295), FS10 (296), FS12, FS14 (297), **FS15 (298)** |
| L429V | FS06 (66), FS08 (68), FS09 (69), **FS10 (70),** RS01 (72), RS02 (73), RS04 (75), RS05 (76), RS06 (77), RS07 (78), RS08 (79), RS09 (80), RS10 (81) |
| S463P | **FS02 (264),** FS03 (265), FS05 (267), FS06 (268), FS07 (269), FS08 (270), FS09 (271), FS10 (272), RS08 (281), RS09 (282), RS10 (283), RS11 (275), RS12 |
| V533M | FS01 (322), FS08 (329), FS09 (330), RS08 (345), RS09 (346), RS10 (347), RS11 (348), RS12 (349), **RS13 (350),** RS15 (352), RS16 (353), RS19 |
| V534E | FS01 (364), FS02 (365), FS04 (367), FS05 (368), FS07 (370), FS08 (371), FS10 (373), RS01 (378), **RS11 (388),** RS15 (391), RS16 (392), RS17 (393) |
| P535H | FS01 (181), FS08 (188), FS09 (189), FS10 (190), RS01 (191), RS08 (198), RS09 (199), RS10 (200), RS13 (203), RS14 (204), RS15 (205), RS18 (208), RS19 (209), RS22 (212), RS35 (225), RS36 (226), RS38 (228), RS39 (229), **RS41 (231)** |
| L536H | FS01 (96), FS06 (101), FS09 (104), FS10 (105), RS02 (107), RS05 (110), RS06 (111), RS07 (112), **RS08 (113),** RS09 (114), RS10 (115), RS11 (116), RS12 (117) |
| L536P | **RS04 (134),** RS05 (135), RS07 (137) |
| L536Q | FS01 (141), RS01 (151), RS04 (154), RS05 (155), RS07 (157), **RS08 (158),** RS09 (159), RS10 (160) |
| L536R | FS01 (161), RS02 (172), RS04 (174), RS05 (175), RS06 (176), RS07 (177), **RS08 (178),** RS09 (179), RS10 (180) |
| Y537C | FS01 (397), RS01 (408), **RS08 (415),** RS09 (416), RS10 (417), RS11 (418), RS12 (419), RS13 (420), RS19 (424) |
| Y537N | FS01 (426), RS01 (436), RS06 (441), RS10 (445), RS11 (446), **RS12 (447),** RS13 (448), RS14 |
| Y537S | FS01 (449), RS01 (459), RS08 (466), RS09 (467), RS10 (468), **RS11 (469),** RS12 (470), RS13 (471), RS14 (472) |
| D538G | RS01 (14), RS08 (21), RS09 (22), RS10 (23), **RS11 (24),** RS12 (25) |
| L536_D538 >P | RS01 (84), RS02 (85), RS03 (86), RS04 (87), RS05 (88), RS06 (89), **RS07 (90),** RS08 (91), RS09 (92), RS10 (93) |

In the case of exon 8 RS primers, any exon 8 CF primer can be used, for example, V533M_CF03 (SEQ ID NO:314) or Y537C_CF01 (SEQ ID NO:394). Any exon 8 probe can be used, for example Y537C_R_P01 (SEQ ID NO:407). In general, for any FS allele-specific primer, any corresponding CR primer and F probe can be used. Similarly for any RS allele-specific primer, any corresponding CF primer and R probe can be used. Particular examples include K303R_CR02 (SEQ ID NO:476) and K303R_F_P01 (SEQ ID NO:477); S341L_CR02 (SEQ ID NO:235) and S341L_F_P02 (SEQ ID NO:237); E380QCRP3 (SEQ ID NO:528) and E380Q_F_P01 (SEQ ID NO:31); V422delV_CR01 (SEQ ID NO:285) and V422delV_F_P01 (SEQ ID NO:286); L429V_CR01 (SEQ ID NO:59) and L429_F_P01 (SEQ ID NO:60); and S463P_CR01 (SEQ ID NO:261) and S463P_F_P01 (SEQ ID NO:262).

### Example assay configuration

We sought to design a highly multiplexed assay to detect the maximum number of mutations in a minimal number of reaction vessels. Examples of three-vessel assays are shown below in Tables 6 and 7. Exon 8 mutations are grouped together to allow for use of a common forward primer and probe. If desired, one of skill will understand how to identify the exact exon 8 mutation(s) that results in a mutation positive signal, *e.g.,* using sequencing or PCR with mutation-specific probes.

**Table 6: Exemplary assay configuration for allele-specific multiplex ESR1 mutation**

| **Reporter** | **1** | **2** | **3** |
|---|---|---|---|
| FAM | L536H/ L536P/ L536Q/ L536R/ D538G | Y537C/ Y537N/ Y537S/ L536_D538>P | V533M/ V534E/ P535H |
| HEX | S463P | E380Q | S341L |
| JA270 | V422DELV | K303R | L429V |
| CY5.5 | IC | IC | IC |

**Table 7: Exemplary assay configuration for allele-specific multiplex ESR1 mutation**

| **Reporter** | **1** | **2** | **3** |
|---|---|---|---|
| FAM | L536H/ L536P/ L536Q/ L536R/ L536_D538>P | Y537C/ Y537N/ Y537S/ D538G | V533M/ V534E/ P535H |
| HEX | S463P | S341L | E380Q |
| JA270 | K303R | L429V | V422DELV |
| CY5.5 | IC | IC | IC |

One of skill will understand that different configurations can be made, depending on the mutations of interest and availability of fluorescence channels. For example, the number of tubes can be increased to allow for individual detection of exon 8 mutations. As the number of fluorescence channels increases, the number of mutations that can be included in a single tube will increase.

### Detection of E380Q mutation from FFPET samples in multiplex reactions

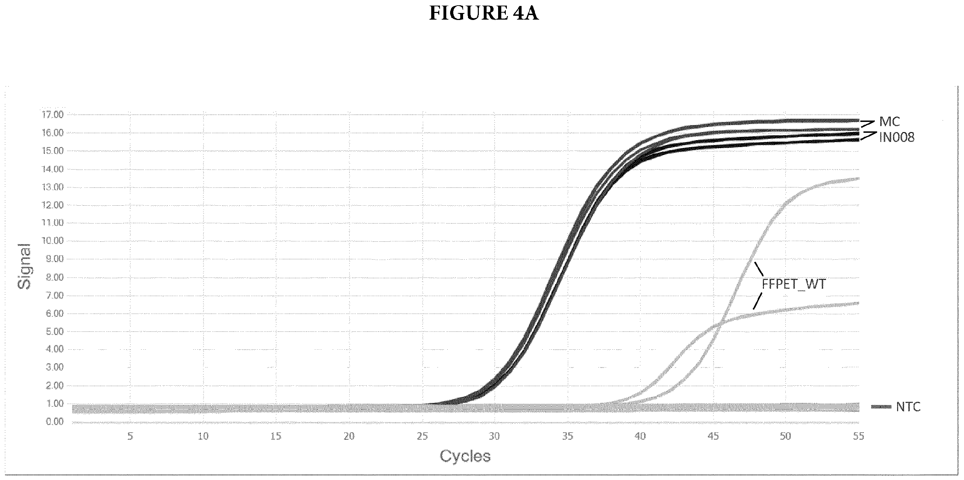

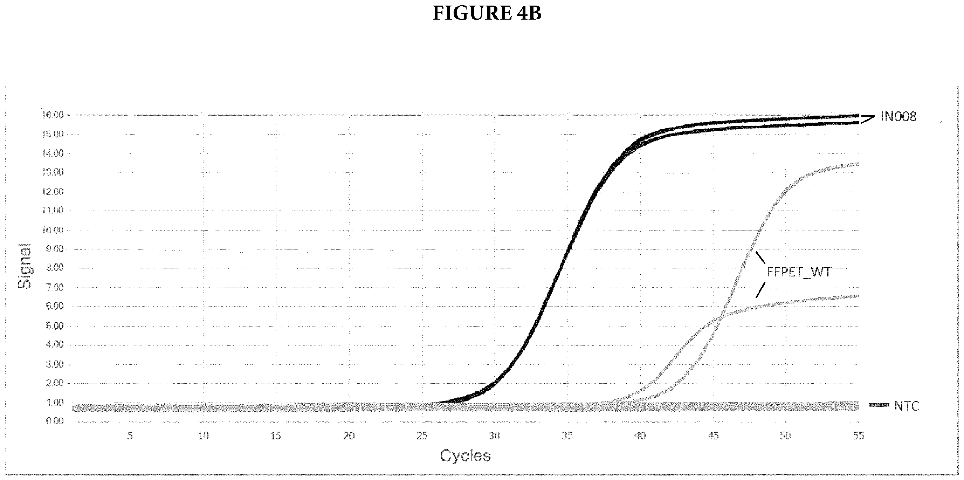

DNA from 142 FFPET samples (breast, lymph node, lung, bone) from cancer patients was extracted using the **cobas®** DNA Sample Preparation Kit. DNA (50 ng/ reaction) was analyzed for ESR1 mutations by the multiplex allele-specific PCR as described herein (performed in duplicate). Results are shown in Figures 4A and 4B. One sample (IN008) was found positive for the E380Q mutation, and has a similar amplification growth curve to the E380Q mutation positive control (MC) (see Figure 4A). Figure 4B shows the same data without the E380Q mutation positive control. Results were confirmed using ddPCR.

The E380Q mutation positive sample shows a profile that is clearly distinguishable from E380 wild type and non-template control. The results also show that the multiplex assays described herein are effective for detecting specific mutations in DNA from FFPET clinical samples.

### Detection of D538G mutation from plasma samples in multiplex reactions

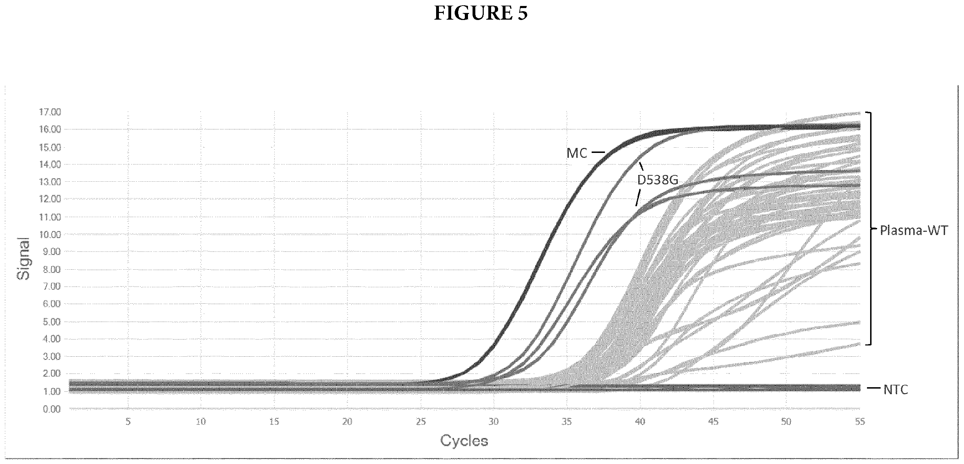

Clinical plasma samples (2 ml plasma) were obtained from 103 post-menopausal metastatic breast cancer patients who relapsed during treatment with an aromatase inhibitor, or within 12 months after discontinuation. DNA was extracted using the **cobas®** cfDNA Sample Preparation Kit and analyzed for ESR1 mutations by multiplex allele-specific PCR as described herein. Three samples tested positive for D538G, as shown in Figure 5. Figure 5 shows results from 51 of the samples, 3 D538G positive and 48 D538 wild type. D538G mutation positive and non-template controls are also shown, and the D538G positive samples show profiles that are clearly distinguishable. Results were confirmed using ddPCR.

The results show that the multiplex assays described herein are effective for detecting specific mutations in DNA from non-invasive (plasma) clinical samples.

## Claims

1. A kit comprising three vessels, wherein:
(i) a first vessel holds a primer pair specific for ESR1 V422DelV, a primer pair specific for ESR1 S463P, a primer pair specific for ESR1 L536H, a primer pair specific for *ESR1 L536P, a primer pair specific for ESR1 L536Q, a primer pair specific for ESR1 L536R, and a primer pair specific for ESR1 D538G;
(ii) a second vessel holds a primer pair specific for ESR1 K303R, a primer pair specific for ESR1 E380Q, a primer pair specific for ESR1 L536_D538>P, a primer pair specific for ESR1 Y537C, a primer pair specific for ESR1 Y537N, and a primer pair specific for ESR1 Y537S; and
(iii) a third vessel holds a primer pair specific for ESR1 S341L, a primer pair specific for ESR1 L429V, a primer pair specific for ESR1 V533M, a primer pair specific for ESR1 V534E, and a primer pair specific for ESR1 P535H;
each of the vessels holding two or more probes specific for a different sequence in the ESR1 gene, wherein each probe is labeled with a fluorophore and quencher;
a primer pair specific for an internal control sequence; and
a probe specific for the internal control sequence and labeled with a fluorophore and quencher; and
wherein the vessels comprise oligonucleotides having the sequences of SEQ ID NOs:24, 113, 134, 158, 178, 264, 261, 262, 285, 286, 298, 314, 394, 407, 31, 42, 90, 415, 447, 469, 476, 477, 481, 528, 59, 60, 70, 231, 235, 237, 245, 350, and 388.

2. The kit of claim 1, wherein each of the vessels holds three to four probes.

3. The kit of any one of the foregoing claims, wherein at least one primer includes a modified, non-naturally occurring nucleotide.

4. The kit of any one of the foregoing claims, wherein each vessel further holds a thermostable DNA polymerase.

5. A method for determining the presence or absence of two or more ESR1 mutations in a sample from an individual, comprising:
(i) obtaining a sample from the individual;
(ii) carrying out multiplex allele-specific PCR to determine the presence or absence of two or more ESR1 mutations, wherein the multiplex allele-specific PCR is carried out in three vessels, wherein the presence or absence of ESR1 mutations is determined in the three vessels as follows:
(i) ESR1 V422DelV, ESR1 S463P, ESR1 L536H, ESR1 L536P, ESR1 L536Q, ESR1 L536R, and ESR1 D538G in a first vessel;
(ii) ESR1 K303R, ESR1 E380Q, ESR1 L536_D538>P, ESR1 Y537C, ESR1 Y537N, and ESR1 Y537S in a second vessel;
(iii) ESR1 S341L, ESR1 L429V, ESR1 V533M, ESR1 V534E, and ESR1 P535H in a third vessel; and
wherein the vessels comprise oligonucleotides having the sequences of SEQ ID NOs:24, 113, 134, 158, 178, 264, 261, 262, 285, 286, 298, 314, 394, 407, 31, 42, 90, 415, 447, 469, 476, 477, 481, 528, 59, 60, 70, 231, 235, 237, 245, 350, and 388.

6. The method of claim 5, wherein the individual has breast cancer and is undergoing hormone therapy.

7. The method of claim 5 or 6, comprising carrying out multiplex allele-specific PCR to determine the presence or absence of ten or more ESR1 mutations.

## Patentansprüche

1. Kit, umfassend drei Gefäße, wobei:
(i) ein erstes Gefäß ein für ESR1 V422DelV spezifisches Primer-Paar, ein für ESR1 S463P spezifisches Primer-Paar, ein für ESR1 L536H spezifisches Primer-Paar, ein für ESR1 L536P spezifisches Primer-Paar, ein für ESR1 L536Q spezifisches Primer-Paar, ein für ESR1 L536R spezifisches Primer-Paar und ein für ESR1 D538G spezifisches Primer-Paar beinhaltet;
(ii) ein zweites Gefäß ein für ESR1 K303R spezifisches Primer-Paar, ein für ESR1 E380Q spezifisches Primer-Paar, ein für ESR1 L536_D538>P spezifisches Primer-Paar, ein für ESR1 Y537C spezifisches Primer-Paar, ein für ESR1 Y537N spezifisches Primer-Paar und ein für ESR1 Y537S spezifisches Primer-Paar beinhaltet; und
(iii) ein drittes Gefäß ein für ESR1 S341L spezifisches Primer-Paar, ein für ESR1 L429V spezifisches Primer-Paar, ein für ESR1 V533M spezifisches Primer-Paar, ein für ESR1 V534E spezifisches Primer-Paar und ein für ESR1 P535H spezifisches Primer-Paar beinhaltet;
wobei jedes der Gefäße zwei oder mehr Sonden, die für eine andere Sequenz in dem ESR1-Gen spezifisch sind, beinhaltet, wobei jede Sonde mit einem Fluorophor und einem Quencher markiert ist;
ein für eine interne Kontrollsequenz spezifisches Primer-Paar und
eine Sonde, die für die interne Kontrollsequenz spezifisch und mit einem Fluorophor und einem Quencher markiert ist; und
wobei die Gefäße Oligonukleotide mit den Sequenzen von SEQ ID NO:24, 113, 134, 158, 178, 264, 261, 262, 285, 286, 298, 314, 394, 407, 31, 42, 90, 415, 447, 469, 476, 477, 481, 528, 59, 60, 70, 231, 235, 237, 245, 350 und 388 umfassen.

2. Kit nach Anspruch 1, wobei jedes der Gefäße drei bis vier Sonden beinhaltet.

3. Kit nach einem der vorstehenden Ansprüche, wobei mindestens ein Primer ein modifiziertes, nicht natürlich vorkommendes Nukleotid einschließt.

4. Kit nach einem der vorstehenden Ansprüche, wobei jedes Gefäß ferner eine thermostabile DNA-Polymerase beinhaltet.

5. Verfahren zum Bestimmen des Vorhandenseins oder Fehlens von zwei oder mehr ESR1-Mutationen in einer Probe von einem Individuum, umfassend:
(i) das Erhalten einer Probe von dem Individuum;
(ii) das Durchführen einer multiplexen Allel-spezifischen PCR zum Bestimmen der Anwesenheit oder Abwesenheit von zwei oder mehr ESR1-Mutationen, wobei die multiplexe Allel-spezifische PCR in drei Gefäßen durchgeführt wird, wobei die Anwesenheit oder Abwesenheit von ESR1-Mutationen in den drei Gefäßen wie folgt bestimmt wird:
(i) ESR1 V422DelV, ESR1 S463P, ESR1 L536H, ESR1 L536P, ESR1 L536Q, ESR1 L536R und ESR1 D538G in einem ersten Gefäß;
(ii) ESR1 K303R, ESR1 E380Q, ESR1 L536_D538>P, ESR1 Y537C, ESR1 Y537N und ESR1 Y537S in einem zweiten Gefäß;
(iii) ESR1 S341L, ESR1 L429V, ESR1 V533M, ESR1 V534E und ESR1 P535H in einem dritten Gefäß; und
wobei die Gefäße Oligonukleotide mit den Sequenzen von SEQ ID NO:24, 113, 134, 158, 178, 264, 261, 262, 285, 286, 298, 314, 394, 407, 31, 42, 90, 415, 447, 469, 476, 477, 481, 528, 59, 60, 70, 231, 235, 237, 245, 350 und 388 umfassen.

6. Verfahren nach Anspruch 5, wobei das Individuum Brustkrebs hat und sich einer Hormontherapie unterzieht.

7. Verfahren nach Anspruch 5 oder 6, umfassend das Durchführen von multiplexer Allelspezifischer PCR zum Bestimmen der Anwesenheit oder Abwesenheit von zehn oder mehr ESR1-Mutationen.

## Revendications

1. Kit comprenant trois récipients, dans lequel :
(i) un premier récipient contient une paire d'amorces spécifique pour ESR1 V422DelV, une paire d'amorces spécifique pour ESR1 S463P, une paire d'amorces spécifique pour ESR1 L536H, une paire d'amorces spécifique pour ESR1 L536P, une paire d'amorces spécifique pour ESR1 L536Q, une paire d'amorces spécifique pour ESR1 L536R et une paire d'amorces spécifique pour ESR1 D538G ;
(ii) un deuxième récipient contient une paire d'amorces spécifique pour ESR1 K303R, une paire d'amorces spécifique pour ESR1 E380Q, une paire d'amorces spécifique pour ESR1 L536_D538>P, une paire d'amorces spécifique pour ESR1 Y537C, une paire d'amorces spécifique pour ESR1 Y537N et une paire d'amorces spécifique pour ESR1 Y537S ; et
(iii) un troisième récipient contient une paire d'amorces spécifique pour ESR1 S341L, une paire d'amorces spécifique pour ESR1 L429V, une paire d'amorces spécifique pour ESR1 V533M, une paire d'amorces spécifique pour ESR1 V534E et une paire d'amorces spécifique pour ESR1 P535H ;
chacun des récipients contenant deux sondes ou plus spécifiques pour une séquence différente dans le gène ESR1, dans lequel chaque sonde est marquée avec un fluorophore et un extincteur ;
une paire d'amorces spécifique pour une séquence de contrôle interne ; et
une sonde spécifique de la séquence de contrôle interne et marquée avec un fluorophore et un extincteur ; et
dans lequel les récipients comprennent les oligonucléotides ayant les séquences de SEQ ID NO : 24, 113, 134, 158, 178, 264, 261, 262, 285, 286, 298, 314, 394, 407, 31, 42, 90, 415, 447, 469, 476, 477, 481, 528, 59, 60, 70, 231, 235, 237, 245, 350 et 388.

2. Kit selon la revendication 1, dans lequel chacun des récipients contient trois à quatre sondes.

3. Kit selon l'une quelconque des revendications précédentes, dans lequel au moins une amorce inclut un nucléotide modifié, non naturel.

4. Kit selon l'une quelconque des revendications précédentes, dans lequel chaque récipient contient en outre une ADN polymérase thermostable.

5. Procédé de détermination de la présence ou de l'absence de deux mutations ESR1 ou plus dans un échantillon d'un individu, comprenant :
(i) l'obtention d'un échantillon de l'individu ;
(ii) la mise en œuvre d'une PCR multiplex spécifique à des allèles pour déterminer la présence ou l'absence de deux mutations ESR1 ou plus, dans lequel la PCR multiplex spécifique à des allèles est mise en œuvre dans trois récipients, dans lequel la présence ou l'absence de mutations ESR1 est déterminée dans les trois récipients de la façon suivante :
(i) ESR1 V422DelV, ESR1 S463P, ESR1 L536H, ESR1 L536P, ESR1 L536Q, ESR1 L536R et ESR1 D538G dans un premier récipient ;
(ii) ESR1 K303R, ESR1 E380Q, ESR1 L536_D538>P, ESR1 Y537C, ESR1 Y537N et ESR1 Y537S dans un deuxième récipient ;
(iii) ESR1 S341L, ESR1 L429V, ESR1 V533M, ESR1 V534E et ESR1 P535H dans un troisième récipient ; et
dans lequel les récipients comprennent les oligonucléotides ayant les séquences de SEQ ID NO : 24, 113, 134, 158, 178, 264, 261, 262, 285, 286, 298, 314, 394, 407, 31, 42, 90, 415, 447, 469, 476, 477, 481, 528, 59, 60, 70, 231, 235, 237, 245, 350 et 388.

6. Procédé selon la revendication 5, dans lequel l'individu a un cancer du sein et suit une hormonothérapie.

7. Procédé selon la revendication 5 ou 6, comprenant la mise en œuvre d'une PCR multiplex spécifique à des allèles pour déterminer la présence ou l'absence de dix mutations ESR1 ou plus.


### SEQUENCE LISTING

<110> Roche Diagnostics GmbH F. Hoffmann-La Roche AG Roche Molecular Systems, Inc.
<120> MULTIPLEX ALLELE SPECIFIC PCR ASSAYS FOR DETECTION OF ESTROGEN RECEPTOR ESR1 MUTATIONS
<130> P33815-WO-KOE
<150> 62/376,799
   <151> 2016-08-18
<160> 579
<170> Patent In version 3.5
<210> 1
   <211> 23
   <212> DNA
   <213> Artificial Sequence
<220>
   <221> source
   <223> /note="Description of Artificial Sequence: Synthetic oligonucleotide"
<400> 1
   tggccctact gcatcagatc caa 23
<210> 2
   <211> 22
   <212> DNA
   <213> Artificial Sequence
<220>
   <221> source
   <223> /note="Description of Artificial Sequence: Synthetic oligonucleotide"
<400> 2
   atcagatcca agggaacgag ct 22
<210> 3
   <211> 21
   <212> DNA
   <213> Artificial Sequence
<220>
   <221> source
   <223> /note="Description of Artificial Sequence: Synthetic oligonucleotide"
<400> 3
   gcttgctgct gtccaggtac a 21
<210> 4
   <211> 18
   <212> DNA
   <213> Artificial Sequence
<220>
   <221> source
   <223> /note="Description of Artificial Sequence: Synthetic oligonucleotide"
<400> 4
   cgtggtgccc ctctatgg 18
<210> 5
   <211> 18
   <212> DNA
   <213> Artificial Sequence
<220>
   <221> source
   <223> /note="Description of Artificial Sequence: Synthetic oligonucleotide"
<400> 5
   cgtggtgccc ctctatag 18
<210> 6
   <211> 18
   <212> DNA
   <213> Artificial Sequence
<220>
   <221> source
   <223> /note="Description of Artificial Sequence: Synthetic oligonucleotide"
<400> 6
   cgtggtgccc ctctatcg 18
<210> 7
   <211> 18
   <212> DNA
   <213> Artificial Sequence
<220>
   <221> source
   <223> /note="Description of Artificial Sequence: Synthetic oligonucleotide"
<400> 7
   cgtggtgccc ctctattg 18
<210> 8
   <211> 18
   <212> DNA
   <213> Artificial Sequence
<220>
   <221> source
   <223> /note="Description of Artificial Sequence: Synthetic oligonucleotide"
<400> 8
   cgtggtgccc ctctaagg 18
<210> 9
   <211> 18
   <212> DNA
   <213> Artificial Sequence
<220>
   <221> source
   <223> /note="Description of Artificial Sequence: Synthetic oligonucleotide"
<400> 9
   cgtggtgccc ctctacgg 18
<210> 10
   <211> 18
   <212> DNA
   <213> Artificial Sequence
<220>
   <221> source
   <223> /note="Description of Artificial Sequence: Synthetic oligonucleotide"
<400> 10
   cgtggtgccc ctctaggg 18
<210> 11
   <211> 18
   <212> DNA
   <213> Artificial Sequence
<220>
   <221> source
   <223> /note="Description of Artificial Sequence: Synthetic oligonucleotide"
<400> 11
   cgtggtgccc ctctctgg 18
<210> 12
   <211> 18
   <212> DNA
   <213> Artificial Sequence
<220>
   <221> source
   <223> /note="Description of Artificial Sequence: Synthetic oligonucleotide"
<400> 12
   cgtggtgccc ctctgtgg 18
<210> 13
   <211> 18
   <212> DNA
   <213> Artificial Sequence
<220>
   <221> source
   <223> /note="Description of Artificial Sequence: Synthetic oligonucleotide"
<400> 13
   cgtggtgccc ctctttgg 18
<210> 14
   <211> 18
   <212> DNA
   <213> Artificial Sequence
<220>
   <221> source
   <223> /note="Description of Artificial Sequence: Synthetic oligonucleotide"
<400> 14
   gcatctccag cagcaggc 18
<210> 15
   <211> 18
   <212> DNA
   <213> Artificial Sequence
<220>
   <221> source
   <223> /note="Description of Artificial Sequence: Synthetic oligonucleotide"
<400> 15
   gcatctccag cagcagac 18
<210> 16
   <211> 18
   <212> DNA
   <213> Artificial Sequence
<220>
   <221> source
   <223> /note="Description of Artificial Sequence: Synthetic oligonucleotide"
<400> 16
   gcatctccag cagcagcc 18
<210> 17
   <211> 18
   <212> DNA
   <213> Artificial Sequence
<220>
   <221> source
   <223> /note="Description of Artificial Sequence: Synthetic oligonucleotide"
<400> 17
   gcatctccag cagcagtc 18
<210> 18
   <211> 18
   <212> DNA
   <213> Artificial Sequence
<220>
   <221> source
   <223> /note="Description of Artificial Sequence: Synthetic oligonucleotide"
<400> 18
   gcatctccag cagcaagc 18
<210> 19
   <211> 18
   <212> DNA
   <213> Artificial Sequence
<220>
   <221> source
   <223> /note="Description of Artificial Sequence: Synthetic oligonucleotide"
<400> 19
   gcatctccag cagcacgc 18
<210> 20
   <211> 18
   <212> DNA
   <213> Artificial Sequence
<220>
   <221> source
   <223> /note="Description of Artificial Sequence: Synthetic oligonucleotide"
<400> 20
   gcatctccag cagcatgc 18
<210> 21
   <211> 18
   <212> DNA
   <213> Artificial Sequence
<220>
   <221> source
   <223> /note="Description of Artificial Sequence: Synthetic oligonucleotide"
<400> 21
   gcatctccag cagccggc 18
<210> 22
   <211> 18
   <212> DNA
   <213> Artificial Sequence
<220>
   <221> source
   <223> /note="Description of Artificial Sequence: Synthetic oligonucleotide"
<400> 22
   gcatctccag cagcgggc 18
<210> 23
   <211> 18
   <212> DNA
   <213> Artificial Sequence
<220>
   <221> source
   <223> /note="Description of Artificial Sequence: Synthetic oligonucleotide"
<400> 23
   gcatctccag cagctggc 18
<210> 24
   <211> 18
   <212> DNA
   <213> Artificial Sequence
<220>
   <221> source
   <223> /note="Description of Artificial Sequence: Synthetic oligonucleotide"
<400> 24
   gcatctccag cagcgggc 18
<210> 25
   <211> 20
   <212> DNA
   <213> Artificial Sequence
<220>
   <221> source
   <223> /note="Description of Artificial Sequence: Synthetic oligonucleotide"
<400> 25
   cagcatctcc agcagctggc 20
<210> 26
   <211> 20
   <212> DNA
   <213> Artificial Sequence
<220>
   <221> source
   <223> /note="Description of Artificial Sequence: Synthetic oligonucleotide"
<400> 26
   cagcatctcc agcagcaggc 20
<210> 27
   <211> 20
   <212> DNA
   <213> Artificial Sequence
<220>
   <221> source
   <223> /note="Description of Artificial Sequence: Synthetic oligonucleotide"
<400> 27
   cagcatctcc agcagcgggc 20
<210> 28
   <211> 16
   <212> DNA
   <213> Artificial Sequence
<220>
   <221> source
   <223> /note="Description of Artificial Sequence: Synthetic oligonucleotide"
<400> 28
   tgagtcagca gggttt 16
<210> 29
   <211> 21
   <212> DNA
   <213> Artificial Sequence
<220>
   <221> source
   <223> /note="Description of Artificial Sequence: Synthetic oligonucleotide"
<400> 29
   taggagcaaa cagtagcttc c 21
<210> 30
   <211> 24
   <212> DNA
   <213> Artificial Sequence
<220>
   <221> source
   <223> /note="Description of Artificial Sequence: Synthetic oligonucleotide"
<400> 30
   agttaggagc aaacagtagc ttcc 24
<210> 31
   <211> 30
   <212> DNA
   <213> Artificial Sequence
<220>
   <221> source
   <223> /note="Description of Artificial Sequence: Synthetic oligonucleotide"
<400> 31
   tgtgcctggc tagagatcct gatgattggt 30
<210> 32
   <211> 23
   <212> DNA
   <213> Artificial Sequence
<220>
   <221> source
   <223> /note="Description of Artificial Sequence: Synthetic oligonucleotide"
<400> 32
   ccatgatcag gtccaccttc tac 23
<210> 33
   <211> 23
   <212> DNA
   <213> Artificial Sequence
<220>
   <221> source
   <223> /note="Description of Artificial Sequence: Synthetic oligonucleotide"
<400> 33
   ccatgatcag gtccaccttc tcc 23
<210> 34
   <211> 23
   <212> DNA
   <213> Artificial Sequence
<220>
   <221> source
   <223> /note="Description of Artificial Sequence: Synthetic oligonucleotide"
<400> 34
   ccatgatcag gtccaccttc tgc 23
<210> 35
   <211> 23
   <212> DNA
   <213> Artificial Sequence
<220>
   <221> source
   <223> /note="Description of Artificial Sequence: Synthetic oligonucleotide"
<400> 35
   ccatgatcag gtccaccttc ttc 23
<210> 36
   <211> 23
   <212> DNA
   <213> Artificial Sequence
<220>
   <221> source
   <223> /note="Description of Artificial Sequence: Synthetic oligonucleotide"
<400> 36
   ccatgatcag gtccaccttc aac 23
<210> 37
   <211> 23
   <212> DNA
   <213> Artificial Sequence
<220>
   <221> source
   <223> /note="Description of Artificial Sequence: Synthetic oligonucleotide"
<400> 37
   ccatgatcag gtccaccttc cac 23
<210> 38
   <211> 23
   <212> DNA
   <213> Artificial Sequence
<220>
   <221> source
   <223> /note="Description of Artificial Sequence: Synthetic oligonucleotide"
<400> 38
   ccatgatcag gtccaccttc gac 23
<210> 39
   <211> 23
   <212> DNA
   <213> Artificial Sequence
<220>
   <221> source
   <223> /note="Description of Artificial Sequence: Synthetic oligonucleotide"
<400> 39
   ccatgatcag gtccacctta tac 23
<210> 40
   <211> 23
   <212> DNA
   <213> Artificial Sequence
<220>
   <221> source
   <223> /note="Description of Artificial Sequence: Synthetic oligonucleotide"
<400> 40
   ccatgatcag gtccaccttg tac 23
<210> 41
   <211> 23
   <212> DNA
   <213> Artificial Sequence
<220>
   <221> source
   <223> /note="Description of Artificial Sequence: Synthetic oligonucleotide"
<400> 41
   ccatgatcag gtccaccttt tac 23
<210> 42
   <211> 25
   <212> DNA
   <213> Artificial Sequence
<220>
   <221> source
   <223> /note="Description of Artificial Sequence: Synthetic oligonucleotide"
<400> 42
   ctccatgatc aggtccacct tgtac 25
<210> 43
   <211> 30
   <212> DNA
   <213> Artificial Sequence
<220>
   <221> source
   <223> /note="Description of Artificial Sequence: Synthetic oligonucleotide"
<400> 43
   tggacctgat catggagggt caaatccaca 30
<210> 44
   <211> 22
   <212> DNA
   <213> Artificial Sequence
<220>
   <221> source
   <223> /note="Description of Artificial Sequence: Synthetic oligonucleotide"
<400> 44
   ggatctctag ccaggcacat tg 22
<210> 45
   <211> 22
   <212> DNA
   <213> Artificial Sequence
<220>
   <221> source
   <223> /note="Description of Artificial Sequence: Synthetic oligonucleotide"
<400> 45
   ggatctctag ccaggcacat ag 22
<210> 46
   <211> 22
   <212> DNA
   <213> Artificial Sequence
<220>
   <221> source
   <223> /note="Description of Artificial Sequence: Synthetic oligonucleotide"
<400> 46
   ggatctctag ccaggcacat cg 22
<210> 47
   <211> 22
   <212> DNA
   <213> Artificial Sequence
<220>
   <221> source
   <223> /note="Description of Artificial Sequence: Synthetic oligonucleotide"
<400> 47
   ggatctctag ccaggcacat gg 22
<210> 48
   <211> 22
   <212> DNA
   <213> Artificial Sequence
<220>
   <221> source
   <223> /note="Description of Artificial Sequence: Synthetic oligonucleotide"
<400> 48
   ggatctctag ccaggcacaa tg 22
<210> 49
   <211> 22
   <212> DNA
   <213> Artificial Sequence
<220>
   <221> source
   <223> /note="Description of Artificial Sequence: Synthetic oligonucleotide"
<400> 49
   ggatctctag ccaggcacac tg 22
<210> 50
   <211> 22
   <212> DNA
   <213> Artificial Sequence
<220>
   <221> source
   <223> /note="Description of Artificial Sequence: Synthetic oligonucleotide"
<400> 50
   ggatctctag ccaggcacag tg 22
<210> 51
   <211> 22
   <212> DNA
   <213> Artificial Sequence
<220>
   <221> source
   <223> /note="Description of Artificial Sequence: Synthetic oligonucleotide"
<400> 51
   ggatctctag ccaggcacct tg 22
<210> 52
   <211> 22
   <212> DNA
   <213> Artificial Sequence
<220>
   <221> source
   <223> /note="Description of Artificial Sequence: Synthetic oligonucleotide"
<400> 52
   ggatctctag ccaggcacgt tg 22
<210> 53
   <211> 22
   <212> DNA
   <213> Artificial Sequence
<220>
   <221> source
   <223> /note="Description of Artificial Sequence: Synthetic oligonucleotide"
<400> 53
   ggatctctag ccaggcactt tg 22
<210> 54
   <211> 25
   <212> DNA
   <213> Artificial Sequence
<220>
   <221> source
   <223> /note="Description of Artificial Sequence: Synthetic oligonucleotide"
<400> 54
   ccctgaaccg tccgcagctc aagat 25
<210> 55
   <211> 20
   <212> DNA
   <213> Artificial Sequence
<220>
   <221> source
   <223> /note="Description of Artificial Sequence: Synthetic oligonucleotide"
<400> 55
   ccctactgca tcagatccaa 20
<210> 56
   <211> 20
   <212> DNA
   <213> Artificial Sequence
<220>
   <221> source
   <223> /note="Description of Artificial Sequence: Synthetic oligonucleotide"
<400> 56
   atcagatcca agggaacgag 20
<210> 57
   <211> 19
   <212> DNA
   <213> Artificial Sequence
<220>
   <221> source
   <223> /note="Description of Artificial Sequence: Synthetic oligonucleotide"
<400> 57
   ttgctgctgt ccaggtaca 19
<210> 58
   <211> 24
   <212> DNA
   <213> Artificial Sequence
<220>
   <221> source
   <223> /note="Description of Artificial Sequence: Synthetic oligonucleotide"
<400> 58
   tgctatgttt tcataggaac cagg 24
<210> 59
   <211> 22
   <212> DNA
   <213> Artificial Sequence
<220>
   <221> source
   <223> /note="Description of Artificial Sequence: Synthetic oligonucleotide"
<400> 59
   gatttgaggc acacaaactc ct 22
<210> 60
   <211> 30
   <212> DNA
   <213> Artificial Sequence
<220>
   <221> source
   <223> /note="Description of Artificial Sequence: Synthetic oligonucleotide"
<400> 60
   tcatctcggt tccgcatgat gaatctgcag 30
<210> 61
   <211> 21
   <212> DNA
   <213> Artificial Sequence
<220>
   <221> source
   <223> /note="Description of Artificial Sequence: Synthetic oligonucleotide"
<400> 61
   tggagatctt cgacatgctg g 21
<210> 62
   <211> 21
   <212> DNA
   <213> Artificial Sequence
<220>
   <221> source
   <223> /note="Description of Artificial Sequence: Synthetic oligonucleotide"
<400> 62
   tggagatctt cgacatgcta g 21
<210> 63
   <211> 21
   <212> DNA
   <213> Artificial Sequence
<220>
   <221> source
   <223> /note="Description of Artificial Sequence: Synthetic oligonucleotide"
<400> 63
   tggagatctt cgacatgctc g 21
<210> 64
   <211> 21
   <212> DNA
   <213> Artificial Sequence
<220>
   <221> source
   <223> /note="Description of Artificial Sequence: Synthetic oligonucleotide"
<400> 64
   tggagatctt cgacatgctt g 21
<210> 65
   <211> 21
   <212> DNA
   <213> Artificial Sequence
<220>
   <221> source
   <223> /note="Description of Artificial Sequence: Synthetic oligonucleotide"
<400> 65
   tggagatctt cgacatgcag g 21
<210> 66
   <211> 21
   <212> DNA
   <213> Artificial Sequence
<220>
   <221> source
   <223> /note="Description of Artificial Sequence: Synthetic oligonucleotide"
<400> 66
   tggagatctt cgacatgccg g 21
<210> 67
   <211> 21
   <212> DNA
   <213> Artificial Sequence
<220>
   <221> source
   <223> /note="Description of Artificial Sequence: Synthetic oligonucleotide"
<400> 67
   tggagatctt cgacatgcgg g 21
<210> 68
   <211> 21
   <212> DNA
   <213> Artificial Sequence
<220>
   <221> source
   <223> /note="Description of Artificial Sequence: Synthetic oligonucleotide"
<400> 68
   tggagatctt cgacatgatg g 21
<210> 69
   <211> 21
   <212> DNA
   <213> Artificial Sequence
<220>
   <221> source
   <223> /note="Description of Artificial Sequence: Synthetic oligonucleotide"
<400> 69
   tggagatctt cgacatggtg g 21
<210> 70
   <211> 21
   <212> DNA
   <213> Artificial Sequence
<220>
   <221> source
   <223> /note="Description of Artificial Sequence: Synthetic oligonucleotide"
<400> 70
   tggagatctt cgacatgttg g 21
<210> 71
   <211> 30
   <212> DNA
   <213> Artificial Sequence
<220>
   <221> source
   <223> /note="Description of Artificial Sequence: Synthetic oligonucleotide"
<400> 71
   tcgaagatct ccaccatgcc ctctacacat 30
<210> 72
   <211> 22
   <212> DNA
   <213> Artificial Sequence
<220>
   <221> source
   <223> /note="Description of Artificial Sequence: Synthetic oligonucleotide"
<400> 72
   ggaaccgaga tgatgtagcc ac 22
<210> 73
   <211> 22
   <212> DNA
   <213> Artificial Sequence
<220>
   <221> source
   <223> /note="Description of Artificial Sequence: Synthetic oligonucleotide"
<400> 73
   ggaaccgaga tgatgtagcc cc 22
<210> 74
   <211> 22
   <212> DNA
   <213> Artificial Sequence
<220>
   <221> source
   <223> /note="Description of Artificial Sequence: Synthetic oligonucleotide"
<400> 74
   ggaaccgaga tgatgtagcc gc 22
<210> 75
   <211> 22
   <212> DNA
   <213> Artificial Sequence
<220>
   <221> source
   <223> /note="Description of Artificial Sequence: Synthetic oligonucleotide"
<400> 75
   ggaaccgaga tgatgtagcc tc 22
<210> 76
   <211> 22
   <212> DNA
   <213> Artificial Sequence
<220>
   <221> source
   <223> /note="Description of Artificial Sequence: Synthetic oligonucleotide"
<400> 76
   ggaaccgaga tgatgtagca ac 22
<210> 77
   <211> 22
   <212> DNA
   <213> Artificial Sequence
<220>
   <221> source
   <223> /note="Description of Artificial Sequence: Synthetic oligonucleotide"
<400> 77
   ggaaccgaga tgatgtagcg ac 22
<210> 78
   <211> 22
   <212> DNA
   <213> Artificial Sequence
<220>
   <221> source
   <223> /note="Description of Artificial Sequence: Synthetic oligonucleotide"
<400> 78
   ggaaccgaga tgatgtagct ac 22
<210> 79
   <211> 22
   <212> DNA
   <213> Artificial Sequence
<220>
   <221> source
   <223> /note="Description of Artificial Sequence: Synthetic oligonucleotide"
<400> 79
   ggaaccgaga tgatgtagac ac 22
<210> 80
   <211> 22
   <212> DNA
   <213> Artificial Sequence
<220>
   <221> source
   <223> /note="Description of Artificial Sequence: Synthetic oligonucleotide"
<400> 80
   ggaaccgaga tgatgtaggc ac 22
<210> 81
   <211> 22
   <212> DNA
   <213> Artificial Sequence
<220>
   <221> source
   <223> /note="Description of Artificial Sequence: Synthetic oligonucleotide"
<400> 81
   ggaaccgaga tgatgtagtc ac 22
<210> 82
   <211> 24
   <212> DNA
   <213> Artificial Sequence
<220>
   <221> source
   <223> /note="Description of Artificial Sequence: Synthetic oligonucleotide"
<400> 82
   ctttctgtgt cttcccacct acag 24
<210> 83
   <211> 18
   <212> DNA
   <213> Artificial Sequence
<220>
   <221> source
   <223> /note="Description of Artificial Sequence: Synthetic oligonucleotide"
<400> 83
   aagtggcttt ggtccgtc 18
<210> 84
   <211> 15
   <212> DNA
   <213> Artificial Sequence
<220>
   <221> source
   <223> /note="Description of Artificial Sequence: Synthetic oligonucleotide"
<400> 84
   ccagcagcag ggggg 15
<210> 85
   <211> 14
   <212> DNA
   <213> Artificial Sequence
<220>
   <221> source
   <223> /note="Description of Artificial Sequence: Synthetic oligonucleotide"
<400> 85
   cagcagcagg gggg 14
<210> 86
   <211> 14
   <212> DNA
   <213> Artificial Sequence
<220>
   <221> source
   <223> /note="Description of Artificial Sequence: Synthetic oligonucleotide"
<400> 86
   ccagcagcag gggg 14
<210> 87
   <211> 15
   <212> DNA
   <213> Artificial Sequence
<220>
   <221> source
   <223> /note="Description of Artificial Sequence: Synthetic oligonucleotide"
<400> 87
   tccagcagca ggggg 15
<210> 88
   <211> 16
   <212> DNA
   <213> Artificial Sequence
<220>
   <221> source
   <223> /note="Description of Artificial Sequence: Synthetic oligonucleotide"
<400> 88
   ctccagcagc aggggg 16
<210> 89
   <211> 15
   <212> DNA
   <213> Artificial Sequence
<220>
   <221> source
   <223> /note="Description of Artificial Sequence: Synthetic oligonucleotide"
<400> 89
   ctccagcagc agggg 15
<210> 90
   <211> 16
   <212> DNA
   <213> Artificial Sequence
<220>
   <221> source
   <223> /note="Description of Artificial Sequence: Synthetic oligonucleotide"
<400> 90
   tctccagcag cagggg 16
<210> 91
   <211> 17
   <212> DNA
   <213> Artificial Sequence
<220>
   <221> source
   <223> /note="Description of Artificial Sequence: Synthetic oligonucleotide"
<400> 91
   atctccagca gcagggg 17
<210> 92
   <211> 17
   <212> DNA
   <213> Artificial Sequence
<220>
   <221> source
   <223> /note="Description of Artificial Sequence: Synthetic oligonucleotide"
<400> 92
   catctccagc agcaggg 17
<210> 93
   <211> 18
   <212> DNA
   <213> Artificial Sequence
<220>
   <221> source
   <223> /note="Description of Artificial Sequence: Synthetic oligonucleotide"
<400> 93
   gcatctccag cagcaggg 18
<210> 94
   <211> 23
   <212> DNA
   <213> Artificial Sequence
<220>
   <221> source
   <223> /note="Description of Artificial Sequence: Synthetic oligonucleotide"
<400> 94
   tacatgcgcc cactagccgt gga 23
<210> 95
   <211> 30
   <212> DNA
   <213> Artificial Sequence
<220>
   <221> source
   <223> /note="Description of Artificial Sequence: Synthetic oligonucleotide"
<400> 95
   tgcacttcat gctgtacaga tgctccatgc 30
<210> 96
   <211> 19
   <212> DNA
   <213> Artificial Sequence
<220>
   <221> source
   <223> /note="Description of Artificial Sequence: Synthetic oligonucleotide"
<400> 96
   gcaagaacgt ggtgcccca 19
<210> 97
   <211> 19
   <212> DNA
   <213> Artificial Sequence
<220>
   <221> source
   <223> /note="Description of Artificial Sequence: Synthetic oligonucleotide"
<400> 97
   gcaagaacgt ggtgcccaa 19
<210> 98
   <211> 19
   <212> DNA
   <213> Artificial Sequence
<220>
   <221> source
   <223> /note="Description of Artificial Sequence: Synthetic oligonucleotide"
<400> 98
   gcaagaacgt ggtgcccga 19
<210> 99
   <211> 19
   <212> DNA
   <213> Artificial Sequence
<220>
   <221> source
   <223> /note="Description of Artificial Sequence: Synthetic oligonucleotide"
<400> 99
   gcaagaacgt ggtgcccta 19
<210> 100
   <211> 19
   <212> DNA
   <213> Artificial Sequence
<220>
   <221> source
   <223> /note="Description of Artificial Sequence: Synthetic oligonucleotide"
<400> 100
   gcaagaacgt ggtgccaca 19
<210> 101
   <211> 19
   <212> DNA
   <213> Artificial Sequence
<220>
   <221> source
   <223> /note="Description of Artificial Sequence: Synthetic oligonucleotide"
<400> 101
   gcaagaacgt ggtgccgca 19
<210> 102
   <211> 19
   <212> DNA
   <213> Artificial Sequence
<220>
   <221> source
   <223> /note="Description of Artificial Sequence: Synthetic oligonucleotide"
<400> 102
   gcaagaacgt ggtgcctca 19
<210> 103
   <211> 19
   <212> DNA
   <213> Artificial Sequence
<220>
   <221> source
   <223> /note="Description of Artificial Sequence: Synthetic oligonucleotide"
<400> 103
   gcaagaacgt ggtgcacca 19
<210> 104
   <211> 19
   <212> DNA
   <213> Artificial Sequence
<220>
   <221> source
   <223> /note="Description of Artificial Sequence: Synthetic oligonucleotide"
<400> 104
   gcaagaacgt ggtgcgcca 19
<210> 105
   <211> 19
   <212> DNA
   <213> Artificial Sequence
<220>
   <221> source
   <223> /note="Description of Artificial Sequence: Synthetic oligonucleotide"
<400> 105
   gcaagaacgt ggtgctcca 19
<210> 106
   <211> 20
   <212> DNA
   <213> Artificial Sequence
<220>
   <221> source
   <223> /note="Description of Artificial Sequence: Synthetic oligonucleotide"
<400> 106
   ctccagcagc aggtcatagt 20
<210> 107
   <211> 20
   <212> DNA
   <213> Artificial Sequence
<220>
   <221> source
   <223> /note="Description of Artificial Sequence: Synthetic oligonucleotide"
<400> 107
   ctccagcagc aggtcataat 20
<210> 108
   <211> 20
   <212> DNA
   <213> Artificial Sequence
<220>
   <221> source
   <223> /note="Description of Artificial Sequence: Synthetic oligonucleotide"
<400> 108
   ctccagcagc aggtcatact 20
<210> 109
   <211> 20
   <212> DNA
   <213> Artificial Sequence
<220>
   <221> source
   <223> /note="Description of Artificial Sequence: Synthetic oligonucleotide"
<400> 109
   ctccagcagc aggtcatatt 20
<210> 110
   <211> 20
   <212> DNA
   <213> Artificial Sequence
<220>
   <221> source
   <223> /note="Description of Artificial Sequence: Synthetic oligonucleotide"
<400> 110
   ctccagcagc aggtcatcgt 20
<210> 111
   <211> 20
   <212> DNA
   <213> Artificial Sequence
<220>
   <221> source
   <223> /note="Description of Artificial Sequence: Synthetic oligonucleotide"
<400> 111
   ctccagcagc aggtcatggt 20
<210> 112
   <211> 20
   <212> DNA
   <213> Artificial Sequence
<220>
   <221> source
   <223> /note="Description of Artificial Sequence: Synthetic oligonucleotide"
<400> 112
   ctccagcagc aggtcattgt 20
<210> 113
   <211> 20
   <212> DNA
   <213> Artificial Sequence
<220>
   <221> source
   <223> /note="Description of Artificial Sequence: Synthetic oligonucleotide"
<400> 113
   ctccagcagc aggtcaaagt 20
<210> 114
   <211> 20
   <212> DNA
   <213> Artificial Sequence
<220>
   <221> source
   <223> /note="Description of Artificial Sequence: Synthetic oligonucleotide"
<400> 114
   ctccagcagc aggtcacagt 20
<210> 115
   <211> 20
   <212> DNA
   <213> Artificial Sequence
<220>
   <221> source
   <223> /note="Description of Artificial Sequence: Synthetic oligonucleotide"
<400> 115
   ctccagcagc aggtcagagt 20
<210> 116
   <211> 23
   <212> DNA
   <213> Artificial Sequence
<220>
   <221> source
   <223> /note="Description of Artificial Sequence: Synthetic oligonucleotide"
<400> 116
   catctccagc agcaggtcat aat 23
<210> 117
   <211> 23
   <212> DNA
   <213> Artificial Sequence
<220>
   <221> source
   <223> /note="Description of Artificial Sequence: Synthetic oligonucleotide"
<400> 117
   catctccagc agcaggtcat att 23
<210> 118
   <211> 23
   <212> DNA
   <213> Artificial Sequence
<220>
   <221> source
   <223> /note="Description of Artificial Sequence: Synthetic oligonucleotide"
<400> 118
   catctccagc agcaggtcgt agt 23
<210> 119
   <211> 23
   <212> DNA
   <213> Artificial Sequence
<220>
   <221> source
   <223> /note="Description of Artificial Sequence: Synthetic oligonucleotide"
<400> 119
   catctccagc agcaggtctt agt 23
<210> 120
   <211> 23
   <212> DNA
   <213> Artificial Sequence
<220>
   <221> source
   <223> /note="Description of Artificial Sequence: Synthetic oligonucleotide"
<400> 120
   catctccagc agcaggtcct agt 23
<210> 121
   <211> 16
   <212> DNA
   <213> Artificial Sequence
<220>
   <221> source
   <223> /note="Description of Artificial Sequence: Synthetic oligonucleotide"
<400> 121
   agaacgtggt gccccc 16
<210> 122
   <211> 16
   <212> DNA
   <213> Artificial Sequence
<220>
   <221> source
   <223> /note="Description of Artificial Sequence: Synthetic oligonucleotide"
<400> 122
   agaacgtggt gcccac 16
<210> 123
   <211> 16
   <212> DNA
   <213> Artificial Sequence
<220>
   <221> source
   <223> /note="Description of Artificial Sequence: Synthetic oligonucleotide"
<400> 123
   agaacgtggt gcccgc 16
<210> 124
   <211> 16
   <212> DNA
   <213> Artificial Sequence
<220>
   <221> source
   <223> /note="Description of Artificial Sequence: Synthetic oligonucleotide"
<400> 124
   agaacgtggt gccctc 16
<210> 125
   <211> 16
   <212> DNA
   <213> Artificial Sequence
<220>
   <221> source
   <223> /note="Description of Artificial Sequence: Synthetic oligonucleotide"
<400> 125
   agaacgtggt gccacc 16
<210> 126
   <211> 16
   <212> DNA
   <213> Artificial Sequence
<220>
   <221> source
   <223> /note="Description of Artificial Sequence: Synthetic oligonucleotide"
<400> 126
   agaacgtggt gccgcc 16
<210> 127
   <211> 16
   <212> DNA
   <213> Artificial Sequence
<220>
   <221> source
   <223> /note="Description of Artificial Sequence: Synthetic oligonucleotide"
<400> 127
   agaacgtggt gcctcc 16
<210> 128
   <211> 16
   <212> DNA
   <213> Artificial Sequence
<220>
   <221> source
   <223> /note="Description of Artificial Sequence: Synthetic oligonucleotide"
<400> 128
   agaacgtggt gcaccc 16
<210> 129
   <211> 16
   <212> DNA
   <213> Artificial Sequence
<220>
   <221> source
   <223> /note="Description of Artificial Sequence: Synthetic oligonucleotide"
<400> 129
   agaacgtggt gcgccc 16
<210> 130
   <211> 16
   <212> DNA
   <213> Artificial Sequence
<220>
   <221> source
   <223> /note="Description of Artificial Sequence: Synthetic oligonucleotide"
<400> 130
   agaacgtggt gctccc 16
<210> 131
   <211> 20
   <212> DNA
   <213> Artificial Sequence
<220>
   <221> source
   <223> /note="Description of Artificial Sequence: Synthetic oligonucleotide"
<400> 131
   ctccagcagc aggtcatagg 20
<210> 132
   <211> 20
   <212> DNA
   <213> Artificial Sequence
<220>
   <221> source
   <223> /note="Description of Artificial Sequence: Synthetic oligonucleotide"
<400> 132
   ctccagcagc aggtcataag 20
<210> 133
   <211> 20
   <212> DNA
   <213> Artificial Sequence
<220>
   <221> source
   <223> /note="Description of Artificial Sequence: Synthetic oligonucleotide"
<400> 133
   ctccagcagc aggtcatacg 20
<210> 134
   <211> 20
   <212> DNA
   <213> Artificial Sequence
<220>
   <221> source
   <223> /note="Description of Artificial Sequence: Synthetic oligonucleotide"
<400> 134
   ctccagcagc aggtcatatg 20
<210> 135
   <211> 20
   <212> DNA
   <213> Artificial Sequence
<220>
   <221> source
   <223> /note="Description of Artificial Sequence: Synthetic oligonucleotide"
<400> 135
   ctccagcagc aggtcatcgg 20
<210> 136
   <211> 20
   <212> DNA
   <213> Artificial Sequence
<220>
   <221> source
   <223> /note="Description of Artificial Sequence: Synthetic oligonucleotide"
<400> 136
   ctccagcagc aggtcatggg 20
<210> 137
   <211> 20
   <212> DNA
   <213> Artificial Sequence
<220>
   <221> source
   <223> /note="Description of Artificial Sequence: Synthetic oligonucleotide"
<400> 137
   ctccagcagc aggtcattgg 20
<210> 138
   <211> 20
   <212> DNA
   <213> Artificial Sequence
<220>
   <221> source
   <223> /note="Description of Artificial Sequence: Synthetic oligonucleotide"
<400> 138
   ctccagcagc aggtcaaagg 20
<210> 139
   <211> 20
   <212> DNA
   <213> Artificial Sequence
<220>
   <221> source
   <223> /note="Description of Artificial Sequence: Synthetic oligonucleotide"
<400> 139
   ctccagcagc aggtcacagg 20
<210> 140
   <211> 20
   <212> DNA
   <213> Artificial Sequence
<220>
   <221> source
   <223> /note="Description of Artificial Sequence: Synthetic oligonucleotide"
<400> 140
   ctccagcagc aggtcagagg 20
<210> 141
   <211> 17
   <212> DNA
   <213> Artificial Sequence
<220>
   <221> source
   <223> /note="Description of Artificial Sequence: Synthetic oligonucleotide"
<400> 141
   agaacgtggt gccccag 17
<210> 142
   <211> 17
   <212> DNA
   <213> Artificial Sequence
<220>
   <221> source
   <223> /note="Description of Artificial Sequence: Synthetic oligonucleotide"
<400> 142
   agaacgtggt gcccccg 17
<210> 143
   <211> 17
   <212> DNA
   <213> Artificial Sequence
<220>
   <221> source
   <223> /note="Description of Artificial Sequence: Synthetic oligonucleotide"
<400> 143
   agaacgtggt gccccgg 17
<210> 144
   <211> 17
   <212> DNA
   <213> Artificial Sequence
<220>
   <221> source
   <223> /note="Description of Artificial Sequence: Synthetic oligonucleotide"
<400> 144
   agaacgtggt gcccctg 17
<210> 145
   <211> 17
   <212> DNA
   <213> Artificial Sequence
<220>
   <221> source
   <223> /note="Description of Artificial Sequence: Synthetic oligonucleotide"
<400> 145
   agaacgtggt gcccaag 17
<210> 146
   <211> 17
   <212> DNA
   <213> Artificial Sequence
<220>
   <221> source
   <223> /note="Description of Artificial Sequence: Synthetic oligonucleotide"
<400> 146
   agaacgtggt gcccgag 17
<210> 147
   <211> 17
   <212> DNA
   <213> Artificial Sequence
<220>
   <221> source
   <223> /note="Description of Artificial Sequence: Synthetic oligonucleotide"
<400> 147
   agaacgtggt gccctag 17
<210> 148
   <211> 17
   <212> DNA
   <213> Artificial Sequence
<220>
   <221> source
   <223> /note="Description of Artificial Sequence: Synthetic oligonucleotide"
<400> 148
   agaacgtggt gccacag 17
<210> 149
   <211> 17
   <212> DNA
   <213> Artificial Sequence
<220>
   <221> source
   <223> /note="Description of Artificial Sequence: Synthetic oligonucleotide"
<400> 149
   agaacgtggt gccgcag 17
<210> 150
   <211> 17
   <212> DNA
   <213> Artificial Sequence
<220>
   <221> source
   <223> /note="Description of Artificial Sequence: Synthetic oligonucleotide"
<400> 150
   agaacgtggt gcctcag 17
<210> 151
   <211> 20
   <212> DNA
   <213> Artificial Sequence
<220>
   <221> source
   <223> /note="Description of Artificial Sequence: Synthetic oligonucleotide"
<400> 151
   ctccagcagc aggtcatact 20
<210> 152
   <211> 20
   <212> DNA
   <213> Artificial Sequence
<220>
   <221> source
   <223> /note="Description of Artificial Sequence: Synthetic oligonucleotide"
<400> 152
   ctccagcagc aggtcataat 20
<210> 153
   <211> 20
   <212> DNA
   <213> Artificial Sequence
<220>
   <221> source
   <223> /note="Description of Artificial Sequence: Synthetic oligonucleotide"
<400> 153
   ctccagcagc aggtcatagt 20
<210> 154
   <211> 20
   <212> DNA
   <213> Artificial Sequence
<220>
   <221> source
   <223> /note="Description of Artificial Sequence: Synthetic oligonucleotide"
<400> 154
   ctccagcagc aggtcatatt 20
<210> 155
   <211> 20
   <212> DNA
   <213> Artificial Sequence
<220>
   <221> source
   <223> /note="Description of Artificial Sequence: Synthetic oligonucleotide"
<400> 155
   ctccagcagc aggtcatcct 20
<210> 156
   <211> 20
   <212> DNA
   <213> Artificial Sequence
<220>
   <221> source
   <223> /note="Description of Artificial Sequence: Synthetic oligonucleotide"
<400> 156
   ctccagcagc aggtcatgct 20
<210> 157
   <211> 20
   <212> DNA
   <213> Artificial Sequence
<220>
   <221> source
   <223> /note="Description of Artificial Sequence: Synthetic oligonucleotide"
<400> 157
   ctccagcagc aggtcattct 20
<210> 158
   <211> 20
   <212> DNA
   <213> Artificial Sequence
<220>
   <221> source
   <223> /note="Description of Artificial Sequence: Synthetic oligonucleotide"
<400> 158
   ctccagcagc aggtcaaact 20
<210> 159
   <211> 20
   <212> DNA
   <213> Artificial Sequence
<220>
   <221> source
   <223> /note="Description of Artificial Sequence: Synthetic oligonucleotide"
<400> 159
   ctccagcagc aggtcacact 20
<210> 160
   <211> 20
   <212> DNA
   <213> Artificial Sequence
<220>
   <221> source
   <223> /note="Description of Artificial Sequence: Synthetic oligonucleotide"
<400> 160
   ctccagcagc aggtcagact 20
<210> 161
   <211> 16
   <212> DNA
   <213> Artificial Sequence
<220>
   <221> source
   <223> /note="Description of Artificial Sequence: Synthetic oligonucleotide"
<400> 161
   agaacgtggt gccccg 16
<210> 162
   <211> 16
   <212> DNA
   <213> Artificial Sequence
<220>
   <221> source
   <223> /note="Description of Artificial Sequence: Synthetic oligonucleotide"
<400> 162
   agaacgtggt gcccag 16
<210> 163
   <211> 16
   <212> DNA
   <213> Artificial Sequence
<220>
   <221> source
   <223> /note="Description of Artificial Sequence: Synthetic oligonucleotide"
<400> 163
   agaacgtggt gcccgg 16
<210> 164
   <211> 16
   <212> DNA
   <213> Artificial Sequence
<220>
   <221> source
   <223> /note="Description of Artificial Sequence: Synthetic oligonucleotide"
<400> 164
   agaacgtggt gccctg 16
<210> 165
   <211> 16
   <212> DNA
   <213> Artificial Sequence
<220>
   <221> source
   <223> /note="Description of Artificial Sequence: Synthetic oligonucleotide"
<400> 165
   agaacgtggt gccacg 16
<210> 166
   <211> 16
   <212> DNA
   <213> Artificial Sequence
<220>
   <221> source
   <223> /note="Description of Artificial Sequence: Synthetic oligonucleotide"
<400> 166
   agaacgtggt gccgcg 16
<210> 167
   <211> 16
   <212> DNA
   <213> Artificial Sequence
<220>
   <221> source
   <223> /note="Description of Artificial Sequence: Synthetic oligonucleotide"
<400> 167
   agaacgtggt gcctcg 16
<210> 168
   <211> 16
   <212> DNA
   <213> Artificial Sequence
<220>
   <221> source
   <223> /note="Description of Artificial Sequence: Synthetic oligonucleotide"
<400> 168
   agaacgtggt gcaccg 16
<210> 169
   <211> 16
   <212> DNA
   <213> Artificial Sequence
<220>
   <221> source
   <223> /note="Description of Artificial Sequence: Synthetic oligonucleotide"
<400> 169
   agaacgtggt gcgccg 16
<210> 170
   <211> 16
   <212> DNA
   <213> Artificial Sequence
<220>
   <221> source
   <223> /note="Description of Artificial Sequence: Synthetic oligonucleotide"
<400> 170
   agaacgtggt gctccg 16
<210> 171
   <211> 20
   <212> DNA
   <213> Artificial Sequence
<220>
   <221> source
   <223> /note="Description of Artificial Sequence: Synthetic oligonucleotide"
<400> 171
   ctccagcagc aggtcatagc 20
<210> 172
   <211> 20
   <212> DNA
   <213> Artificial Sequence
<220>
   <221> source
   <223> /note="Description of Artificial Sequence: Synthetic oligonucleotide"
<400> 172
   ctccagcagc aggtcataac 20
<210> 173
   <211> 20
   <212> DNA
   <213> Artificial Sequence
<220>
   <221> source
   <223> /note="Description of Artificial Sequence: Synthetic oligonucleotide"
<400> 173
   ctccagcagc aggtcatacc 20
<210> 174
   <211> 20
   <212> DNA
   <213> Artificial Sequence
<220>
   <221> source
   <223> /note="Description of Artificial Sequence: Synthetic oligonucleotide"
<400> 174
   ctccagcagc aggtcatatc 20
<210> 175
   <211> 20
   <212> DNA
   <213> Artificial Sequence
<220>
   <221> source
   <223> /note="Description of Artificial Sequence: Synthetic oligonucleotide"
<400> 175
   ctccagcagc aggtcatcgc 20
<210> 176
   <211> 20
   <212> DNA
   <213> Artificial Sequence
<220>
   <221> source
   <223> /note="Description of Artificial Sequence: Synthetic oligonucleotide"
<400> 176
   ctccagcagc aggtcatggc 20
<210> 177
   <211> 20
   <212> DNA
   <213> Artificial Sequence
<220>
   <221> source
   <223> /note="Description of Artificial Sequence: Synthetic oligonucleotide"
<400> 177
   ctccagcagc aggtcattgc 20
<210> 178
   <211> 20
   <212> DNA
   <213> Artificial Sequence
<220>
   <221> source
   <223> /note="Description of Artificial Sequence: Synthetic oligonucleotide"
<400> 178
   ctccagcagc aggtcaaagc 20
<210> 179
   <211> 20
   <212> DNA
   <213> Artificial Sequence
<220>
   <221> source
   <223> /note="Description of Artificial Sequence: Synthetic oligonucleotide"
<400> 179
   ctccagcagc aggtcacagc 20
<210> 180
   <211> 20
   <212> DNA
   <213> Artificial Sequence
<220>
   <221> source
   <223> /note="Description of Artificial Sequence: Synthetic oligonucleotide"
<400> 180
   ctccagcagc aggtcagagc 20
<210> 181
   <211> 20
   <212> DNA
   <213> Artificial Sequence
<220>
   <221> source
   <223> /note="Description of Artificial Sequence: Synthetic oligonucleotide"
<400> 181
   aagtgcaaga acgtggtgca 20
<210> 182
   <211> 20
   <212> DNA
   <213> Artificial Sequence
<220>
   <221> source
   <223> /note="Description of Artificial Sequence: Synthetic oligonucleotide"
<400> 182
   aagtgcaaga acgtggtgaa 20
<210> 183
   <211> 20
   <212> DNA
   <213> Artificial Sequence
<220>
   <221> source
   <223> /note="Description of Artificial Sequence: Synthetic oligonucleotide"
<400> 183
   aagtgcaaga acgtggtgga 20
<210> 184
   <211> 20
   <212> DNA
   <213> Artificial Sequence
<220>
   <221> source
   <223> /note="Description of Artificial Sequence: Synthetic oligonucleotide"
<400> 184
   aagtgcaaga acgtggtgta 20
<210> 185
   <211> 20
   <212> DNA
   <213> Artificial Sequence
<220>
   <221> source
   <223> /note="Description of Artificial Sequence: Synthetic oligonucleotide"
<400> 185
   aagtgcaaga acgtggtaca 20
<210> 186
   <211> 20
   <212> DNA
   <213> Artificial Sequence
<220>
   <221> source
   <223> /note="Description of Artificial Sequence: Synthetic oligonucleotide"
<400> 186
   aagtgcaaga acgtggtcca 20
<210> 187
   <211> 20
   <212> DNA
   <213> Artificial Sequence
<220>
   <221> source
   <223> /note="Description of Artificial Sequence: Synthetic oligonucleotide"
<400> 187
   aagtgcaaga acgtggttca 20
<210> 188
   <211> 20
   <212> DNA
   <213> Artificial Sequence
<220>
   <221> source
   <223> /note="Description of Artificial Sequence: Synthetic oligonucleotide"
<400> 188
   aagtgcaaga acgtggagca 20
<210> 189
   <211> 20
   <212> DNA
   <213> Artificial Sequence
<220>
   <221> source
   <223> /note="Description of Artificial Sequence: Synthetic oligonucleotide"
<400> 189
   aagtgcaaga acgtggcgca 20
<210> 190
   <211> 20
   <212> DNA
   <213> Artificial Sequence
<220>
   <221> source
   <223> /note="Description of Artificial Sequence: Synthetic oligonucleotide"
<400> 190
   aagtgcaaga acgtggggca 20
<210> 191
   <211> 19
   <212> DNA
   <213> Artificial Sequence
<220>
   <221> source
   <223> /note="Description of Artificial Sequence: Synthetic oligonucleotide"
<400> 191
   agcagcaggt catagaggt 19
<210> 192
   <211> 19
   <212> DNA
   <213> Artificial Sequence
<220>
   <221> source
   <223> /note="Description of Artificial Sequence: Synthetic oligonucleotide"
<400> 192
   agcagcaggt catagagat 19
<210> 193
   <211> 19
   <212> DNA
   <213> Artificial Sequence
<220>
   <221> source
   <223> /note="Description of Artificial Sequence: Synthetic oligonucleotide"
<400> 193
   agcagcaggt catagagct 19
<210> 194
   <211> 19
   <212> DNA
   <213> Artificial Sequence
<220>
   <221> source
   <223> /note="Description of Artificial Sequence: Synthetic oligonucleotide"
<400> 194
   agcagcaggt catagagtt 19
<210> 195
   <211> 19
   <212> DNA
   <213> Artificial Sequence
<220>
   <221> source
   <223> /note="Description of Artificial Sequence: Synthetic oligonucleotide"
<400> 195
   agcagcaggt catagaagt 19
<210> 196
   <211> 19
   <212> DNA
   <213> Artificial Sequence
<220>
   <221> source
   <223> /note="Description of Artificial Sequence: Synthetic oligonucleotide"
<400> 196
   agcagcaggt catagacgt 19
<210> 197
   <211> 19
   <212> DNA
   <213> Artificial Sequence
<220>
   <221> source
   <223> /note="Description of Artificial Sequence: Synthetic oligonucleotide"
<400> 197
   agcagcaggt catagatgt 19
<210> 198
   <211> 19
   <212> DNA
   <213> Artificial Sequence
<220>
   <221> source
   <223> /note="Description of Artificial Sequence: Synthetic oligonucleotide"
<400> 198
   agcagcaggt catagcggt 19
<210> 199
   <211> 19
   <212> DNA
   <213> Artificial Sequence
<220>
   <221> source
   <223> /note="Description of Artificial Sequence: Synthetic oligonucleotide"
<400> 199
   agcagcaggt cataggggt 19
<210> 200
   <211> 19
   <212> DNA
   <213> Artificial Sequence
<220>
   <221> source
   <223> /note="Description of Artificial Sequence: Synthetic oligonucleotide"
<400> 200
   agcagcaggt catagtggt 19
<210> 201
   <211> 21
   <212> DNA
   <213> Artificial Sequence
<220>
   <221> source
   <223> /note="Description of Artificial Sequence: Synthetic oligonucleotide"
<400> 201
   ccagcagcag gtcatagagg t 21
<210> 202
   <211> 20
   <212> DNA
   <213> Artificial Sequence
<220>
   <221> source
   <223> /note="Description of Artificial Sequence: Synthetic oligonucleotide"
<400> 202
   ccagcagcag gtcatgmggt 20
<210> 203
   <211> 20
   <212> DNA
   <213> Artificial Sequence
<220>
   <221> source
   <223> /note="Description of Artificial Sequence: Synthetic oligonucleotide"
<400> 203
   ccagcagcag gtcatagggt 20
<210> 204
   <211> 21
   <212> DNA
   <213> Artificial Sequence
<220>
   <221> source
   <223> /note="Description of Artificial Sequence: Synthetic oligonucleotide"
<400> 204
   ccagcagcag gtcatagtgg t 21
<210> 205
   <211> 19
   <212> DNA
   <213> Artificial Sequence
<220>
   <221> source
   <223> /note="Description of Artificial Sequence: Synthetic oligonucleotide"
<400> 205
   agcagcaggt cataaaggt 19
<210> 206
   <211> 19
   <212> DNA
   <213> Artificial Sequence
<220>
   <221> source
   <223> /note="Description of Artificial Sequence: Synthetic oligonucleotide"
<400> 206
   agcagcaggt catacaggt 19
<210> 207
   <211> 19
   <212> DNA
   <213> Artificial Sequence
<220>
   <221> source
   <223> /note="Description of Artificial Sequence: Synthetic oligonucleotide"
<400> 207
   agcagcaggt catataggt 19
<210> 208
   <211> 19
   <212> DNA
   <213> Artificial Sequence
<220>
   <221> source
   <223> /note="Description of Artificial Sequence: Synthetic oligonucleotide"
<400> 208
   agcagcaggt catagaggt 19
<210> 209
   <211> 19
   <212> DNA
   <213> Artificial Sequence
<220>
   <221> source
   <223> /note="Description of Artificial Sequence: Synthetic oligonucleotide"
<400> 209
   agcagcaggt catagaggt 19
<210> 210
   <211> 19
   <212> DNA
   <213> Artificial Sequence
<220>
   <221> source
   <223> /note="Description of Artificial Sequence: Synthetic oligonucleotide"
<400> 210
   agcagcaggt catagaggt 19
<210> 211
   <211> 19
   <212> DNA
   <213> Artificial Sequence
<220>
   <221> source
   <223> /note="Description of Artificial Sequence: Synthetic oligonucleotide"
<400> 211
   agcagcaggt catagaggt 19
<210> 212
   <211> 21
   <212> DNA
   <213> Artificial Sequence
<220>
   <221> source
   <223> /note="Description of Artificial Sequence: Synthetic oligonucleotide"
<400> 212
   ccagcagcag gtcatagcgg t 21
<210> 213
   <211> 19
   <212> DNA
   <213> Artificial Sequence
<220>
   <221> source
   <223> /note="Description of Artificial Sequence: Synthetic oligonucleotide"
<400> 213
   agcagcaggt cataaacgt 19
<210> 214
   <211> 19
   <212> DNA
   <213> Artificial Sequence
<220>
   <221> source
   <223> /note="Description of Artificial Sequence: Synthetic oligonucleotide"
<400> 214
   agcagcaggt catacacgt 19
<210> 215
   <211> 19
   <212> DNA
   <213> Artificial Sequence
<220>
   <221> source
   <223> /note="Description of Artificial Sequence: Synthetic oligonucleotide"
<400> 215
   agcagcaggt catatacgt 19
<210> 216
   <211> 19
   <212> DNA
   <213> Artificial Sequence
<220>
   <221> source
   <223> /note="Description of Artificial Sequence: Synthetic oligonucleotide"
<400> 216
   agcagcaggt cataaatgt 19
<210> 217
   <211> 19
   <212> DNA
   <213> Artificial Sequence
<220>
   <221> source
   <223> /note="Description of Artificial Sequence: Synthetic oligonucleotide"
<400> 217
   agcagcaggt catacatgt 19
<210> 218
   <211> 19
   <212> DNA
   <213> Artificial Sequence
<220>
   <221> source
   <223> /note="Description of Artificial Sequence: Synthetic oligonucleotide"
<400> 218
   agcagcaggt catatatgt 19
<210> 219
   <211> 19
   <212> DNA
   <213> Artificial Sequence
<220>
   <221> source
   <223> /note="Description of Artificial Sequence: Synthetic oligonucleotide"
<400> 219
   agcagcaggt cataaaagt 19
<210> 220
   <211> 19
   <212> DNA
   <213> Artificial Sequence
<220>
   <221> source
   <223> /note="Description of Artificial Sequence: Synthetic oligonucleotide"
<400> 220
   agcagcaggt catacaagt 19
<210> 221
   <211> 19
   <212> DNA
   <213> Artificial Sequence
<220>
   <221> source
   <223> /note="Description of Artificial Sequence: Synthetic oligonucleotide"
<400> 221
   agcagcaggt catataagt 19
<210> 222
   <211> 19
   <212> DNA
   <213> Artificial Sequence
<220>
   <221> source
   <223> /note="Description of Artificial Sequence: Synthetic oligonucleotide"
<400> 222
   agcagcaggt catagaggt 19
<210> 223
   <211> 21
   <212> DNA
   <213> Artificial Sequence
<220>
   <221> source
   <223> /note="Description of Artificial Sequence: Synthetic oligonucleotide"
<400> 223
   ccagcagcag gtcatagagg t 21
<210> 224
   <211> 21
   <212> DNA
   <213> Artificial Sequence
<220>
   <221> source
   <223> /note="Description of Artificial Sequence: Synthetic oligonucleotide"
<400> 224
   ccagcagcag gtcatagtgg t 21
<210> 225
   <211> 19
   <212> DNA
   <213> Artificial Sequence
<220>
   <221> source
   <223> /note="Description of Artificial Sequence: Synthetic oligonucleotide"
<400> 225
   agcagcaggt catagaggt 19
<210> 226
   <211> 20
   <212> DNA
   <213> Artificial Sequence
<220>
   <221> source
   <223> /note="Description of Artificial Sequence: Synthetic oligonucleotide"
<400> 226
   cagcagcagg tcataaaggt 20
<210> 227
   <211> 20
   <212> DNA
   <213> Artificial Sequence
<220>
   <221> source
   <223> /note="Description of Artificial Sequence: Synthetic oligonucleotide"
<400> 227
   cagcagcagg tcatacaggt 20
<210> 228
   <211> 20
   <212> DNA
   <213> Artificial Sequence
<220>
   <221> source
   <223> /note="Description of Artificial Sequence: Synthetic oligonucleotide"
<400> 228
   cagcagcagg tcatataggt 20
<210> 229
   <211> 21
   <212> DNA
   <213> Artificial Sequence
<220>
   <221> source
   <223> /note="Description of Artificial Sequence: Synthetic oligonucleotide"
<400> 229
   ccagcagcag gtcataaagg t 21
<210> 230
   <211> 21
   <212> DNA
   <213> Artificial Sequence
<220>
   <221> source
   <223> /note="Description of Artificial Sequence: Synthetic oligonucleotide"
<400> 230
   ccagcagcag gtcatacagg t 21
<210> 231
   <211> 21
   <212> DNA
   <213> Artificial Sequence
<220>
   <221> source
   <223> /note="Description of Artificial Sequence: Synthetic oligonucleotide"
<400> 231
   ccagcagcag gtcatatagg t 21
<210> 232
   <211> 20
   <212> DNA
   <213> Artificial Sequence
<220>
   <221> source
   <223> /note="Description of Artificial Sequence: Synthetic oligonucleotide"
<400> 232
   agatggtcag tgccttgttg 20
<210> 233
   <211> 20
   <212> DNA
   <213> Artificial Sequence
<220>
   <221> source
   <223> /note="Description of Artificial Sequence: Synthetic oligonucleotide"
<400> 233
   cagatggtca gtgccttgtt 20
<210> 234
   <211> 22
   <212> DNA
   <213> Artificial Sequence
<220>
   <221> source
   <223> /note="Description of Artificial Sequence: Synthetic oligonucleotide"
<400> 234
   attcttacct ggcaccctct tc 22
<210> 235
   <211> 20
   <212> DNA
   <213> Artificial Sequence
<220>
   <221> source
   <223> /note="Description of Artificial Sequence: Synthetic oligonucleotide"
<400> 235
   ctcttcgccc agttgatcat 20
<210> 236
   <211> 30
   <212> DNA
   <213> Artificial Sequence
<220>
   <221> source
   <223> /note="Description of Artificial Sequence: Synthetic oligonucleotide"
<400> 236
   agacagggag ctggttcaca tgatcaactg 30
<210> 237
   <211> 30
   <212> DNA
   <213> Artificial Sequence
<220>
   <221> source
   <223> /note="Description of Artificial Sequence: Synthetic oligonucleotide"
<400> 237
   tgatgggctt actgaccaac ctggcagaca 30
<210> 238
   <211> 22
   <212> DNA
   <213> Artificial Sequence
<220>
   <221> source
   <223> /note="Description of Artificial Sequence: Synthetic oligonucleotide"
<400> 238
   ccagaccctt cagtgaagct tt 22
<210> 239
   <211> 22
   <212> DNA
   <213> Artificial Sequence
<220>
   <221> source
   <223> /note="Description of Artificial Sequence: Synthetic oligonucleotide"
<400> 239
   ccagaccctt cagtgaagct at 22
<210> 240
   <211> 22
   <212> DNA
   <213> Artificial Sequence
<220>
   <221> source
   <223> /note="Description of Artificial Sequence: Synthetic oligonucleotide"
<400> 240
   ccagaccctt cagtgaagct ct 22
<210> 241
   <211> 22
   <212> DNA
   <213> Artificial Sequence
<220>
   <221> source
   <223> /note="Description of Artificial Sequence: Synthetic oligonucleotide"
<400> 241
   ccagaccctt cagtgaagct gt 22
<210> 242
   <211> 22
   <212> DNA
   <213> Artificial Sequence
<220>
   <221> source
   <223> /note="Description of Artificial Sequence: Synthetic oligonucleotide"
<400> 242
   ccagaccctt cagtgaagca tt 22
<210> 243
   <211> 22
   <212> DNA
   <213> Artificial Sequence
<220>
   <221> source
   <223> /note="Description of Artificial Sequence: Synthetic oligonucleotide"
<400> 243
   ccagaccctt cagtgaagcc tt 22
<210> 244
   <211> 22
   <212> DNA
   <213> Artificial Sequence
<220>
   <221> source
   <223> /note="Description of Artificial Sequence: Synthetic oligonucleotide"
<400> 244
   ccagaccctt cagtgaagcg tt 22
<210> 245
   <211> 22
   <212> DNA
   <213> Artificial Sequence
<220>
   <221> source
   <223> /note="Description of Artificial Sequence: Synthetic oligonucleotide"
<400> 245
   ccagaccctt cagtgaagat tt 22
<210> 246
   <211> 22
   <212> DNA
   <213> Artificial Sequence
<220>
   <221> source
   <223> /note="Description of Artificial Sequence: Synthetic oligonucleotide"
<400> 246
   ccagaccctt cagtgaaggt tt 22
<210> 247
   <211> 22
   <212> DNA
   <213> Artificial Sequence
<220>
   <221> source
   <223> /note="Description of Artificial Sequence: Synthetic oligonucleotide"
<400> 247
   ccagaccctt cagtgaagtt tt 22
<210> 248
   <211> 30
   <212> DNA
   <213> Artificial Sequence
<220>
   <221> source
   <223> /note="Description of Artificial Sequence: Synthetic oligonucleotide"
<400> 248
   actgaagggt ctggtaggat catactcgga 30
<210> 249
   <211> 32
   <212> DNA
   <213> Artificial Sequence
<220>
   <221> source
   <223> /note="Description of Artificial Sequence: Synthetic oligonucleotide"
<400> 249
   ctgaagggtc tggtaggatc atactcggaa ta 32
<210> 250
   <211> 23
   <212> DNA
   <213> Artificial Sequence
<220>
   <221> source
   <223> /note="Description of Artificial Sequence: Synthetic oligonucleotide"
<400> 250
   gttggtcagt aagcccatca tca 23
<210> 251
   <211> 23
   <212> DNA
   <213> Artificial Sequence
<220>
   <221> source
   <223> /note="Description of Artificial Sequence: Synthetic oligonucleotide"
<400> 251
   gttggtcagt aagcccatca taa 23
<210> 252
   <211> 23
   <212> DNA
   <213> Artificial Sequence
<220>
   <221> source
   <223> /note="Description of Artificial Sequence: Synthetic oligonucleotide"
<400> 252
   gttggtcagt aagcccatca tga 23
<210> 253
   <211> 23
   <212> DNA
   <213> Artificial Sequence
<220>
   <221> source
   <223> /note="Description of Artificial Sequence: Synthetic oligonucleotide"
<400> 253
   gttggtcagt aagcccatca tta 23
<210> 254
   <211> 23
   <212> DNA
   <213> Artificial Sequence
<220>
   <221> source
   <223> /note="Description of Artificial Sequence: Synthetic oligonucleotide"
<400> 254
   gttggtcagt aagcccatca aca 23
<210> 255
   <211> 23
   <212> DNA
   <213> Artificial Sequence
<220>
   <221> source
   <223> /note="Description of Artificial Sequence: Synthetic oligonucleotide"
<400> 255
   gttggtcagt aagcccatca cca 23
<210> 256
   <211> 23
   <212> DNA
   <213> Artificial Sequence
<220>
   <221> source
   <223> /note="Description of Artificial Sequence: Synthetic oligonucleotide"
<400> 256
   gttggtcagt aagcccatca gca 23
<210> 257
   <211> 23
   <212> DNA
   <213> Artificial Sequence
<220>
   <221> source
   <223> /note="Description of Artificial Sequence: Synthetic oligonucleotide"
<400> 257
   gttggtcagt aagcccatcc tca 23
<210> 258
   <211> 23
   <212> DNA
   <213> Artificial Sequence
<220>
   <221> source
   <223> /note="Description of Artificial Sequence: Synthetic oligonucleotide"
<400> 258
   gttggtcagt aagcccatcg tca 23
<210> 259
   <211> 23
   <212> DNA
   <213> Artificial Sequence
<220>
   <221> source
   <223> /note="Description of Artificial Sequence: Synthetic oligonucleotide"
<400> 259
   gttggtcagt aagcccatct tca 23
<210> 260
   <211> 22
   <212> DNA
   <213> Artificial Sequence
<220>
   <221> source
   <223> /note="Description of Artificial Sequence: Synthetic oligonucleotide"
<400> 260
   ctagacctca tcctctttga gc 22
<210> 261
   <211> 24
   <212> DNA
   <213> Artificial Sequence
<220>
   <221> source
   <223> /note="Description of Artificial Sequence: Synthetic oligonucleotide"
<400> 261
   ccatcaggtg gatcaaagtg tctg 24
<210> 262
   <211> 30
   <212> DNA
   <213> Artificial Sequence
<220>
   <221> source
   <223> /note="Description of Artificial Sequence: Synthetic oligonucleotide"
<400> 262
   accatatcca ccgagtcctg gacaagatca 30
<210> 263
   <211> 25
   <212> DNA
   <213> Artificial Sequence
<220>
   <221> source
   <223> /note="Description of Artificial Sequence: Synthetic oligonucleotide"
<400> 263
   cattcaggag tgtacacatt tctgc 25
<210> 264
   <211> 25
   <212> DNA
   <213> Artificial Sequence
<220>
   <221> source
   <223> /note="Description of Artificial Sequence: Synthetic oligonucleotide"
<400> 264
   cattcaggag tgtacacatt tctac 25
<210> 265
   <211> 25
   <212> DNA
   <213> Artificial Sequence
<220>
   <221> source
   <223> /note="Description of Artificial Sequence: Synthetic oligonucleotide"
<400> 265
   cattcaggag tgtacacatt tctcc 25
<210> 266
   <211> 25
   <212> DNA
   <213> Artificial Sequence
<220>
   <221> source
   <223> /note="Description of Artificial Sequence: Synthetic oligonucleotide"
<400> 266
   cattcaggag tgtacacatt tcttc 25
<210> 267
   <211> 25
   <212> DNA
   <213> Artificial Sequence
<220>
   <221> source
   <223> /note="Description of Artificial Sequence: Synthetic oligonucleotide"
<400> 267
   cattcaggag tgtacacatt tcagc 25
<210> 268
   <211> 25
   <212> DNA
   <213> Artificial Sequence
<220>
   <221> source
   <223> /note="Description of Artificial Sequence: Synthetic oligonucleotide"
<400> 268
   cattcaggag tgtacacatt tccgc 25
<210> 269
   <211> 25
   <212> DNA
   <213> Artificial Sequence
<220>
   <221> source
   <223> /note="Description of Artificial Sequence: Synthetic oligonucleotide"
<400> 269
   cattcaggag tgtacacatt tcggc 25
<210> 270
   <211> 25
   <212> DNA
   <213> Artificial Sequence
<220>
   <221> source
   <223> /note="Description of Artificial Sequence: Synthetic oligonucleotide"
<400> 270
   cattcaggag tgtacacatt tatgc 25
<210> 271
   <211> 25
   <212> DNA
   <213> Artificial Sequence
<220>
   <221> source
   <223> /note="Description of Artificial Sequence: Synthetic oligonucleotide"
<400> 271
   cattcaggag tgtacacatt tgtgc 25
<210> 272
   <211> 25
   <212> DNA
   <213> Artificial Sequence
<220>
   <221> source
   <223> /note="Description of Artificial Sequence: Synthetic oligonucleotide"
<400> 272
   cattcaggag tgtacacatt tttgc 25
<210> 273
   <211> 30
   <212> DNA
   <213> Artificial Sequence
<220>
   <221> source
   <223> /note="Description of Artificial Sequence: Synthetic oligonucleotide"
<400> 273
   tgtgtacact cctgaatgcg cagagagaga 30
<210> 274
   <211> 18
   <212> DNA
   <213> Artificial Sequence
<220>
   <221> source
   <223> /note="Description of Artificial Sequence: Synthetic oligonucleotide"
<400> 274
   agacttcagg gtgctggg 18
<210> 275
   <211> 19
   <212> DNA
   <213> Artificial Sequence
<220>
   <221> source
   <223> /note="Description of Artificial Sequence: Synthetic oligonucleotide"
<400> 275
   cagacttcag ggtgctgag 19
<210> 276
   <211> 18
   <212> DNA
   <213> Artificial Sequence
<220>
   <221> source
   <223> /note="Description of Artificial Sequence: Synthetic oligonucleotide"
<400> 276
   agacttcagg gtgctgcg 18
<210> 277
   <211> 18
   <212> DNA
   <213> Artificial Sequence
<220>
   <221> source
   <223> /note="Description of Artificial Sequence: Synthetic oligonucleotide"
<400> 277
   agacttcagg gtgctgtg 18
<210> 278
   <211> 18
   <212> DNA
   <213> Artificial Sequence
<220>
   <221> source
   <223> /note="Description of Artificial Sequence: Synthetic oligonucleotide"
<400> 278
   agacttcagg gtgctagg 18
<210> 279
   <211> 18
   <212> DNA
   <213> Artificial Sequence
<220>
   <221> source
   <223> /note="Description of Artificial Sequence: Synthetic oligonucleotide"
<400> 279
   agacttcagg gtgctcgg 18
<210> 280
   <211> 18
   <212> DNA
   <213> Artificial Sequence
<220>
   <221> source
   <223> /note="Description of Artificial Sequence: Synthetic oligonucleotide"
<400> 280
   agacttcagg gtgcttgg 18
<210> 281
   <211> 18
   <212> DNA
   <213> Artificial Sequence
<220>
   <221> source
   <223> /note="Description of Artificial Sequence: Synthetic oligonucleotide"
<400> 281
   agacttcagg gtgcaggg 18
<210> 282
   <211> 18
   <212> DNA
   <213> Artificial Sequence
<220>
   <221> source
   <223> /note="Description of Artificial Sequence: Synthetic oligonucleotide"
<400> 282
   agacttcagg gtgccggg 18
<210> 283
   <211> 18
   <212> DNA
   <213> Artificial Sequence
<220>
   <221> source
   <223> /note="Description of Artificial Sequence: Synthetic oligonucleotide"
<400> 283
   agacttcagg gtgcgggg 18
<210> 284
   <211> 21
   <212> DNA
   <213> Artificial Sequence
<220>
   <221> source
   <223> /note="Description of Artificial Sequence: Synthetic oligonucleotide"
<400> 284
   gtcttgtgga agattttctg t 21
<210> 285
   <211> 20
   <212> DNA
   <213> Artificial Sequence
<220>
   <221> source
   <223> /note="Description of Artificial Sequence: Synthetic oligonucleotide"
<400> 285
   ttgaggcaca caaactcctc 20
<210> 286
   <211> 30
   <212> DNA
   <213> Artificial Sequence
<220>
   <221> source
   <223> /note="Description of Artificial Sequence: Synthetic oligonucleotide"
<400> 286
   tggctacatc atctcggttc cgcatgatga 30
<210> 287
   <211> 21
   <212> DNA
   <213> Artificial Sequence
<220>
   <221> source
   <223> /note="Description of Artificial Sequence: Synthetic oligonucleotide"
<400> 287
   atgtgtagag ggcatggaga t 21
<210> 288
   <211> 21
   <212> DNA
   <213> Artificial Sequence
<220>
   <221> source
   <223> /note="Description of Artificial Sequence: Synthetic oligonucleotide"
<400> 288
   atgtgtagag ggcatggagc t 21
<210> 289
   <211> 21
   <212> DNA
   <213> Artificial Sequence
<220>
   <221> source
   <223> /note="Description of Artificial Sequence: Synthetic oligonucleotide"
<400> 289
   atgtgtagag ggcatggagg t 21
<210> 290
   <211> 21
   <212> DNA
   <213> Artificial Sequence
<220>
   <221> source
   <223> /note="Description of Artificial Sequence: Synthetic oligonucleotide"
<400> 290
   atgtgtagag ggcatggagt t 21
<210> 291
   <211> 21
   <212> DNA
   <213> Artificial Sequence
<220>
   <221> source
   <223> /note="Description of Artificial Sequence: Synthetic oligonucleotide"
<400> 291
   atgtgtagag ggcatggaaa t 21
<210> 292
   <211> 21
   <212> DNA
   <213> Artificial Sequence
<220>
   <221> source
   <223> /note="Description of Artificial Sequence: Synthetic oligonucleotide"
<400> 292
   atgtgtagag ggcatggaca t 21
<210> 293
   <211> 21
   <212> DNA
   <213> Artificial Sequence
<220>
   <221> source
   <223> /note="Description of Artificial Sequence: Synthetic oligonucleotide"
<400> 293
   atgtgtagag ggcatggata t 21
<210> 294
   <211> 21
   <212> DNA
   <213> Artificial Sequence
<220>
   <221> source
   <223> /note="Description of Artificial Sequence: Synthetic oligonucleotide"
<400> 294
   atgtgtagag ggcatggcga t 21
<210> 295
   <211> 21
   <212> DNA
   <213> Artificial Sequence
<220>
   <221> source
   <223> /note="Description of Artificial Sequence: Synthetic oligonucleotide"
<400> 295
   atgtgtagag ggcatgggga t 21
<210> 296
   <211> 21
   <212> DNA
   <213> Artificial Sequence
<220>
   <221> source
   <223> /note="Description of Artificial Sequence: Synthetic oligonucleotide"
<400> 296
   atgtgtagag ggcatggtga t 21
<210> 297
   <211> 23
   <212> DNA
   <213> Artificial Sequence
<220>
   <221> source
   <223> /note="Description of Artificial Sequence: Synthetic oligonucleotide"
<220>
   <221> Variation
   <222> (13)..(13)
   <223> /replace="deoxyinosine"
<220>
   <221> misc_feature
   <222> (1)..(23)
   <223> /note="Variant nucleotides given in the sequence have no preference with respect to those in the annotations for variant positions"
<400> 297
   aaatgtgtag agggcatggc gat 23
<210> 298
   <211> 23
   <212> DNA
   <213> Artificial Sequence
<220>
   <221> source
   <223> /note="Description of Artificial Sequence: Synthetic oligonucleotide"
<400> 298
   aaatgtgtag agggcatggt gat 23
<210> 299
   <211> 23
   <212> DNA
   <213> Artificial Sequence
<220>
   <221> source
   <223> /note="Description of Artificial Sequence: Synthetic oligonucleotide"
<400> 299
   aaatgtgtag agggcatgta gat 23
<210> 300
   <211> 23
   <212> DNA
   <213> Artificial Sequence
<220>
   <221> source
   <223> /note="Description of Artificial Sequence: Synthetic oligonucleotide"
<400> 300
   aaatgtgtag agggcatgaa gat 23
<210> 301
   <211> 29
   <212> DNA
   <213> Artificial Sequence
<220>
   <221> source
   <223> /note="Description of Artificial Sequence: Synthetic oligonucleotide"
<400> 301
   cctctacaca ttttccctgg ttcctatga 29
<210> 302
   <211> 22
   <212> DNA
   <213> Artificial Sequence
<220>
   <221> source
   <223> /note="Description of Artificial Sequence: Synthetic oligonucleotide"
<400> 302
   gcagcatgtc gaagatctcc at 22
<210> 303
   <211> 22
   <212> DNA
   <213> Artificial Sequence
<220>
   <221> source
   <223> /note="Description of Artificial Sequence: Synthetic oligonucleotide"
<400> 303
   gcagcatgtc gaagatctcc ct 22
<210> 304
   <211> 22
   <212> DNA
   <213> Artificial Sequence
<220>
   <221> source
   <223> /note="Description of Artificial Sequence: Synthetic oligonucleotide"
<400> 304
   gcagcatgtc gaagatctcc gt 22
<210> 305
   <211> 22
   <212> DNA
   <213> Artificial Sequence
<220>
   <221> source
   <223> /note="Description of Artificial Sequence: Synthetic oligonucleotide"
<400> 305
   gcagcatgtc gaagatctcc tt 22
<210> 306
   <211> 22
   <212> DNA
   <213> Artificial Sequence
<220>
   <221> source
   <223> /note="Description of Artificial Sequence: Synthetic oligonucleotide"
<400> 306
   gcagcatgtc gaagatctca at 22
<210> 307
   <211> 22
   <212> DNA
   <213> Artificial Sequence
<220>
   <221> source
   <223> /note="Description of Artificial Sequence: Synthetic oligonucleotide"
<400> 307
   gcagcatgtc gaagatctcg at 22
<210> 308
   <211> 22
   <212> DNA
   <213> Artificial Sequence
<220>
   <221> source
   <223> /note="Description of Artificial Sequence: Synthetic oligonucleotide"
<400> 308
   gcagcatgtc gaagatctct at 22
<210> 309
   <211> 22
   <212> DNA
   <213> Artificial Sequence
<220>
   <221> source
   <223> /note="Description of Artificial Sequence: Synthetic oligonucleotide"
<400> 309
   gcagcatgtc gaagatctac at 22
<210> 310
   <211> 22
   <212> DNA
   <213> Artificial Sequence
<220>
   <221> source
   <223> /note="Description of Artificial Sequence: Synthetic oligonucleotide"
<400> 310
   gcagcatgtc gaagatctgc at 22
<210> 311
   <211> 22
   <212> DNA
   <213> Artificial Sequence
<220>
   <221> source
   <223> /note="Description of Artificial Sequence: Synthetic oligonucleotide"
<400> 311
   gcagcatgtc gaagatcttc at 22
<210> 312
   <211> 22
   <212> DNA
   <213> Artificial Sequence
<220>
   <221> source
   <223> /note="Description of Artificial Sequence: Synthetic oligonucleotide"
<400> 312
   gtagtccttt ctgtgtcttc cc 22
<210> 313
   <211> 22
   <212> DNA
   <213> Artificial Sequence
<220>
   <221> source
   <223> /note="Description of Artificial Sequence: Synthetic oligonucleotide"
<400> 313
   ctttctgtgt cttcccacct ac 22
<210> 314
   <211> 24
   <212> DNA
   <213> Artificial Sequence
<220>
   <221> source
   <223> /note="Description of Artificial Sequence: Synthetic oligonucleotide"
<400> 314
   tgtcttccca cctacagtaa caaa 24
<210> 315
   <211> 27
   <212> DNA
   <213> Artificial Sequence
<220>
   <221> source
   <223> /note="Description of Artificial Sequence: Synthetic oligonucleotide"
<400> 315
   ctctaaagta gtcctttctg tgtcttc 27
<210> 316
   <211> 26
   <212> DNA
   <213> Artificial Sequence
<220>
   <221> source
   <223> /note="Description of Artificial Sequence: Synthetic oligonucleotide"
<400> 316
   tctaaagtag tcctttctgt gtcttc 26
<210> 317
   <211> 25
   <212> DNA
   <213> Artificial Sequence
<220>
   <221> source
   <223> /note="Description of Artificial Sequence: Synthetic oligonucleotide"
<400> 317
   taaagtagtc ctttctgtgt cttcc 25
<210> 318
   <211> 22
   <212> DNA
   <213> Artificial Sequence
<220>
   <221> source
   <223> /note="Description of Artificial Sequence: Synthetic oligonucleotide"
<400> 318
   agtagtcctt tctgtgtctt cc 22
<210> 319
   <211> 17
   <212> DNA
   <213> Artificial Sequence
<220>
   <221> source
   <223> /note="Description of Artificial Sequence: Synthetic oligonucleotide"
<400> 319
   gctagtgggc gcatgta 17
<210> 320
   <211> 16
   <212> DNA
   <213> Artificial Sequence
<220>
   <221> source
   <223> /note="Description of Artificial Sequence: Synthetic oligonucleotide"
<400> 320
   ctagtgggcg catgta 16
<210> 321
   <211> 28
   <212> DNA
   <213> Artificial Sequence
<220>
   <221> source
   <223> /note="Description of Artificial Sequence: Synthetic oligonucleotide"
<400> 321
   tctatgacct gctgctggag atgctgga 28
<210> 322
   <211> 21
   <212> DNA
   <213> Artificial Sequence
<220>
   <221> source
   <223> /note="Description of Artificial Sequence: Synthetic oligonucleotide"
<400> 322
   acagcatgaa gtgcaagaac a 21
<210> 323
   <211> 21
   <212> DNA
   <213> Artificial Sequence
<220>
   <221> source
   <223> /note="Description of Artificial Sequence: Synthetic oligonucleotide"
<400> 323
   acagcatgaa gtgcaagaaa a 21
<210> 324
   <211> 21
   <212> DNA
   <213> Artificial Sequence
<220>
   <221> source
   <223> /note="Description of Artificial Sequence: Synthetic oligonucleotide"
<400> 324
   acagcatgaa gtgcaagaag a 21
<210> 325
   <211> 21
   <212> DNA
   <213> Artificial Sequence
<220>
   <221> source
   <223> /note="Description of Artificial Sequence: Synthetic oligonucleotide"
<400> 325
   acagcatgaa gtgcaagaat a 21
<210> 326
   <211> 21
   <212> DNA
   <213> Artificial Sequence
<220>
   <221> source
   <223> /note="Description of Artificial Sequence: Synthetic oligonucleotide"
<400> 326
   acagcatgaa gtgcaagacc a 21
<210> 327
   <211> 21
   <212> DNA
   <213> Artificial Sequence
<220>
   <221> source
   <223> /note="Description of Artificial Sequence: Synthetic oligonucleotide"
<400> 327
   acagcatgaa gtgcaagagc a 21
<210> 328
   <211> 21
   <212> DNA
   <213> Artificial Sequence
<220>
   <221> source
   <223> /note="Description of Artificial Sequence: Synthetic oligonucleotide"
<400> 328
   acagcatgaa gtgcaagatc a 21
<210> 329
   <211> 21
   <212> DNA
   <213> Artificial Sequence
<220>
   <221> source
   <223> /note="Description of Artificial Sequence: Synthetic oligonucleotide"
<400> 329
   acagcatgaa gtgcaagcac a 21
<210> 330
   <211> 21
   <212> DNA
   <213> Artificial Sequence
<220>
   <221> source
   <223> /note="Description of Artificial Sequence: Synthetic oligonucleotide"
<400> 330
   acagcatgaa gtgcaaggac a 21
<210> 331
   <211> 21
   <212> DNA
   <213> Artificial Sequence
<220>
   <221> source
   <223> /note="Description of Artificial Sequence: Synthetic oligonucleotide"
<400> 331
   acagcatgaa gtgcaagtac a 21
<210> 332
   <211> 24
   <212> DNA
   <213> Artificial Sequence
<220>
   <221> source
   <223> /note="Description of Artificial Sequence: Synthetic oligonucleotide"
<400> 332
   tgtacagcat gaagtgcaag caca 24
<210> 333
   <211> 24
   <212> DNA
   <213> Artificial Sequence
<220>
   <221> source
   <223> /note="Description of Artificial Sequence: Synthetic oligonucleotide"
<400> 333
   tgtacagcat gaagtgcaag gaca 24
<210> 334
   <211> 30
   <212> DNA
   <213> Artificial Sequence
<220>
   <221> source
   <223> /note="Description of Artificial Sequence: Synthetic oligonucleotide"
<400> 334
   tgcacttcat gctgtacaga tgctccatgc 30
<210> 335
   <211> 30
   <212> DNA
   <213> Artificial Sequence
<220>
   <221> source
   <223> /note="Description of Artificial Sequence: Synthetic oligonucleotide"
<400> 335
   tgcacttcat gctgtacaga tgctccatgc 30
<210> 336
   <211> 19
   <212> DNA
   <213> Artificial Sequence
<220>
   <221> source
   <223> /note="Description of Artificial Sequence: Synthetic oligonucleotide"
<400> 336
   ggtcatagag gggcaccat 19
<210> 337
   <211> 19
   <212> DNA
   <213> Artificial Sequence
<220>
   <221> source
   <223> /note="Description of Artificial Sequence: Synthetic oligonucleotide"
<400> 337
   ggtcatagag gggcaccat 19
<210> 338
   <211> 19
   <212> DNA
   <213> Artificial Sequence
<220>
   <221> source
   <223> /note="Description of Artificial Sequence: Synthetic oligonucleotide"
<400> 338
   ggtcatagag gggcaccct 19
<210> 339
   <211> 19
   <212> DNA
   <213> Artificial Sequence
<220>
   <221> source
   <223> /note="Description of Artificial Sequence: Synthetic oligonucleotide"
<400> 339
   ggtcatagag gggcaccmt 19
<210> 340
   <211> 19
   <212> DNA
   <213> Artificial Sequence
<220>
   <221> source
   <223> /note="Description of Artificial Sequence: Synthetic oligonucleotide"
<400> 340
   ggtcatagag gggcaccgt 19
<210> 341
   <211> 19
   <212> DNA
   <213> Artificial Sequence
<220>
   <221> source
   <223> /note="Description of Artificial Sequence: Synthetic oligonucleotide"
<400> 341
   ggtcatagag gggcacctt 19
<210> 342
   <211> 19
   <212> DNA
   <213> Artificial Sequence
<220>
   <221> source
   <223> /note="Description of Artificial Sequence: Synthetic oligonucleotide"
<400> 342
   ggtcatagag gggcacaat 19
<210> 343
   <211> 19
   <212> DNA
   <213> Artificial Sequence
<220>
   <221> source
   <223> /note="Description of Artificial Sequence: Synthetic oligonucleotide"
<400> 343
   ggtcatagag gggcacgat 19
<210> 344
   <211> 19
   <212> DNA
   <213> Artificial Sequence
<220>
   <221> source
   <223> /note="Description of Artificial Sequence: Synthetic oligonucleotide"
<400> 344
   ggtcatagag gggcactat 19
<210> 345
   <211> 19
   <212> DNA
   <213> Artificial Sequence
<220>
   <221> source
   <223> /note="Description of Artificial Sequence: Synthetic oligonucleotide"
<400> 345
   ggtcatagag gggcaacat 19
<210> 346
   <211> 19
   <212> DNA
   <213> Artificial Sequence
<220>
   <221> source
   <223> /note="Description of Artificial Sequence: Synthetic oligonucleotide"
<400> 346
   ggtcatagag gggcagcat 19
<210> 347
   <211> 19
   <212> DNA
   <213> Artificial Sequence
<220>
   <221> source
   <223> /note="Description of Artificial Sequence: Synthetic oligonucleotide"
<400> 347
   ggtcatagag gggcatcat 19
<210> 348
   <211> 20
   <212> DNA
   <213> Artificial Sequence
<220>
   <221> source
   <223> /note="Description of Artificial Sequence: Synthetic oligonucleotide"
<400> 348
   aggtcataga ggggcagcat 20
<210> 349
   <211> 20
   <212> DNA
   <213> Artificial Sequence
<220>
   <221> source
   <223> /note="Description of Artificial Sequence: Synthetic oligonucleotide"
<400> 349
   aggtcataga ggggctccat 20
<210> 350
   <211> 20
   <212> DNA
   <213> Artificial Sequence
<220>
   <221> source
   <223> /note="Description of Artificial Sequence: Synthetic oligonucleotide"
<400> 350
   aggtcataga ggggcgccat 20
<210> 351
   <211> 20
   <212> DNA
   <213> Artificial Sequence
<220>
   <221> source
   <223> /note="Description of Artificial Sequence: Synthetic oligonucleotide"
<400> 351
   aggtcataga ggggccccat 20
<210> 352
   <211> 21
   <212> DNA
   <213> Artificial Sequence
<220>
   <221> source
   <223> /note="Description of Artificial Sequence: Synthetic oligonucleotide"
<400> 352
   caggtcatag aggggctcca t 21
<210> 353
   <211> 21
   <212> DNA
   <213> Artificial Sequence
<220>
   <221> source
   <223> /note="Description of Artificial Sequence: Synthetic oligonucleotide"
<400> 353
   caggtcatag aggggcgcca t 21
<210> 354
   <211> 21
   <212> DNA
   <213> Artificial Sequence
<220>
   <221> source
   <223> /note="Description of Artificial Sequence: Synthetic oligonucleotide"
<400> 354
   caggtcatag aggggcccca t 21
<210> 355
   <211> 21
   <212> DNA
   <213> Artificial Sequence
<220>
   <221> source
   <223> /note="Description of Artificial Sequence: Synthetic oligonucleotide"
<400> 355
   acagcatgaa gtgcaagaac a 21
<210> 356
   <211> 21
   <212> DNA
   <213> Artificial Sequence
<220>
   <221> source
   <223> /note="Description of Artificial Sequence: Synthetic oligonucleotide"
<400> 356
   acagcatgaa gtgcaagaac a 21
<210> 357
   <211> 22
   <212> DNA
   <213> Artificial Sequence
<220>
   <221> source
   <223> /note="Description of Artificial Sequence: Synthetic oligonucleotide"
<400> 357
   ctttctgtgt cttcccacct ac 22
<210> 358
   <211> 17
   <212> DNA
   <213> Artificial Sequence
<220>
   <221> source
   <223> /note="Description of Artificial Sequence: Synthetic oligonucleotide"
<400> 358
   gctttggtcc gtctcct 17
<210> 359
   <211> 19
   <212> DNA
   <213> Artificial Sequence
<220>
   <221> source
   <223> /note="Description of Artificial Sequence: Synthetic oligonucleotide"
<400> 359
   tggctttggt ccgtctcct 19
<210> 360
   <211> 18
   <212> DNA
   <213> Artificial Sequence
<220>
   <221> source
   <223> /note="Description of Artificial Sequence: Synthetic oligonucleotide"
<400> 360
   atgtaggcgg tgggcgtc 18
<210> 361
   <211> 18
   <212> DNA
   <213> Artificial Sequence
<220>
   <221> source
   <223> /note="Description of Artificial Sequence: Synthetic oligonucleotide"
<400> 361
   ctccacggct agtgggcg 18
<210> 362
   <211> 18
   <212> DNA
   <213> Artificial Sequence
<220>
   <221> source
   <223> /note="Description of Artificial Sequence: Synthetic oligonucleotide"
<400> 362
   tgcccctcca cggctagt 18
<210> 363
   <211> 28
   <212> DNA
   <213> Artificial Sequence
<220>
   <221> source
   <223> /note="Description of Artificial Sequence: Synthetic oligonucleotide"
<400> 363
   tctatgacct gctgctggag atgctgga 28
<210> 364
   <211> 22
   <212> DNA
   <213> Artificial Sequence
<220>
   <221> source
   <223> /note="Description of Artificial Sequence: Synthetic oligonucleotide"
<400> 364
   gcatgaagtg caagaacgtg ga 22
<210> 365
   <211> 22
   <212> DNA
   <213> Artificial Sequence
<220>
   <221> source
   <223> /note="Description of Artificial Sequence: Synthetic oligonucleotide"
<400> 365
   gcatgaagtg caagaacgtg aa 22
<210> 366
   <211> 22
   <212> DNA
   <213> Artificial Sequence
<220>
   <221> source
   <223> /note="Description of Artificial Sequence: Synthetic oligonucleotide"
<400> 366
   gcatgaagtg caagaacgtg ca 22
<210> 367
   <211> 22
   <212> DNA
   <213> Artificial Sequence
<220>
   <221> source
   <223> /note="Description of Artificial Sequence: Synthetic oligonucleotide"
<400> 367
   gcatgaagtg caagaacgtg ta 22
<210> 368
   <211> 22
   <212> DNA
   <213> Artificial Sequence
<220>
   <221> source
   <223> /note="Description of Artificial Sequence: Synthetic oligonucleotide"
<400> 368
   gcatgaagtg caagaacgta ga 22
<210> 369
   <211> 22
   <212> DNA
   <213> Artificial Sequence
<220>
   <221> source
   <223> /note="Description of Artificial Sequence: Synthetic oligonucleotide"
<400> 369
   gcatgaagtg caagaacgtc ga 22
<210> 370
   <211> 22
   <212> DNA
   <213> Artificial Sequence
<220>
   <221> source
   <223> /note="Description of Artificial Sequence: Synthetic oligonucleotide"
<400> 370
   gcatgaagtg caagaacgtt ga 22
<210> 371
   <211> 22
   <212> DNA
   <213> Artificial Sequence
<220>
   <221> source
   <223> /note="Description of Artificial Sequence: Synthetic oligonucleotide"
<400> 371
   gcatgaagtg caagaacgag ga 22
<210> 372
   <211> 22
   <212> DNA
   <213> Artificial Sequence
<220>
   <221> source
   <223> /note="Description of Artificial Sequence: Synthetic oligonucleotide"
<400> 372
   gcatgaagtg caagaacgcg ga 22
<210> 373
   <211> 22
   <212> DNA
   <213> Artificial Sequence
<220>
   <221> source
   <223> /note="Description of Artificial Sequence: Synthetic oligonucleotide"
<400> 373
   gcatgaagtg caagaacggg ga 22
<210> 374
   <211> 29
   <212> DNA
   <213> Artificial Sequence
<220>
   <221> source
   <223> /note="Description of Artificial Sequence: Synthetic oligonucleotide"
<400> 374
   tgcacttcat gctgtacaga tgctccatg 29
<210> 375
   <211> 35
   <212> DNA
   <213> Artificial Sequence
<220>
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<400> 375
   tgcacttcau uugctguuua cagatgcuuu ccatg 35
<210> 376
   <211> 28
   <212> DNA
   <213> Artificial Sequence
<220>
   <221> source
   <223> /note="Description of Artificial Sequence: Synthetic oligonucleotide"
<400> 376
   tgcacttcat gctgtacaga tgctccat 28
<210> 377
   <211> 40
   <212> DNA
   <213> Artificial Sequence
<220>
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<400> 377
   tgcacttcau uuugctguuu uacagauuuu gctccauuuu 40
<210> 378
   <211> 18
   <212> DNA
   <213> Artificial Sequence
<220>
   <221> source
   <223> /note="Description of Artificial Sequence: Synthetic oligonucleotide"
<400> 378
   gcaggtcata gaggggct 18
<210> 379
   <211> 18
   <212> DNA
   <213> Artificial Sequence
<220>
   <221> source
   <223> /note="Description of Artificial Sequence: Synthetic oligonucleotide"
<400> 379
   gcaggtcata gaggggat 18
<210> 380
   <211> 18
   <212> DNA
   <213> Artificial Sequence
<220>
   <221> source
   <223> /note="Description of Artificial Sequence: Synthetic oligonucleotide"
<400> 380
   gcaggtcata gagggggt 18
<210> 381
   <211> 18
   <212> DNA
   <213> Artificial Sequence
<220>
   <221> source
   <223> /note="Description of Artificial Sequence: Synthetic oligonucleotide"
<400> 381
   gcaggtcata gaggggtt 18
<210> 382
   <211> 18
   <212> DNA
   <213> Artificial Sequence
<220>
   <221> source
   <223> /note="Description of Artificial Sequence: Synthetic oligonucleotide"
<400> 382
   gcaggtcata gagggact 18
<210> 383
   <211> 18
   <212> DNA
   <213> Artificial Sequence
<220>
   <221> source
   <223> /note="Description of Artificial Sequence: Synthetic oligonucleotide"
<400> 383
   gcaggtcata gagggcct 18
<210> 384
   <211> 18
   <212> DNA
   <213> Artificial Sequence
<220>
   <221> source
   <223> /note="Description of Artificial Sequence: Synthetic oligonucleotide"
<400> 384
   gcaggtcata gagggtct 18
<210> 385
   <211> 18
   <212> DNA
   <213> Artificial Sequence
<220>
   <221> source
   <223> /note="Description of Artificial Sequence: Synthetic oligonucleotide"
<400> 385
   gcaggtcata gaggagct 18
<210> 386
   <211> 18
   <212> DNA
   <213> Artificial Sequence
<220>
   <221> source
   <223> /note="Description of Artificial Sequence: Synthetic oligonucleotide"
<400> 386
   gcaggtcata gaggcgct 18
<210> 387
   <211> 18
   <212> DNA
   <213> Artificial Sequence
<220>
   <221> source
   <223> /note="Description of Artificial Sequence: Synthetic oligonucleotide"
<400> 387
   gcaggtcata gaggtgct 18
<210> 388
   <211> 18
   <212> DNA
   <213> Artificial Sequence
<220>
   <221> source
   <223> /note="Description of Artificial Sequence: Synthetic oligonucleotide"
<400> 388
   gcaggtcata gaggggct 18
<210> 389
   <211> 18
   <212> DNA
   <213> Artificial Sequence
<220>
   <221> source
   <223> /note="Description of Artificial Sequence: Synthetic oligonucleotide"
<400> 389
   gcaggtcata gagtggct 18
<210> 390
   <211> 18
   <212> DNA
   <213> Artificial Sequence
<220>
   <221> source
   <223> /note="Description of Artificial Sequence: Synthetic oligonucleotide"
<400> 390
   gcaggtcata gagcggct 18
<210> 391
   <211> 20
   <212> DNA
   <213> Artificial Sequence
<220>
   <221> source
   <223> /note="Description of Artificial Sequence: Synthetic oligonucleotide"
<400> 391
   cagcaggtca tagagaggct 20
<210> 392
   <211> 20
   <212> DNA
   <213> Artificial Sequence
<220>
   <221> source
   <223> /note="Description of Artificial Sequence: Synthetic oligonucleotide"
<400> 392
   cagcaggtca tagagtggct 20
<210> 393
   <211> 20
   <212> DNA
   <213> Artificial Sequence
<220>
   <221> source
   <223> /note="Description of Artificial Sequence: Synthetic oligonucleotide"
<400> 393
   cagcaggtca tagagcggct 20
<210> 394
   <211> 22
   <212> DNA
   <213> Artificial Sequence
<220>
   <221> source
   <223> /note="Description of Artificial Sequence: Synthetic oligonucleotide"
<400> 394
   ctgtgtcttc ccacctacag ta 22
<210> 395
   <211> 17
   <212> DNA
   <213> Artificial Sequence
<220>
   <221> source
   <223> /note="Description of Artificial Sequence: Synthetic oligonucleotide"
<400> 395
   aagtggcttt ggtccgt 17
<210> 396
   <211> 23
   <212> DNA
   <213> Artificial Sequence
<220>
   <221> source
   <223> /note="Description of Artificial Sequence: Synthetic oligonucleotide"
<400> 396
   tacatgcgcc cactagccgt gga 23
<210> 397
   <211> 16
   <212> DNA
   <213> Artificial Sequence
<220>
   <221> source
   <223> /note="Description of Artificial Sequence: Synthetic oligonucleotide"
<400> 397
   acgtggtgcc cctctg 16
<210> 398
   <211> 16
   <212> DNA
   <213> Artificial Sequence
<220>
   <221> source
   <223> /note="Description of Artificial Sequence: Synthetic oligonucleotide"
<400> 398
   acgtggtgcc cctcag 16
<210> 399
   <211> 16
   <212> DNA
   <213> Artificial Sequence
<220>
   <221> source
   <223> /note="Description of Artificial Sequence: Synthetic oligonucleotide"
<400> 399
   acgtggtgcc cctccg 16
<210> 400
   <211> 16
   <212> DNA
   <213> Artificial Sequence
<220>
   <221> source
   <223> /note="Description of Artificial Sequence: Synthetic oligonucleotide"
<400> 400
   acgtggtgcc cctcgg 16
<210> 401
   <211> 16
   <212> DNA
   <213> Artificial Sequence
<220>
   <221> source
   <223> /note="Description of Artificial Sequence: Synthetic oligonucleotide"
<400> 401
   acgtggtgcc cctatg 16
<210> 402
   <211> 16
   <212> DNA
   <213> Artificial Sequence
<220>
   <221> source
   <223> /note="Description of Artificial Sequence: Synthetic oligonucleotide"
<400> 402
   acgtggtgcc cctgtg 16
<210> 403
   <211> 16
   <212> DNA
   <213> Artificial Sequence
<220>
   <221> source
   <223> /note="Description of Artificial Sequence: Synthetic oligonucleotide"
<400> 403
   acgtggtgcc cctttg 16
<210> 404
   <211> 16
   <212> DNA
   <213> Artificial Sequence
<220>
   <221> source
   <223> /note="Description of Artificial Sequence: Synthetic oligonucleotide"
<400> 404
   acgtggtgcc ccactg 16
<210> 405
   <211> 16
   <212> DNA
   <213> Artificial Sequence
<220>
   <221> source
   <223> /note="Description of Artificial Sequence: Synthetic oligonucleotide"
<400> 405
   acgtggtgcc cccctg 16
<210> 406
   <211> 16
   <212> DNA
   <213> Artificial Sequence
<220>
   <221> source
   <223> /note="Description of Artificial Sequence: Synthetic oligonucleotide"
<400> 406
   acgtggtgcc ccgctg 16
<210> 407
   <211> 30
   <212> DNA
   <213> Artificial Sequence
<220>
   <221> source
   <223> /note="Description of Artificial Sequence: Synthetic oligonucleotide"
<400> 407
   tgcacttcat gctgtacaga tgctccatgc 30
<210> 408
   <211> 17
   <212> DNA
   <213> Artificial Sequence
<220>
   <221> source
   <223> /note="Description of Artificial Sequence: Synthetic oligonucleotide"
<400> 408
   ctccagcagc aggtcac 17
<210> 409
   <211> 17
   <212> DNA
   <213> Artificial Sequence
<220>
   <221> source
   <223> /note="Description of Artificial Sequence: Synthetic oligonucleotide"
<400> 409
   ctccagcagc aggtccc 17
<210> 410
   <211> 17
   <212> DNA
   <213> Artificial Sequence
<220>
   <221> source
   <223> /note="Description of Artificial Sequence: Synthetic oligonucleotide"
<400> 410
   ctccagcagc aggtcgc 17
<210> 411
   <211> 17
   <212> DNA
   <213> Artificial Sequence
<220>
   <221> source
   <223> /note="Description of Artificial Sequence: Synthetic oligonucleotide"
<400> 411
   ctccagcagc aggtctc 17
<210> 412
   <211> 17
   <212> DNA
   <213> Artificial Sequence
<220>
   <221> source
   <223> /note="Description of Artificial Sequence: Synthetic oligonucleotide"
<400> 412
   ctccagcagc aggtaac 17
<210> 413
   <211> 17
   <212> DNA
   <213> Artificial Sequence
<220>
   <221> source
   <223> /note="Description of Artificial Sequence: Synthetic oligonucleotide"
<400> 413
   ctccagcagc aggtgac 17
<210> 414
   <211> 17
   <212> DNA
   <213> Artificial Sequence
<220>
   <221> source
   <223> /note="Description of Artificial Sequence: Synthetic oligonucleotide"
<400> 414
   ctccagcagc aggttac 17
<210> 415
   <211> 17
   <212> DNA
   <213> Artificial Sequence
<220>
   <221> source
   <223> /note="Description of Artificial Sequence: Synthetic oligonucleotide"
<400> 415
   ctccagcagc aggacac 17
<210> 416
   <211> 17
   <212> DNA
   <213> Artificial Sequence
<220>
   <221> source
   <223> /note="Description of Artificial Sequence: Synthetic oligonucleotide"
<400> 416
   ctccagcagc aggccac 17
<210> 417
   <211> 17
   <212> DNA
   <213> Artificial Sequence
<220>
   <221> source
   <223> /note="Description of Artificial Sequence: Synthetic oligonucleotide"
<400> 417
   ctccagcagc agggcac 17
<210> 418
   <211> 19
   <212> DNA
   <213> Artificial Sequence
<220>
   <221> source
   <223> /note="Description of Artificial Sequence: Synthetic oligonucleotide"
<400> 418
   atctccagca gcaggacac 19
<210> 419
   <211> 20
   <212> DNA
   <213> Artificial Sequence
<220>
   <221> source
   <223> /note="Description of Artificial Sequence: Synthetic oligonucleotide"
<400> 419
   catctccagc agcaggacac 20
<210> 420
   <211> 19
   <212> DNA
   <213> Artificial Sequence
<220>
   <221> source
   <223> /note="Description of Artificial Sequence: Synthetic oligonucleotide"
<400> 420
   atctccagca gcaggtcac 19
<210> 421
   <211> 19
   <212> DNA
   <213> Artificial Sequence
<220>
   <221> source
   <223> /note="Description of Artificial Sequence: Synthetic oligonucleotide"
<400> 421
   atctccagca gcaggtcac 19
<210> 422
   <211> 19
   <212> DNA
   <213> Artificial Sequence
<220>
   <221> source
   <223> /note="Description of Artificial Sequence: Synthetic oligonucleotide"
<400> 422
   atctccagca gcaggtcac 19
<210> 423
   <211> 17
   <212> DNA
   <213> Artificial Sequence
<220>
   <221> source
   <223> /note="Description of Artificial Sequence: Synthetic oligonucleotide"
<400> 423
   ctccagcagc aggtcac 17
<210> 424
   <211> 17
   <212> DNA
   <213> Artificial Sequence
<220>
   <221> source
   <223> /note="Description of Artificial Sequence: Synthetic oligonucleotide"
<400> 424
   ctccagcagc aggtcac 17
<210> 425
   <211> 17
   <212> DNA
   <213> Artificial Sequence
<220>
   <221> source
   <223> /note="Description of Artificial Sequence: Synthetic oligonucleotide"
<400> 425
   ctccagcagc aggtcac 17
<210> 426
   <211> 16
   <212> DNA
   <213> Artificial Sequence
<220>
   <221> source
   <223> /note="Description of Artificial Sequence: Synthetic oligonucleotide"
<400> 426
   aacgtggtgc ccctca 16
<210> 427
   <211> 16
   <212> DNA
   <213> Artificial Sequence
<220>
   <221> source
   <223> /note="Description of Artificial Sequence: Synthetic oligonucleotide"
<400> 427
   aacgtggtgc ccctaa 16
<210> 428
   <211> 16
   <212> DNA
   <213> Artificial Sequence
<220>
   <221> source
   <223> /note="Description of Artificial Sequence: Synthetic oligonucleotide"
<400> 428
   aacgtggtgc ccctga 16
<210> 429
   <211> 16
   <212> DNA
   <213> Artificial Sequence
<220>
   <221> source
   <223> /note="Description of Artificial Sequence: Synthetic oligonucleotide"
<400> 429
   aacgtggtgc ccctta 16
<210> 430
   <211> 16
   <212> DNA
   <213> Artificial Sequence
<220>
   <221> source
   <223> /note="Description of Artificial Sequence: Synthetic oligonucleotide"
<400> 430
   aacgtggtgc cccaca 16
<210> 431
   <211> 16
   <212> DNA
   <213> Artificial Sequence
<220>
   <221> source
   <223> /note="Description of Artificial Sequence: Synthetic oligonucleotide"
<400> 431
   aacgtggtgc ccccca 16
<210> 432
   <211> 16
   <212> DNA
   <213> Artificial Sequence
<220>
   <221> source
   <223> /note="Description of Artificial Sequence: Synthetic oligonucleotide"
<400> 432
   aacgtggtgc cccgca 16
<210> 433
   <211> 16
   <212> DNA
   <213> Artificial Sequence
<220>
   <221> source
   <223> /note="Description of Artificial Sequence: Synthetic oligonucleotide"
<400> 433
   aacgtggtgc ccatca 16
<210> 434
   <211> 16
   <212> DNA
   <213> Artificial Sequence
<220>
   <221> source
   <223> /note="Description of Artificial Sequence: Synthetic oligonucleotide"
<400> 434
   aacgtggtgc ccgtca 16
<210> 435
   <211> 16
   <212> DNA
   <213> Artificial Sequence
<220>
   <221> source
   <223> /note="Description of Artificial Sequence: Synthetic oligonucleotide"
<400> 435
   aacgtggtgc ccttca 16
<210> 436
   <211> 18
   <212> DNA
   <213> Artificial Sequence
<220>
   <221> source
   <223> /note="Description of Artificial Sequence: Synthetic oligonucleotide"
<400> 436
   ctccagcagc aggtcatt 18
<210> 437
   <211> 18
   <212> DNA
   <213> Artificial Sequence
<220>
   <221> source
   <223> /note="Description of Artificial Sequence: Synthetic oligonucleotide"
<400> 437
   ctccagcagc aggtcaat 18
<210> 438
   <211> 18
   <212> DNA
   <213> Artificial Sequence
<220>
   <221> source
   <223> /note="Description of Artificial Sequence: Synthetic oligonucleotide"
<400> 438
   ctccagcagc aggtcact 18
<210> 439
   <211> 18
   <212> DNA
   <213> Artificial Sequence
<220>
   <221> source
   <223> /note="Description of Artificial Sequence: Synthetic oligonucleotide"
<400> 439
   ctccagcagc aggtcagt 18
<210> 440
   <211> 18
   <212> DNA
   <213> Artificial Sequence
<220>
   <221> source
   <223> /note="Description of Artificial Sequence: Synthetic oligonucleotide"
<400> 440
   ctccagcagc aggtcctt 18
<210> 441
   <211> 18
   <212> DNA
   <213> Artificial Sequence
<220>
   <221> source
   <223> /note="Description of Artificial Sequence: Synthetic oligonucleotide"
<400> 441
   ctccagcagc aggtcgtt 18
<210> 442
   <211> 18
   <212> DNA
   <213> Artificial Sequence
<220>
   <221> source
   <223> /note="Description of Artificial Sequence: Synthetic oligonucleotide"
<400> 442
   ctccagcagc aggtcttt 18
<210> 443
   <211> 18
   <212> DNA
   <213> Artificial Sequence
<220>
   <221> source
   <223> /note="Description of Artificial Sequence: Synthetic oligonucleotide"
<400> 443
   ctccagcagc aggtaatt 18
<210> 444
   <211> 18
   <212> DNA
   <213> Artificial Sequence
<220>
   <221> source
   <223> /note="Description of Artificial Sequence: Synthetic oligonucleotide"
<400> 444
   ctccagcagc aggtgatt 18
<210> 445
   <211> 18
   <212> DNA
   <213> Artificial Sequence
<220>
   <221> source
   <223> /note="Description of Artificial Sequence: Synthetic oligonucleotide"
<400> 445
   ctccagcagc aggttatt 18
<210> 446
   <211> 20
   <212> DNA
   <213> Artificial Sequence
<220>
   <221> source
   <223> /note="Description of Artificial Sequence: Synthetic oligonucleotide"
<400> 446
   atctccagca gcaggttatt 20
<210> 447
   <211> 21
   <212> DNA
   <213> Artificial Sequence
<220>
   <221> source
   <223> /note="Description of Artificial Sequence: Synthetic oligonucleotide"
<400> 447
   catctccagc agcaggttat t 21
<210> 448
   <211> 22
   <212> DNA
   <213> Artificial Sequence
<220>
   <221> source
   <223> /note="Description of Artificial Sequence: Synthetic oligonucleotide"
<400> 448
   gcatctccag cagcaggtta tt 22
<210> 449
   <211> 16
   <212> DNA
   <213> Artificial Sequence
<220>
   <221> source
   <223> /note="Description of Artificial Sequence: Synthetic oligonucleotide"
<400> 449
   acgtggtgcc cctctc 16
<210> 450
   <211> 16
   <212> DNA
   <213> Artificial Sequence
<220>
   <221> source
   <223> /note="Description of Artificial Sequence: Synthetic oligonucleotide"
<400> 450
   acgtggtgcc cctcac 16
<210> 451
   <211> 16
   <212> DNA
   <213> Artificial Sequence
<220>
   <221> source
   <223> /note="Description of Artificial Sequence: Synthetic oligonucleotide"
<400> 451
   acgtggtgcc cctccc 16
<210> 452
   <211> 16
   <212> DNA
   <213> Artificial Sequence
<220>
   <221> source
   <223> /note="Description of Artificial Sequence: Synthetic oligonucleotide"
<400> 452
   acgtggtgcc cctcgc 16
<210> 453
   <211> 16
   <212> DNA
   <213> Artificial Sequence
<220>
   <221> source
   <223> /note="Description of Artificial Sequence: Synthetic oligonucleotide"
<400> 453
   acgtggtgcc cctatc 16
<210> 454
   <211> 16
   <212> DNA
   <213> Artificial Sequence
<220>
   <221> source
   <223> /note="Description of Artificial Sequence: Synthetic oligonucleotide"
<400> 454
   acgtggtgcc cctgtc 16
<210> 455
   <211> 16
   <212> DNA
   <213> Artificial Sequence
<220>
   <221> source
   <223> /note="Description of Artificial Sequence: Synthetic oligonucleotide"
<400> 455
   acgtggtgcc cctttc 16
<210> 456
   <211> 16
   <212> DNA
   <213> Artificial Sequence
<220>
   <221> source
   <223> /note="Description of Artificial Sequence: Synthetic oligonucleotide"
<400> 456
   acgtggtgcc ccactc 16
<210> 457
   <211> 16
   <212> DNA
   <213> Artificial Sequence
<220>
   <221> source
   <223> /note="Description of Artificial Sequence: Synthetic oligonucleotide"
<400> 457
   acgtggtgcc cccctc 16
<210> 458
   <211> 16
   <212> DNA
   <213> Artificial Sequence
<220>
   <221> source
   <223> /note="Description of Artificial Sequence: Synthetic oligonucleotide"
<400> 458
   acgtggtgcc ccgctc 16
<210> 459
   <211> 19
   <212> DNA
   <213> Artificial Sequence
<220>
   <221> source
   <223> /note="Description of Artificial Sequence: Synthetic oligonucleotide"
<400> 459
   atctccagca gcaggtcag 19
<210> 460
   <211> 19
   <212> DNA
   <213> Artificial Sequence
<220>
   <221> source
   <223> /note="Description of Artificial Sequence: Synthetic oligonucleotide"
<400> 460
   atctccagca gcaggtccg 19
<210> 461
   <211> 19
   <212> DNA
   <213> Artificial Sequence
<220>
   <221> source
   <223> /note="Description of Artificial Sequence: Synthetic oligonucleotide"
<400> 461
   atctccagca gcaggtcgg 19
<210> 462
   <211> 19
   <212> DNA
   <213> Artificial Sequence
<220>
   <221> source
   <223> /note="Description of Artificial Sequence: Synthetic oligonucleotide"
<400> 462
   atctccagca gcaggtctg 19
<210> 463
   <211> 19
   <212> DNA
   <213> Artificial Sequence
<220>
   <221> source
   <223> /note="Description of Artificial Sequence: Synthetic oligonucleotide"
<400> 463
   atctccagca gcaggtaag 19
<210> 464
   <211> 19
   <212> DNA
   <213> Artificial Sequence
<220>
   <221> source
   <223> /note="Description of Artificial Sequence: Synthetic oligonucleotide"
<400> 464
   atctccagca gcaggtgag 19
<210> 465
   <211> 19
   <212> DNA
   <213> Artificial Sequence
<220>
   <221> source
   <223> /note="Description of Artificial Sequence: Synthetic oligonucleotide"
<400> 465
   atctccagca gcaggttag 19
<210> 466
   <211> 19
   <212> DNA
   <213> Artificial Sequence
<220>
   <221> source
   <223> /note="Description of Artificial Sequence: Synthetic oligonucleotide"
<400> 466
   atctccagca gcaggacag 19
<210> 467
   <211> 19
   <212> DNA
   <213> Artificial Sequence
<220>
   <221> source
   <223> /note="Description of Artificial Sequence: Synthetic oligonucleotide"
<400> 467
   atctccagca gcaggccag 19
<210> 468
   <211> 19
   <212> DNA
   <213> Artificial Sequence
<220>
   <221> source
   <223> /note="Description of Artificial Sequence: Synthetic oligonucleotide"
<400> 468
   atctccagca gcagggcag 19
<210> 469
   <211> 20
   <212> DNA
   <213> Artificial Sequence
<220>
   <221> source
   <223> /note="Description of Artificial Sequence: Synthetic oligonucleotide"
<400> 469
   catctccagc agcaggacag 20
<210> 470
   <211> 20
   <212> DNA
   <213> Artificial Sequence
<220>
   <221> source
   <223> /note="Description of Artificial Sequence: Synthetic oligonucleotide"
<400> 470
   catctccagc agcagggcag 20
<210> 471
   <211> 21
   <212> DNA
   <213> Artificial Sequence
<220>
   <221> source
   <223> /note="Description of Artificial Sequence: Synthetic oligonucleotide"
<400> 471
   gcatctccag cagcaggaca g 21
<210> 472
   <211> 17
   <212> DNA
   <213> Artificial Sequence
<220>
   <221> source
   <223> /note="Description of Artificial Sequence: Synthetic oligonucleotide"
<400> 472
   ctccagcagc agggcag 17
<210> 473
   <211> 19
   <212> DNA
   <213> Artificial Sequence
<220>
   <221> source
   <223> /note="Description of Artificial Sequence: Synthetic oligonucleotide"
<400> 473
   agagatgatg gggagggca 19
<210> 474
   <211> 17
   <212> DNA
   <213> Artificial Sequence
<220>
   <221> source
   <223> /note="Description of Artificial Sequence: Synthetic oligonucleotide"
<400> 474
   agatgatggg gagggca 17
<210> 475
   <211> 18
   <212> DNA
   <213> Artificial Sequence
<220>
   <221> source
   <223> /note="Description of Artificial Sequence: Synthetic oligonucleotide"
<400> 475
   tcagcatcca acaaggca 18
<210> 476
   <211> 19
   <212> DNA
   <213> Artificial Sequence
<220>
   <221> source
   <223> /note="Description of Artificial Sequence: Synthetic oligonucleotide"
<400> 476
   ctcagcatcc aacaaggca 19
<210> 477
   <211> 28
   <212> DNA
   <213> Artificial Sequence
<220>
   <221> source
   <223> /note="Description of Artificial Sequence: Synthetic oligonucleotide"
<400> 477
   ttgtccctga cggccgacca gatggtca 28
<210> 478
   <211> 25
   <212> DNA
   <213> Artificial Sequence
<220>
   <221> source
   <223> /note="Description of Artificial Sequence: Synthetic oligonucleotide"
<400> 478
   cgctcatgat caaacgctct aagag 25
<210> 479
   <211> 25
   <212> DNA
   <213> Artificial Sequence
<220>
   <221> source
   <223> /note="Description of Artificial Sequence: Synthetic oligonucleotide"
<400> 479
   cgctcatgat caaacgctct aagcg 25
<210> 480
   <211> 25
   <212> DNA
   <213> Artificial Sequence
<220>
   <221> source
   <223> /note="Description of Artificial Sequence: Synthetic oligonucleotide"
<400> 480
   cgctcatgat caaacgctct aaggg 25
<210> 481
   <211> 25
   <212> DNA
   <213> Artificial Sequence
<220>
   <221> source
   <223> /note="Description of Artificial Sequence: Synthetic oligonucleotide"
<400> 481
   cgctcatgat caaacgctct aagtg 25
<210> 482
   <211> 25
   <212> DNA
   <213> Artificial Sequence
<220>
   <221> source
   <223> /note="Description of Artificial Sequence: Synthetic oligonucleotide"
<400> 482
   cgctcatgat caaacgctct aaaag 25
<210> 483
   <211> 25
   <212> DNA
   <213> Artificial Sequence
<220>
   <221> source
   <223> /note="Description of Artificial Sequence: Synthetic oligonucleotide"
<400> 483
   cgctcatgat caaacgctct aacag 25
<210> 484
   <211> 25
   <212> DNA
   <213> Artificial Sequence
<220>
   <221> source
   <223> /note="Description of Artificial Sequence: Synthetic oligonucleotide"
<400> 484
   cgctcatgat caaacgctct aatag 25
<210> 485
   <211> 25
   <212> DNA
   <213> Artificial Sequence
<220>
   <221> source
   <223> /note="Description of Artificial Sequence: Synthetic oligonucleotide"
<400> 485
   cgctcatgat caaacgctct acgag 25
<210> 486
   <211> 25
   <212> DNA
   <213> Artificial Sequence
<220>
   <221> source
   <223> /note="Description of Artificial Sequence: Synthetic oligonucleotide"
<400> 486
   cgctcatgat caaacgctct aggag 25
<210> 487
   <211> 25
   <212> DNA
   <213> Artificial Sequence
<220>
   <221> source
   <223> /note="Description of Artificial Sequence: Synthetic oligonucleotide"
<400> 487
   cgctcatgat caaacgctct atgag 25
<210> 488
   <211> 30
   <212> DNA
   <213> Artificial Sequence
<220>
   <221> source
   <223> /note="Description of Artificial Sequence: Synthetic oligonucleotide"
<400> 488
   tttgatcatg agcgggcttg gccaaaggtt 30
<210> 489
   <211> 18
   <212> DNA
   <213> Artificial Sequence
<220>
   <221> source
   <223> /note="Description of Artificial Sequence: Synthetic oligonucleotide"
<400> 489
   acaaggccag gctgttcc 18
<210> 490
   <211> 18
   <212> DNA
   <213> Artificial Sequence
<220>
   <221> source
   <223> /note="Description of Artificial Sequence: Synthetic oligonucleotide"
<400> 490
   acaaggccag gctgttac 18
<210> 491
   <211> 18
   <212> DNA
   <213> Artificial Sequence
<220>
   <221> source
   <223> /note="Description of Artificial Sequence: Synthetic oligonucleotide"
<400> 491
   acaaggccag gctgttgc 18
<210> 492
   <211> 18
   <212> DNA
   <213> Artificial Sequence
<220>
   <221> source
   <223> /note="Description of Artificial Sequence: Synthetic oligonucleotide"
<400> 492
   acaaggccag gctgtttc 18
<210> 493
   <211> 18
   <212> DNA
   <213> Artificial Sequence
<220>
   <221> source
   <223> /note="Description of Artificial Sequence: Synthetic oligonucleotide"
<400> 493
   acaaggccag gctgtacc 18
<210> 494
   <211> 18
   <212> DNA
   <213> Artificial Sequence
<220>
   <221> source
   <223> /note="Description of Artificial Sequence: Synthetic oligonucleotide"
<400> 494
   acaaggccag gctgtccc 18
<210> 495
   <211> 18
   <212> DNA
   <213> Artificial Sequence
<220>
   <221> source
   <223> /note="Description of Artificial Sequence: Synthetic oligonucleotide"
<400> 495
   acaaggccag gctgtgcc 18
<210> 496
   <211> 18
   <212> DNA
   <213> Artificial Sequence
<220>
   <221> source
   <223> /note="Description of Artificial Sequence: Synthetic oligonucleotide"
<400> 496
   acaaggccag gctgatcc 18
<210> 497
   <211> 18
   <212> DNA
   <213> Artificial Sequence
<220>
   <221> source
   <223> /note="Description of Artificial Sequence: Synthetic oligonucleotide"
<400> 497
   acaaggccag gctgctcc 18
<210> 498
   <211> 18
   <212> DNA
   <213> Artificial Sequence
<220>
   <221> source
   <223> /note="Description of Artificial Sequence: Synthetic oligonucleotide"
<400> 498
   acaaggccag gctggtcc 18
<210> 499
   <211> 19
   <212> DNA
   <213> Artificial Sequence
<220>
   <221> source
   <223> /note="Description of Artificial Sequence: Synthetic oligonucleotide"
<400> 499
   gtctggcgag agatgcaaa 19
<210> 500
   <211> 28
   <212> DNA
   <213> Artificial Sequence
<220>
   <221> source
   <223> /note="Description of Artificial Sequence: Synthetic oligonucleotide"
<400> 500
   ctctactttc cttacctcct tccttcca 28
<210> 501
   <211> 22
   <212> DNA
   <213> Artificial Sequence
<220>
   <221> source
   <223> /note="Description of Artificial Sequence: Synthetic oligonucleotide"
<400> 501
   gcctcaatga agacaacttg aa 22
<210> 502
   <211> 22
   <212> DNA
   <213> Artificial Sequence
<220>
   <221> source
   <223> /note="Description of Artificial Sequence: Synthetic oligonucleotide"
<400> 502
   aggataaagt ggatctgctg ca 22
<210> 503
   <211> 20
   <212> DNA
   <213> Artificial Sequence
<220>
   <221> source
   <223> /note="Description of Artificial Sequence: Synthetic oligonucleotide"
<400> 503
   cctggcgtcg attatctgaa 20
<210> 504
   <211> 22
   <212> DNA
   <213> Artificial Sequence
<220>
   <221> source
   <223> /note="Description of Artificial Sequence: Synthetic oligonucleotide"
<400> 504
   gctgttaatt gtccatgcat aa 22
<210> 505
   <211> 21
   <212> DNA
   <213> Artificial Sequence
<220>
   <221> source
   <223> /note="Description of Artificial Sequence: Synthetic oligonucleotide"
<400> 505
   gaaaggggag aacaagctaa a 21
<210> 506
   <211> 21
   <212> DNA
   <213> Artificial Sequence
<220>
   <221> source
   <223> /note="Description of Artificial Sequence: Synthetic oligonucleotide"
<400> 506
   gaggaatgga tttcaatgga a 21
<210> 507
   <211> 21
   <212> DNA
   <213> Artificial Sequence
<220>
   <221> source
   <223> /note="Description of Artificial Sequence: Synthetic oligonucleotide"
<400> 507
   ccctgggtct gtgatcacta a 21
<210> 508
   <211> 20
   <212> DNA
   <213> Artificial Sequence
<220>
   <221> source
   <223> /note="Description of Artificial Sequence: Synthetic oligonucleotide"
<400> 508
   gccacggacc atgaccatga 20
<210> 509
   <211> 20
   <212> DNA
   <213> Artificial Sequence
<220>
   <221> source
   <223> /note="Description of Artificial Sequence: Synthetic oligonucleotide"
<400> 509
   cttgagctgc ggacggttca 20
<210> 510
   <211> 25
   <212> DNA
   <213> Artificial Sequence
<220>
   <221> source
   <223> /note="Description of Artificial Sequence: Synthetic oligonucleotide"
<400> 510
   tggccctact gcatcagatc caagg 25
<210> 511
   <211> 22
   <212> DNA
   <213> Artificial Sequence
<220>
   <221> source
   <223> /note="Description of Artificial Sequence: Synthetic oligonucleotide"
<400> 511
   cagagaaaga ttggccagta cc 22
<210> 512
   <211> 23
   <212> DNA
   <213> Artificial Sequence
<220>
   <221> source
   <223> /note="Description of Artificial Sequence: Synthetic oligonucleotide"
<400> 512
   gccagtacca atgacaaggg aag 23
<210> 513
   <211> 21
   <212> DNA
   <213> Artificial Sequence
<220>
   <221> source
   <223> /note="Description of Artificial Sequence: Synthetic oligonucleotide"
<400> 513
   ccagggtggc agagaaagat t 21
<210> 514
   <211> 23
   <212> DNA
   <213> Artificial Sequence
<220>
   <221> source
   <223> /note="Description of Artificial Sequence: Synthetic oligonucleotide"
<400> 514
   cagactccat aatggtagcc tga 23
<210> 515
   <211> 25
   <212> DNA
   <213> Artificial Sequence
<220>
   <221> source
   <223> /note="Description of Artificial Sequence: Synthetic oligonucleotide"
<400> 515
   taatggtagc ctgaagcata gtcat 25
<210> 516
   <211> 23
   <212> DNA
   <213> Artificial Sequence
<220>
   <221> source
   <223> /note="Description of Artificial Sequence: Synthetic oligonucleotide"
<400> 516
   cactgcacag tagcgagtct cct 23
<210> 517
   <211> 28
   <212> DNA
   <213> Artificial Sequence
<220>
   <221> source
   <223> /note="Description of Artificial Sequence: Synthetic oligonucleotide"
<400> 517
   tgacaaggga agtatggcta tggaatct 28
<210> 518
   <211> 28
   <212> DNA
   <213> Artificial Sequence
<220>
   <221> source
   <223> /note="Description of Artificial Sequence: Synthetic oligonucleotide"
<400> 518
   tgacaaggga agtatggcta tggaatct 28
<210> 519
   <211> 28
   <212> DNA
   <213> Artificial Sequence
<220>
   <221> source
   <223> /note="Description of Artificial Sequence: Synthetic oligonucleotide"
<400> 519
   ctatggaatc tgccaaggag actcgcta 28
<210> 520
   <211> 28
   <212> DNA
   <213> Artificial Sequence
<220>
   <221> source
   <223> /note="Description of Artificial Sequence: Synthetic oligonucleotide"
<400> 520
   tgacaaggga agtatggcta tggaatct 28
<210> 521
   <211> 28
   <212> DNA
   <213> Artificial Sequence
<220>
   <221> source
   <223> /note="Description of Artificial Sequence: Synthetic oligonucleotide"
<400> 521
   cagtaccaat gacaagggaa gtatggct 28
<210> 522
   <211> 28
   <212> DNA
   <213> Artificial Sequence
<220>
   <221> source
   <223> /note="Description of Artificial Sequence: Synthetic oligonucleotide"
<400> 522
   tgacaaggga agtatggcta tggaatct 28
<210> 523
   <211> 22
   <212> DNA
   <213> Artificial Sequence
<220>
   <221> source
   <223> /note="Description of Artificial Sequence: Synthetic oligonucleotide"
<400> 523
   catgatcagg tccaccttct ac 22
<210> 524
   <211> 23
   <212> DNA
   <213> Artificial Sequence
<220>
   <221> source
   <223> /note="Description of Artificial Sequence: Synthetic oligonucleotide"
<400> 524
   ccatgatcag gtccaccttc tac 23
<210> 525
   <211> 24
   <212> DNA
   <213> Artificial Sequence
<220>
   <221> source
   <223> /note="Description of Artificial Sequence: Synthetic oligonucleotide"
<400> 525
   tccatgatca ggtccacctt ctac 24
<210> 526
   <211> 23
   <212> DNA
   <213> Artificial Sequence
<220>
   <221> source
   <223> /note="Description of Artificial Sequence: Synthetic oligonucleotide"
<400> 526
   gagcaagtta ggagcaaaca gta 23
<210> 527
   <211> 24
   <212> DNA
   <213> Artificial Sequence
<220>
   <221> source
   <223> /note="Description of Artificial Sequence: Synthetic oligonucleotide"
<400> 527
   agagcaagtt aggagcaaac agta 24
<210> 528
   <211> 25
   <212> DNA
   <213> Artificial Sequence
<220>
   <221> source
   <223> /note="Description of Artificial Sequence: Synthetic oligonucleotide"
<400> 528
   aagagcaagt taggagcaaa cagta 25
<210> 529
   <211> 22
   <212> DNA
   <213> Artificial Sequence
<220>
   <221> source
   <223> /note="Description of Artificial Sequence: Synthetic oligonucleotide"
<400> 529
   catgatcagg tccaccttct ag 22
<210> 530
   <211> 23
   <212> DNA
   <213> Artificial Sequence
<220>
   <221> source
   <223> /note="Description of Artificial Sequence: Synthetic oligonucleotide"
<400> 530
   gagcaagtta ggagcaaaca gta 23
<210> 531
   <211> 24
   <212> DNA
   <213> Artificial Sequence
<220>
   <221> source
   <223> /note="Description of Artificial Sequence: Synthetic oligonucleotide"
<400> 531
   agagcaagtt aggagcaaac agta 24
<210> 532
   <211> 25
   <212> DNA
   <213> Artificial Sequence
<220>
   <221> source
   <223> /note="Description of Artificial Sequence: Synthetic oligonucleotide"
<400> 532
   aagagcaagt taggagcaaa cagta 25
<210> 533
   <211> 28
   <212> DNA
   <213> Artificial Sequence
<220>
   <221> source
   <223> /note="Description of Artificial Sequence: Synthetic oligonucleotide"
<400> 533
   tgcctggcta gagatcctga tgattggt 28
<210> 534
   <211> 23
   <212> DNA
   <213> Artificial Sequence
<220>
   <221> source
   <223> /note="Description of Artificial Sequence: Synthetic oligonucleotide"
<400> 534
   ggtagagatc ttcgacatgc tgg 23
<210> 535
   <211> 25
   <212> DNA
   <213> Artificial Sequence
<220>
   <221> source
   <223> /note="Description of Artificial Sequence: Synthetic oligonucleotide"
<400> 535
   atggtagaga tcttcgacat gctgg 25
<210> 536
   <211> 23
   <212> DNA
   <213> Artificial Sequence
<220>
   <221> source
   <223> /note="Description of Artificial Sequence: Synthetic oligonucleotide"
<400> 536
   atgtgtagag ggcatggaga tct 23
<210> 537
   <211> 26
   <212> DNA
   <213> Artificial Sequence
<220>
   <221> source
   <223> /note="Description of Artificial Sequence: Synthetic oligonucleotide"
<400> 537
   gttatcaact caccagaatt aagcaa 26
<210> 538
   <211> 27
   <212> DNA
   <213> Artificial Sequence
<220>
   <221> source
   <223> /note="Description of Artificial Sequence: Synthetic oligonucleotide"
<400> 538
   tgttatcaac tcaccagaat taagcaa 27
<210> 539
   <211> 28
   <212> DNA
   <213> Artificial Sequence
<220>
   <221> source
   <223> /note="Description of Artificial Sequence: Synthetic oligonucleotide"
<400> 539
   gtgttatcaa ctcaccagaa ttaagcaa 28
<210> 540
   <211> 27
   <212> DNA
   <213> Artificial Sequence
<220>
   <221> source
   <223> /note="Description of Artificial Sequence: Synthetic oligonucleotide"
<400> 540
   catctcggtt ccgcatgatg aatctgc 27
<210> 541
   <211> 23
   <212> DNA
   <213> Artificial Sequence
<220>
   <221> source
   <223> /note="Description of Artificial Sequence: Synthetic oligonucleotide"
<400> 541
   ggtagagatc ttcgacatgc tgc 23
<210> 542
   <211> 26
   <212> DNA
   <213> Artificial Sequence
<220>
   <221> source
   <223> /note="Description of Artificial Sequence: Synthetic oligonucleotide"
<400> 542
   gttatcaact caccagaatt aagcaa 26
<210> 543
   <211> 23
   <212> DNA
   <213> Artificial Sequence
<220>
   <221> source
   <223> /note="Description of Artificial Sequence: Synthetic oligonucleotide"
<400> 543
   ggtagagatc ttcgacatgc tgc 23
<210> 544
   <211> 28
   <212> DNA
   <213> Artificial Sequence
<220>
   <221> source
   <223> /note="Description of Artificial Sequence: Synthetic oligonucleotide"
<400> 544
   tcaccagaat taagcaaaat aatagatt 28
<210> 545
   <211> 28
   <212> DNA
   <213> Artificial Sequence
<220>
   <221> source
   <223> /note="Description of Artificial Sequence: Synthetic oligonucleotide"
<400> 545
   catctcggtt ccgcatgatg aatctgca 28
<210> 546
   <211> 24
   <212> DNA
   <213> Artificial Sequence
<220>
   <221> source
   <223> /note="Description of Artificial Sequence: Synthetic oligonucleotide"
<400> 546
   attcaggagt gtacacattt ctgc 24
<210> 547
   <211> 25
   <212> DNA
   <213> Artificial Sequence
<220>
   <221> source
   <223> /note="Description of Artificial Sequence: Synthetic oligonucleotide"
<400> 547
   cattcaggag tgtacacatt tctgt 25
<210> 548
   <211> 23
   <212> DNA
   <213> Artificial Sequence
<220>
   <221> source
   <223> /note="Description of Artificial Sequence: Synthetic oligonucleotide"
<400> 548
   atcaggtgga tcaaagtgtc tgt 23
<210> 549
   <211> 28
   <212> DNA
   <213> Artificial Sequence
<220>
   <221> source
   <223> /note="Description of Artificial Sequence: Synthetic oligonucleotide"
<400> 549
   tctctggaag agaaggacca tatccacc 28
<210> 550
   <211> 19
   <212> DNA
   <213> Artificial Sequence
<220>
   <221> source
   <223> /note="Description of Artificial Sequence: Synthetic oligonucleotide"
<400> 550
   acgtggtgcc cctctatgg 19
<210> 551
   <211> 18
   <212> DNA
   <213> Artificial Sequence
<220>
   <221> source
   <223> /note="Description of Artificial Sequence: Synthetic oligonucleotide"
<400> 551
   caagaacgtg gtgcccca 18
<210> 552
   <211> 20
   <212> DNA
   <213> Artificial Sequence
<220>
   <221> source
   <223> /note="Description of Artificial Sequence: Synthetic oligonucleotide"
<400> 552
   tgcaagaacg tggtgcccca 20
<210> 553
   <211> 21
   <212> DNA
   <213> Artificial Sequence
<220>
   <221> source
   <223> /note="Description of Artificial Sequence: Synthetic oligonucleotide"
<400> 553
   gtgcaagaac gtggtgcccc a 21
<210> 554
   <211> 21
   <212> DNA
   <213> Artificial Sequence
<220>
   <221> source
   <223> /note="Description of Artificial Sequence: Synthetic oligonucleotide"
<400> 554
   gtgcaagaac gtggtgccac a 21
<210> 555
   <211> 18
   <212> DNA
   <213> Artificial Sequence
<220>
   <221> source
   <223> /note="Description of Artificial Sequence: Synthetic oligonucleotide"
<400> 555
   caagaacgtg gtgccccc 18
<210> 556
   <211> 19
   <212> DNA
   <213> Artificial Sequence
<220>
   <221> source
   <223> /note="Description of Artificial Sequence: Synthetic oligonucleotide"
<400> 556
   caagaacgtg gtgccccag 19
<210> 557
   <211> 18
   <212> DNA
   <213> Artificial Sequence
<220>
   <221> source
   <223> /note="Description of Artificial Sequence: Synthetic oligonucleotide"
<400> 557
   caagaacgtg gtgccccg 18
<210> 558
   <211> 22
   <212> DNA
   <213> Artificial Sequence
<220>
   <221> source
   <223> /note="Description of Artificial Sequence: Synthetic oligonucleotide"
<400> 558
   tgaagtgcaa gaacgtggtg ca 22
<210> 559
   <211> 24
   <212> DNA
   <213> Artificial Sequence
<220>
   <221> source
   <223> /note="Description of Artificial Sequence: Synthetic oligonucleotide"
<400> 559
   catgaagtgc aagaacgtgg tgca 24
<210> 560
   <211> 22
   <212> DNA
   <213> Artificial Sequence
<220>
   <221> source
   <223> /note="Description of Artificial Sequence: Synthetic oligonucleotide"
<400> 560
   tgaagtacaa gaacgtggtg ca 22
<210> 561
   <211> 23
   <212> DNA
   <213> Artificial Sequence
<220>
   <221> source
   <223> /note="Description of Artificial Sequence: Synthetic oligonucleotide"
<400> 561
   atgaagtaca agaacgtggt gca 23
<210> 562
   <211> 24
   <212> DNA
   <213> Artificial Sequence
<220>
   <221> source
   <223> /note="Description of Artificial Sequence: Synthetic oligonucleotide"
<400> 562
   catgaagtac aagaacgtgg tgca 24
<210> 563
   <211> 22
   <212> DNA
   <213> Artificial Sequence
<220>
   <221> source
   <223> /note="Description of Artificial Sequence: Synthetic oligonucleotide"
<400> 563
   tgaagtgcaa gaacgtgatg ca 22
<210> 564
   <211> 26
   <212> DNA
   <213> Artificial Sequence
<220>
   <221> source
   <223> /note="Description of Artificial Sequence: Synthetic oligonucleotide"
<400> 564
   agcatgaagt acaagaacgt ggtgca 26
<210> 565
   <211> 22
   <212> DNA
   <213> Artificial Sequence
<220>
   <221> source
   <223> /note="Description of Artificial Sequence: Synthetic oligonucleotide"
<400> 565
   tgaagtgcaa gaacgtggta ca 22
<210> 566
   <211> 23
   <212> DNA
   <213> Artificial Sequence
<220>
   <221> source
   <223> /note="Description of Artificial Sequence: Synthetic oligonucleotide"
<400> 566
   atgaagtgca agaacgtggt aca 23
<210> 567
   <211> 24
   <212> DNA
   <213> Artificial Sequence
<220>
   <221> source
   <223> /note="Description of Artificial Sequence: Synthetic oligonucleotide"
<400> 567
   catgaagtgc aagaacgtgg taca 24
<210> 568
   <211> 21
   <212> DNA
   <213> Artificial Sequence
<220>
   <221> source
   <223> /note="Description of Artificial Sequence: Synthetic oligonucleotide"
<400> 568
   catgaagtgc aagaacgtgg a 21
<210> 569
   <211> 22
   <212> DNA
   <213> Artificial Sequence
<220>
   <221> source
   <223> /note="Description of Artificial Sequence: Synthetic oligonucleotide"
<400> 569
   gcatgaagtg caagaacgtg ga 22
<210> 570
   <211> 23
   <212> DNA
   <213> Artificial Sequence
<220>
   <221> source
   <223> /note="Description of Artificial Sequence: Synthetic oligonucleotide"
<400> 570
   agcatgaagt gcaagaacgt gga 23
<210> 571
   <211> 20
   <212> DNA
   <213> Artificial Sequence
<220>
   <221> source
   <223> /note="Description of Artificial Sequence: Synthetic oligonucleotide"
<400> 571
   acgtggtgcc cctctatgac 20
<210> 572
   <211> 19
   <212> DNA
   <213> Artificial Sequence
<220>
   <221> source
   <223> /note="Description of Artificial Sequence: Synthetic oligonucleotide"
<400> 572
   gctttggtcc gtctcctcc 19
<210> 573
   <211> 25
   <212> DNA
   <213> Artificial Sequence
<220>
   <221> source
   <223> /note="Description of Artificial Sequence: Synthetic oligonucleotide"
<400> 573
   ctgctggaga tgctggacgc ccacc 25
<210> 574
   <211> 19
   <212> DNA
   <213> Artificial Sequence
<220>
   <221> source
   <223> /note="Description of Artificial Sequence: Synthetic oligonucleotide"
<400> 574
   agaacgtggt gcccctctg 19
<210> 575
   <211> 18
   <212> DNA
   <213> Artificial Sequence
<220>
   <221> source
   <223> /note="Description of Artificial Sequence: Synthetic oligonucleotide"
<400> 575
   agaacgtggt gcccctca 18
<210> 576
   <211> 19
   <212> DNA
   <213> Artificial Sequence
<220>
   <221> source
   <223> /note="Description of Artificial Sequence: Synthetic oligonucleotide"
<400> 576
   aagaacgtgg tgcccctca 19
<210> 577
   <211> 20
   <212> DNA
   <213> Artificial Sequence
<220>
   <221> source
   <223> /note="Description of Artificial Sequence: Synthetic oligonucleotide"
<400> 577
   caagaacgtg gtgcccctca 20
<210> 578
   <211> 19
   <212> DNA
   <213> Artificial Sequence
<220>
   <221> source
   <223> /note="Description of Artificial Sequence: Synthetic oligonucleotide"
<400> 578
   agaacgtggt gcccctctc 19
<210> 579
   <211> 23
   <212> DNA
   <213> Artificial Sequence
<220>
   <221> source
   <223> /note="Description of Artificial Sequence: Synthetic oligonucleotide"
<400> 579
   ctcctagacc tcatcctctt tga 23